(19) Europäisches Patentamt
European Patent Office
Office européen des brevets

(11) **EP 2 676 137 B1**

(12) **EUROPEAN PATENT SPECIFICATION**

(45) Date of publication and mention of the grant of the patent:
**31.12.2014 Bulletin 2015/01**

(21) Application number: **12707193.4**

(22) Date of filing: **16.02.2012**

(51) Int Cl.:
*G01N 33/564* (2006.01)　*G01N 33/94* (2006.01)

(86) International application number:
**PCT/US2012/025437**

(87) International publication number:
**WO 2012/154253 (15.11.2012 Gazette 2012/46)**

(54) **ASSAYS FOR DETECTING AUTOANTIBODIES TO ANTI-TNF DRUGS**

TESTS FÜR DEN NACHWEIS VON AUTOANTIKÖRPERN GEGEN ANTI-TNF-ARZNEIMITTEL

ESSAIS POUR LA DÉTECTION D'AUTO-ANTICORPS DIRIGÉS CONTRE DES MÉDICAMENTS ANTI-TNF

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **17.02.2011　US 201161444097 P**
**10.05.2011　US 201161484594 P**
**13.06.2011　US 201161496501 P**

(43) Date of publication of application:
**25.12.2013　Bulletin 2013/52**

(73) Proprietor: **Nestec S.A.**
**1800 Vevey (CH)**

(72) Inventors:
- **SINGH, Sharat**
  **Rancho Santa Fe**
  **California 92127 (US)**
- **WANG, Shui Long**
  **San Diego**
  **California 92130 (US)**
- **OHRMUND, Linda**
  **San Diego**
  **California 92106 (US)**
- **HAUENSTEIN, Scott**
  **San Diego**
  **California 92130 (US)**

(74) Representative: **Krishnan, Sri**
**Nestec S.A.**
**CT-IAM**
**Av. Nestlé 55**
**1800 Vevey (CH)**

(56) References cited:
**US-A1- 2009 035 216**

- **AARDEN L ET AL: "Immunogenicity of anti-tumor necrosis factor antibodies-toward improved methods of anti-antibody measurement", CURRENT OPINION IN IMMUNOLOGY, ELSEVIER, OXFORD, GB, vol. 20, no. 4, 1 August 2008 (2008-08-01) , pages 431-435, XP023612042, ISSN: 0952-7915, DOI: 10.1016/J.COI.2008.06.011 [retrieved on 2008-07-19]**
- **SICKERT D ET AL: "Improvement of drug tolerance in immunogenicity testing by acid treatment on Biacore", JOURNAL OF IMMUNOLOGICAL METHODS, ELSEVIER SCIENCE PUBLISHERS B.V.,AMSTERDAM, NL, vol. 334, no. 1-2, 20 May 2008 (2008-05-20), pages 29-36, XP022614831, ISSN: 0022-1759, DOI: 10.1016/J.JIM.2008.01.010 [retrieved on 2008-02-14]**

- LOFGREN J A ET AL: "Detection of neutralizing anti-therapeutic protein antibodies in serum or plasma samples containing high levels of the therapeutic protein", JOURNAL OF IMMUNOLOGICAL METHODS, ELSEVIER SCIENCE PUBLISHERS B.V.,AMSTERDAM, NL, vol. 308, no. 1-2, 20 January 2006 (2006-01-20), pages 101-108, XP025158048, ISSN: 0022-1759, DOI: 10.1016/J.JIM.2005.10.007 [retrieved on 2006-01-20]
- BOURDAGE ET AL: "An Affinity Capture Elution (ACE) assay for detection of anti-drug antibody to monoclonal antibody therapeutics in the presence of high levels of drug", JOURNAL OF IMMUNOLOGICAL METHODS, ELSEVIER SCIENCE PUBLISHERS B.V.,AMSTERDAM, NL, vol. 327, no. 1-2, 25 September 2007 (2007-09-25), pages 10-17, XP022266386, ISSN: 0022-1759, DOI: 10.1016/J.JIM.2007.07.004
- SMITH ET AL: "Detection of antibodies against therapeutic proteins in the presence of residual therapeutic protein using a solid-phase extraction with acid dissociation (SPEAD) sample treatment prior to ELISA", REGULATORY TOXICOLOGY AND PHARMACOLOGY, ACADEMIC PRESS,NEW YORK, NY, US, vol. 49, no. 3, 13 November 2007 (2007-11-13), pages 230-237, XP022343220, ISSN: 0273-2300, DOI: 10.1016/J.YRTPH.2007.07.005
- PATTON ET AL: "An acid dissociation bridging ELISA for detection of antibodies directed against therapeutic proteins in the presence of antigen", JOURNAL OF IMMUNOLOGICAL METHODS, ELSEVIER SCIENCE PUBLISHERS B.V.,AMSTERDAM, NL, vol. 304, no. 1-2, 1 September 2005 (2005-09-01), pages 189-195, XP005065150, ISSN: 0022-1759
- CANAN AYBAY ET AL: "Demonstration of specific antibodies against infliximab induced during treatment of a patient with ankylosing spondylitis", RHEUMATOLOGY INTERNATIONAL ; CLINICAL AND EXPERIMENTAL INVESTIGATIONS, SPRINGER, BERLIN, DE, vol. 26, no. 5, 1 March 2006 (2006-03-01), pages 473-480, XP019335344, ISSN: 1437-160X, DOI: 10.1007/S00296-005-0085-0
- KLAUS BENDTZEN ET AL: "Individualized monitoring of drug bioavailability and immunogenicity in rheumatoid arthritis patients treated with the tumor necrosis factor alpha inhibitor infliximab", ARTHRITIS & RHEUMATISM, JOHN WILEY & SONS, INC, US, vol. 54, no. 12, 1 December 2006 (2006-12-01), pages 3782-3789, XP002626408, ISSN: 0004-3591, DOI: 10.1002/ART.22214 [retrieved on 2006-11-28]
- ROJAS J R ET AL: "FORMATION, DISTRIBUTION, AND ELIMINATION OF INFLIXIMAB AND ANTI-INFLIXIMAB IMMUNE COMPLEXES IN CYNOMOLGUS MONKEYS", JOURNAL OF PHARMACOLOGY AND EXPERIMENTAL THERAPEUTICS, AMERICAN SOCIETY FOR PHARMACOLOGY AND EXPERIMENTAL THERAPEUTICS, US, vol. 313, no. 2, 1 May 2005 (2005-05-01), pages 578-585, XP008070606, ISSN: 0022-3565, DOI: 10.1124/JPET.104.079277

**Description**

**BACKGROUND OF THE INVENTION**

Autoimmune disorders are a significant and widespread medical problem. For example, rheumatoid arthritis (RA) is an autoimmune disease affecting more than two million people in the United States. RA causes chronic inflammation of the joints and typically is a progressive illness that has the potential to cause joint destruction and functional disability. The cause of rheumatoid arthritis is unknown, although genetic predisposition, infectious agents and environmental factors have all been implicated in the etiology of the disease. In active RA, symptoms can include fatigue, lack of appetite, low grade fever, muscle and joint aches and stiffness. Also during disease flare ups, joints frequently become red, swollen, painful and tender, due to inflammation of the synovium. Furthermore, since RA is a systemic disease, inflammation can affect organs and areas of the body other than the joints, including glands of the eyes and mouth, the lung lining, the pericardium, and blood vessels.

Aarden et al describe the immunogenicity of anti-tumor necrosis factor antibodies-and provides improved methods of anti-antibody measurement (CURRENT OPINION IN IMMUNOLOGY, ELSEVIER, OXFORD, GB, vol. 20, no. 4, 1 August 2008, pages 431-435).

US 2009/035216 discloses a method for determining in vivo biopharmaceutical concentration and bioavailability.

Sickert et al. discuss the improvement of drug tolerance in immunogenicity testing by acid treatment on Biacore (JOURNAL OF IMMUNOLOGICAL METHODS, ELSEVIER SCIENCE PUBLISHERS B.V., AMSTERDAM, NL, vol. 334, no. 1-2, 20 May 2008, pages 29-36).

Lofgren et al. describes the detection of neutralizing anti-therapeutic protein antibodies in serum or plasma samples containing high levels of the therapeutic protein (JOURNAL OF IMMUNOLOGICAL METHODS, ELSEVIER SCIENCE PUBLISHERS B.V.,AMSTERDAM, NL, vol. 308, no. 1-2, 20 January 2006, pages 101-108).

Bourdage et al. discloses an Affinity Capture Elution (ACE) assay for detection of anti-drug antibody to monoclonal antibody therapeutics in the presence of high levels of drug (JOURNAL OF IMMUNOLOGICAL METHODS, ELSEVIER SCIENCE PUBLISHERS B.V.,AMSTERDAM, NL, vol. 327, no. 1-2, 25 September 2007, pages 10-17).

Smith et al. describes the detection of antibodies against therapeutic proteins in the presence of residual therapeutic protein using a solid-phase extraction with acid dissociation (SPEAD) sample treatment prior to ELISA (REGULATORY TOXICOLOGY AND PHARMACOLOGY, ACADEMIC PRESS,NEW YORK, NY, US, vol. 49, no. 3, 13 November 2007, pages 230-237).

Patton et al. report an acid dissociation bridging ELISA for detection of antibodies directed against therapeutic proteins in the presence of antigen", JOURNAL OF IMMUNOLOGICAL METHODS, ELSEVIER SCIENCE PUBLISHERS B.V.,AMSTERDAM, NL, vol. 304, no. 1-2, 1 September 2005, pages 189-195).

Canan Abay et al. report the demonstration of specific antibodies against infliximab induced during treatment of a patient with ankylosing spondylitis (RHEUMATOLOGY INTERNATIONAL ; CLINICAL AND EXPERIMENTAL INVESTIGATIONS, SPRINGER, BERLIN, DE, vol. 26, no. 5, 1 March 2006, pages 473-480).

Bendtzen et al. describe the individualized monitoring of drug bioavailability and immunogenicity in rheumatoid arthritis patients treated with the tumor necrosis factor alpha inhibitor infliximab (ARTHRITIS & RHEUMATISM, JOHN WILEY & SONS, INC, US, vol. 54, no. 12, 1 December 2006, pages 3782-3789).

Rojas J.R. et al. report the formation, distribution, and elimination of infliximab and anti-infliximab immune complexes in cynomologus monkeys (JOURNAL OF PHARMACOLOGY AND EXPERIMENTAL THERAPEUTICS, AMERICAN SOCIETY FOR PHARMACOLOGY AND EXPERIMENTAL THERAPEUTICS, US, vol. 313, no. 2, 1 May 2005, pages 578-585).

Traditional treatments for the management of RA and other autoimmune disorders include fast acting "first line drugs" and slower acting "second line drugs." The first line drugs reduce pain and inflammation. Example of such first line drugs include aspirin, naproxen, ibuprofen, etodolac and other non-steroidal antiinflammatory drugs (NSAIDs), as well as corticosteroids, given orally or injected directly into tissues and joints. The second line drugs promote disease remission and prevent progressive joint destruction and are also referred to as disease-modifying anti-rheumatic drugs or DMARDs. Examples of second line drugs include gold, hydrochloroquine, azulfidine and immunosuppressive agents, such as methotrexate, azathioprine, cyclophosphamide, chlorambucil and cyclosporine. Many of these drugs, however, can have detrimental side-effects. Thus, additional therapies for rheumatoid arthritis and other autoimmune disorders have been sought.

Tumor necrosis factor alpha (TNF-$\alpha$) is a cytokine produced by numerous cell types, including monocytes and macrophages, that was originally identified based on its ability to induce the necrosis of certain mouse tumors. Subsequently, a factor termed cachectin, associated with cachexia, was shown to be identical to TNF-$\alpha$. TNF-$\alpha$ has been implicated in the pathophysiology of a variety of other human diseases and disorders, including shock, sepsis, infections, autoimmune diseases, RA, Crohn's disease, transplant rejection and graft-versus-host disease.

Because of the harmful role of human TNF-$\alpha$ (hTNF-$\alpha$) in a variety of human disorders, therapeutic strategies have been designed to inhibit or counteract hTNF-$\alpha$ activity. In particular, antibodies that bind to, and neutralize, hTNF-$\alpha$ have been sought as a means to inhibit hTNF-$\alpha$ activity. Some of the earliest of such antibodies were mouse monoclonal antibodies (mAbs), secreted by hybridomas prepared from lymphocytes of mice immunized with hTNF-$\alpha$ (*see, e.g.,* U.S. Pat. No. 5,231,024 to Moeller et al.). While these mouse anti-hTNF-$\alpha$ antibodies often displayed high affinity for hTNF-$\alpha$ and were able to neutralize hTNF-$\alpha$ activity, their use *in vivo* has been limited by problems associated with the administration of mouse antibodies to humans, such as a short serum half-life, an inability to trigger certain human effector functions, and elicitation of an unwanted immune response against the mouse antibody in a human (the "human anti-mouse antibody" (HAMA) reaction).

More recently, biological therapies have been applied to the treatment of autoimmune disorders such as rheumatoid arthritis. For example, four TNF$\alpha$ inhibitors, REMICADE™ (infliximab), a chimeric anti-TNF$\alpha$ mAb, ENBREL™ (etanercept), a TNFR-Ig Fc fusion protein, HUMIRA™ (adalimumab), a human anti-TNF$\alpha$ mAb, and CIMZIA® (certolizumab pegol), a PEGylated Fab fragment, have been approved by the FDA for treatment of rheumatoid arthritis. CIMZIA® is also used for the treatment of moderate to severe Crohn's disease (CD). While such biologic therapies have demonstrated success in the treatment of rheumatoid arthritis and other autoimmune disorders such as CD, not all subjects treated respond, or respond well, to such therapy. Moreover, administration of TNF$\alpha$ inhibitors can induce an immune response to the drug and lead to the production of autoantibodies such as human anti-chimeric antibodies (HACA), human anti-humanized antibodies (HAHA), and human anti-mouse antibodies (HAMA). Such HACA, HAHA, or HAMA immune responses can be associated with hypersensitive reactions and dramatic changes in pharmacokinetics and biodistribution of the immunotherapeutic TNF$\alpha$ inhibitor that preclude further treatment with the drug. Thus, there is a need in the art for assays to detect the presence of autoantibodies to anti-TNF$\alpha$ biologics in a patient sample to monitor TNF$\alpha$ inhibitor therapy and to guide treatment decisions. The present satisfies this need and provides related advantages as well.

## BRIEF SUMMARY OF THE INVENTION

The present invention provides assays for detecting and measuring the presence or level of autoantibodies to anti-TNF$\alpha$ drug therapeutics in a sample. The present invention is useful for optimizing therapy and monitoring patients receiving anti-TNF$\alpha$ drug therapeutics to detect the presence or level of autoantibodies (e.g., HACA and/or HAHA) against the drug. The present invention also provides methods for selecting therapy, optimizing therapy, and/or reducing toxicity in subjects receiving anti-TNF$\alpha$ drugs for the treatment of TNF$\alpha$-mediated disease or disorders.

In one aspect, the present invention provides a method for detecting the presence or level of an autoantibody to an anti-TNF$\alpha$ drug in a sample without interference from the anti-TNF$\alpha$ drug in the sample, the method comprising:

(a) contacting the sample with an acid to dissociate preformed complexes of the autoantibody and the anti-TNFalpha drug, wherein the sample has or is suspected of having an autoantibody to the anti-TNFalpha drug;

(b) contacting the sample with a labeled anti-TNFalpha drug following dissociation of the preformed complexes, wherein optionally step (b) further comprises contacting a labeled internal control with the sample;

(c) neutralizing the acid in the sample to form labeled complexes of the labeled anti-TNFalpha drug and the autoantibody;

(d) subjecting the labeled complexes to size exclusion chromatography to separate the labeled complexes; and

(e) detecting the labeled complexes, thereby detecting the presence or level of the autoantibody without interference from the anti-TNFalpha drug in the sample.

The sample can be contacted with an acid at a concentration of from 0.1M to 5M.

The acid can be neutralized by adding one or more neutralizing agents to the sample.

The sample can be obtained from a subject receiving therapy with the anti-TNFalpha drug.

The complexes can be eluted first, followed by free labeled anti-TNFalpha drug.

The anti-TNFalpha drug can be labeled with a fluorophore or a fluorescent dye.

Methods for detecting anti-TNF$\alpha$ drugs and anti-drug antibodies are further described in PCT Publication No. WO 2011/056590.

In other aspects, the present invention provides a method for selecting a course of therapy (e.g., selecting an appropriate anti-TNF$\alpha$ drug) for the treatment of a TNF$\alpha$-mediated disease or disorder in a subject, the method comprising:

(a) detecting the presence or level of an autoantibody to the anti-TNFalpha drug in a sample from the subject without interference from the anti-TNFalpha drug in the sample, the method comprising:

(i) contacting the sample with an acid to dissociate preformed complexes of the autoantibody and the anti-TNFalpha drug, wherein the sample has or is suspected of having an autoantibody to the anti-TNFalpha drug;

(ii) contacting the sample with a labeled anti-TNFalpha drug following dissociation of the preformed complexes;

(iii) neutralizing the acid in the sample to form labeled complexes of the labeled anti-TNFalpha drug and the autoantibody; (iv) subjecting the labeled complexes to size exclusion chromatography to separate the labeled complexes; and

(v) detecting the labeled complexes to thereby detect the presence or level of the autoantibody without interference from the anti-TNFalpha drug in the sample; and (b) determining a subsequent dose of the course of therapy for the subject or whether a different course of therapy should be administered to the subject based upon the presence or level of the autoantibody,

thereby optimizing therapy and/or reducing toxicity to the anti-TNFalpha drug.

The subsequent dose of the course of therapy can be increased, decreased, or maintained based upon the presence or level of the autoantibody.

The different course of therapy can comprise a different anti-TNFalpha drug, or wherein the different course of therapy comprises the current course of therapy along with an immunosuppressive agent, or wherein the different course of therapy comprises switching to a course of therapy that is not an anti-TNFalpha drug.

The anti-TNFalpha drug can be selected from the group consisting of infliximab, etanercept, adalimumab, certolizumab pegol, golimumab, CNTO 148, and combinations thereof.

The autoantibody to the anti- TNFalpha drug can be selected from the group consisting of a human anti-chimeric antibody (HACA), a human anti-humanized antibody (HAHA), a human anti-mouse antibody (HAMA), and combinations thereof.

The acid can comprise an organic acid, an inorganic acid, or mixtures thereof, wherein optionally the organic acid comprises citric acid.

## BRIEF DESCRIPTION OF THE DRAWINGS

**Figure 1** shows an exemplary assays wherein size exclusion HPLC is used to detect the binding between TNF$\alpha$-Alexa$_{647}$ and HUMIRA™.

**Figure 2** shows dose response curves of HUMIRA™ binding to TNF$\alpha$-Alexa$_{647}$.

**Figure 3** shows a current ELISA-based method for measuring HACA levels, known as the bridging assay.

**Figure 4** illustrates an exemplary outline of the autoantibody detection assays for measuring the concentrations of HACA/HAHA generated against REMICADE™.

**Figure 5** shows a dose response analysis of anti-human IgG antibody binding to REMICADE™-Alexa$_{647}$.

**Figure 6** shows a second dose response analysis of anti-human IgG antibody binding to REMICADETM-Alexa$_{647}$.

**Figure 7** shows dose response curves of anti-human IgG antibody binding to REMICADE™-Alexa$_{647}$.

**Figure 8** shows REMICADE™-Alexa$_{647}$ immunocomplex formation in normal human serum and HACA positive serum.

**Figure 9** provides a summary of HACA measurements from 20 patient serum samples that were performed using the bridging assay or the mobility shift assay.

**Figure 10** provides a summary and comparison of current methods for measuring serum concentrations of HACA to the novel HACA assay.

**Figure 11** shows SE-HPLC profiles of fluorophore (FI)-labeled IFX incubated with normal (NHS) or HACA-positive (HPS) serum. The addition of increasing amounts of HACA-positive serum to the incubation mixture dose-dependently shifted the IFX-FI peak to the higher molecular mass eluting positions, C1 and C2.

**Figure 12** shows dose-response curves of the bound and free IFX-FI generated with increasing dilutions of HACA-positive serum as determined by the mobility shift assay. (A) Increasing dilutions of HACA-positive serum were incubated with 37.5 ng of IFX-FI. The higher the dilution (less HACA) the more free IFX-FI was found in the SE-HPLC analysis. (B) Increasing dilutions of HACA-positive serum were incubated with 37.5 ng of IFX-FI. The higher the dilution (less HACA) the less HACA bound IFX-F1 was found in the SE-HPLC analysis.

**Figure 13** shows SE-HPLC profiles of TNF$\alpha$-FI incubated with normal (NHS) or IFX-spiked serum. The addition of increasing amounts of IFX-spiked serum to the incubation mixture dose-dependently shifted the fluorescent TNF$\alpha$ peak to the higher molecular mass eluting positions.

**Figure 14** shows dose-response curves of the bound and free TNF$\alpha$ generated with increasing dilutions of IFX-spiked serum as determined by the mobility shift assay. Increasing concentrations of IFX added to the incubation mixture decreases the percentage of free TNF$\alpha$ while increasing the percentage of bound TNF$\alpha$.

**Figure 15** shows the measurement of relative HACA level and IFX concentration in IBD patients treated with IFX at different time points by the mobility shift assay.

**Figure 16** shows patient management- measurement of HACA level and IFX concentration in the sera of IBD patients treated with IFX at different time points.

**Figure 17** shows exemplary assays to detect the presence of (A) non-neutralizing or (B) neutralizing autoantibodies such as HACA.

**Figure 18** shows assays to detect the presence of neutralizing autoantibodies such as HACA.

**Figure 19** shows mobility shift profiles of FI-labeled ADL incubated with normal human serum (NHS) in the presence of different amounts of anti-human IgG. The addition of increasing amounts of anti-human IgG to the incubation mixture dose-dependently shifted the free FI-ADL peak (FA) to the higher molecular mass eluting positions, C1 and C2, while the internal control (IC) did not change.

**Figure 20** shows a dose-response curve of anti-human IgG on the shift of free FI-ADL. Increasing amounts of anti-human IgG were incubated with 37.5 ng of FI-ADL and internal control. The more the antibody was added to the reaction mixture the lower the ratio of free FI-ADL to internal control.

**Figure 21** shows mobility shift profiles of FI-labeled TNF-$\alpha$ incubated with normal human serum (NHS) in the presence of different amounts of ADL. Ex = 494 nm; Em = 519 nm. The addition of increasing amounts of ADL to the incubation mixture dose-dependently shifted the free TNF-FI peak (FT) to the higher molecular mass eluting positions, while the internal control (IC) peak did not change.

**Figure 22** shows a dose-response curve of ADL on the shift of free TNF-$\alpha$-FI. Increasing amounts of ADL were incubated with 100 ng of TNF-$\alpha$-FI and internal control. The more the antibody ADL was added to the reaction mixture the lower the ratio of free TNF-$\alpha$-FI to internal control.

**Figure 23** shows the mobility shift profiles of FI-labeled Remicade (IFX) Incubated with Normal (NHS) or Pooled HACA Positive Patient Serum.

**Figure 24** shows the mobility shift profiles of F1-Labeled HUMIRA (ADL) incubated with normal (NHS) or Mouse Anti-Human IgG1 Antibody.

**Figure 25** shows the mobility shift profiles of F1-Labeled HUMIRA (ADL) incubated with normal (NHS) or pooled HAHA positive patient serum.

**Figure 26** shows an illustration of the effect of the acid dissociation step. "A" represents labeled-Remicade, "B" represents HACA, "C" represents Remicade.

**Figure 27** shows the percent free labeled-Infliximab as a function of Log Patient Serum percentage without an acid dissociation step.

**Figure 28** shows the percent free labeled-Infliximab as a function of Log Patient Serum percentage with an acid dissocation step.

**Figure 29** shows the serum IFX levels in a patient treated with Infliximab as a function of time for the Patient Case 1.

**Figure 30** shows the serum IFX levels in a patient treated with Infliximab as a function of time for the Patient Case 3.

**Figure 31** shows the serum TNF$\alpha$ levels in a patient treated with Infliximab as a function of time for the Patient Case 3.

**Figure 32** shows the mobility shift profiles of Fl-Labeled-IFX for Patient Case 1 (A); Patient Case 2 (B, C); and Patient Case 4 (D).

**Figure 33** shows the mobility shift profiles of of Fl-Labeled-IFX for Patient Case 5 (A); Patient Case 6 (B, C); and Patient Case 7 (D, E).

**Figure 34** shows cytokine levels in different patient serum groups.

**Figure 35** shows the analysis of samples containing TNF-Alexa488 and Remicade by mobility shift assay using a fluorescence detector with gain settings at different values.

**Figure 36** shows isoabsorbance plots taken for normal human serum (top panel) and TNF-Alexa488 (bottom panel) in HPLC mobile phase (1X PBS, 0.1% BSA in water). Excitation wavelengths are plotted on the Y-axis and emission wavelengths are plotted on the X-axis.

**Figure 37** shows the HPLC analysis of normal human serum (left) and 25ng TNF-Alexa488 (right) detected with indicated settings. The background level of fluorescence from normal human serum is greatly decreased.

**Figure 38** shows the standard curve generated by HPLC analysis of samples containing a fixed amount of TNF-Alexa488 and titrated with various amounts of Remicade.

**Figure 39** shows a comparison of Infliximab determination in clinical samples by mobility shift assay and ELISA. Dark grey points are for HACA-positive samples and light grey points are for HACA-negative samples. Dashed lines represent lower limits of quantitations for the respective methods.

**Figure 40** shows a comparison of HACA determination in clinical samples by mobility shift assay and ELISA.

**Figure 41** shows the cumulative counts of HACA-positive clinical samples as determined by mobility shift assay and ELISA.

## DETAILED DESCRIPTION

### I. Introduction

The present invention is based in part on the discovery that a homogeneous mobility shift assay using size exclusion chromatography and acid dissociation to enable equilibration of immune complexes is particularly advantageous for measuring the presence or level of autoantibodies *(e.g.,* HACA, HAHA, *etc.)* that are generated against anti-TNF$\alpha$ drugs. Such autoantibodies are also known as anti-drug antibodies or ADA. As a result, the presence or level of autoantibodies to an anti-TNF$\alpha$ drug administered to a subject in need thereof can be measured without substantial interference from the administered anti-TNF$\alpha$ drug that is also present in the subject's sample. In particular, a subject's sample can be incubated with an amount of acid that is sufficient to provide for the measurement of the presence or level of autoantibodies in the presence of the anti-TNF$\alpha$ drug without substantial interference from high anti-TNF$\alpha$ drug levels.

High anti-TNF$\alpha$ drug levels in a sample *(e.g.,* high infliximab levels) interferes with the measumrent of anti-drug antibody levels *(e.g.,* HACA levles). Under certain high drug conditions, the anti-drug antibody present in a sample is complexed with the unlabeled drug also present in the sample. When a labeled drug, *e.g.* labeled-infliximab, is contacted with the sample, the anti-drug antibody present in the sample is kinetically trapped from forming a complex with the labeled drug. In this way, the preformed complexes of anti-drug antibody and the unlabeled drug interfere with the measurement of anti-drug antibody, which depends on the formation of a complex between the anti-drug antibody present and the labeled drug. The acid dissociation step described herein allows for the anti-drug antibody present in the sample to dissociate from the unlabeled drug and reform complexes with both the labeled and unlabeled drug. By dissociating the anti-drug antibody from the unlabeled drug, the anti-drug antibody present in a sample can equilibrate between the labeled drug and the unlabeled drug.

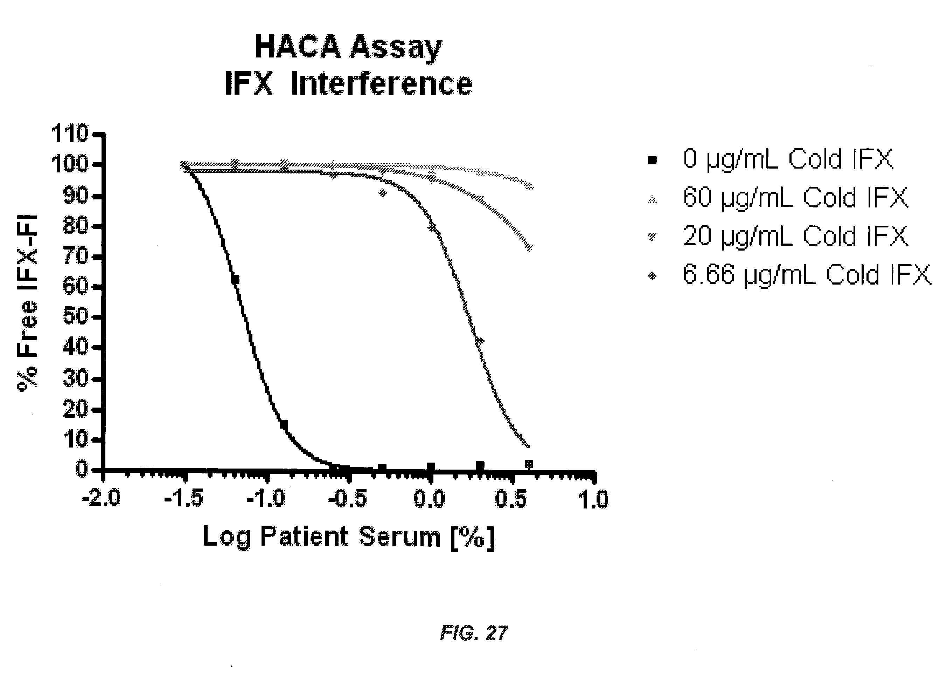

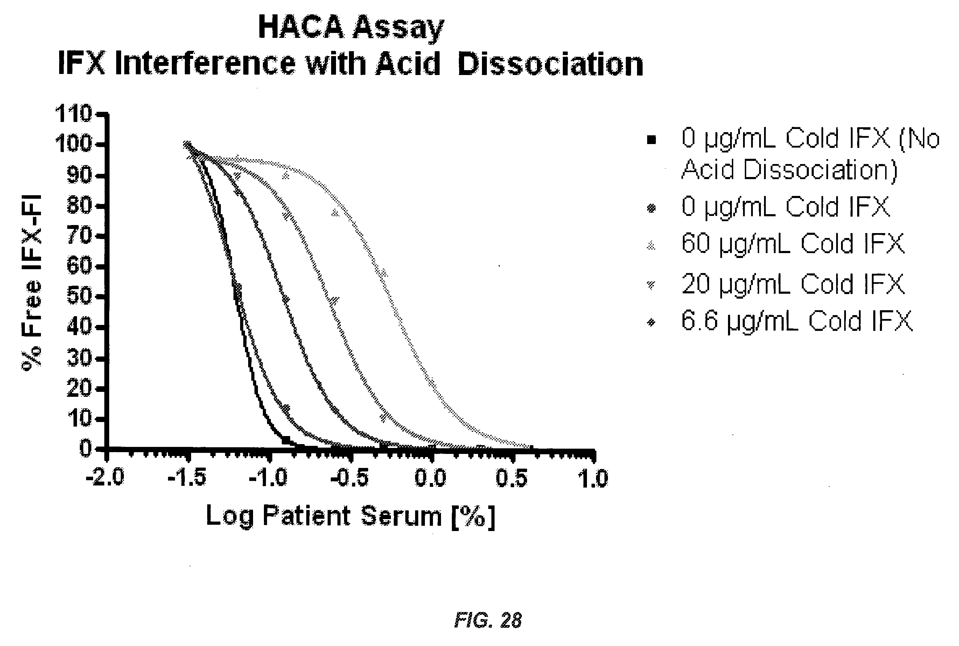

As shown in Figure 27, high levels of anti-TNFα drug (e.g., infliximab) interfere with the detection of anti-drug antibodies (e.g., antibodies to infliximab or ATI) when the mobility shift assay is performed without an acid dissociation step. However, Figure 28 shows that acid dissociation followed by homogeneous solution phase binding kinetics to allow the equilibration and reformation of immune complexes significantly increased the anti-TNFα drug tolerance such that anti-drug antibodies can be measured in the presence of high levels of anti-TNFα drug (e.g., up to or at least about 60 μg/mL). As such, the assays are particularly advantageous over methods currently available because they enable the detection and measurement of anti-drug antibodies at any time during therapy with an anti-TNFα drug (e.g., irrespective of low, medium, or high levels of anti-TNFα drug in a sample such as a blood sample), thereby overcoming a major limitation of methods in the art which require sample collection at trough concentrations of the drug.

In certain aspects, the present method is advantageous because it addresses and overcomes current limitations associated with the administration of anti-TNFα drugs such as infliximab, in part, by providing information useful for guiding treatment decisions for those patients receiving or about to receive anti-TNFα drug therapy. In particular, the methods find utility for selecting an appropriate anti-TNFα therapy for initial treatment, for determining when or how to adjust or modify (e.g., increase or decrease) the subsequent dose of an anti-TNFα drug to optimize therapeutic efficacy and/or to reduce toxicity, for determining when or how to combine an anti-TNFα drug (e.g., at an initial, increased, decreased, or same dose) with one or more immunosuppressive agents such as methotrexate (MTX) or azathioprine (AZA), and/or for determining when or how to change the current course of therapy (e.g., switch to a different anti-TNFα drug or to a drug that targets a different mechanism).

Accordingly, the present method is particularly useful in the following methods of improving patient management by guiding treatment decisions:

1. Crohn's disease prognostics: Treat patients most likely to benefit from therapy
2. Anti-therapeutic antibody monitoring (ATM) + Biomarker-based disease activity index
3. ATM sub-stratification
4. ATM with pharmacokinetic modeling
5. Monitor response and predict risk of relapse:

    a. Avoid chronic maintenance therapy in patients with low risk of recurrence
    b. Markers of mucosal healing
    c. Therapy selection: Whether to combine or not to combine anti-TNF drug therapy with an immunosuppressive agent such as MTX or AZA

6. Patient selection for biologics.

## II. Definitions

As used herein, the following terms have the meanings ascribed to them unless specified otherwise.

The terms "anti-TNFα drug" or "TNFα inhibitor" as used herein is intended to encompass agents including proteins, antibodies, antibody fragments, fusion proteins (e.g., Ig fusion proteins or Fc fusion proteins), multivalent binding proteins (e.g., DVD Ig), small molecule TNFα antagonists and similar naturally- or nonnaturally-occurring molecules, and/or recombinant and/or engineered forms thereof, that, directly or indirectly, inhibit TNFα activity, such as by inhibiting interaction of TNFα with a cell surface receptor for TNFα, inhibiting TNFα protein production, inhibiting TNFα gene expression, inhibiting TNFα secretion from cells, inhibiting TNFα receptor signaling or any other means resulting in decreased TNFα activity in a subject. The term "anti-TNFα drug" or "TNFα inhibitor" preferably includes agents which interfere with TNFα activity. Examples of anti-TNFα drugs include, without limitation, infliximab (REMICADE™, Johnson and Johnson), human anti-TNF monoclonal antibody adalimumab (D2E7/HUMIRA™, Abbott Laboratories), etanercept (ENBREL™, Amgen), certolizumab pegol (CIMZIA®, UCB, Inc.), golimumab (SIMPONI®; CNTO 148), CDP 571 (Celltech), CDP 870 (Celltech), as well as other compounds which inhibit TNFα activity, such that when administered to a subject suffering from or at risk of suffering from a disorder in which TNFα activity is detrimental (e.g., RA), the disorder is treated.

The term "TNFα" is intended to include a human cytokine that exists as a 17 kDa secreted form and a 26 kDa membrane associated form, the biologically active form of which is composed of a trimer of noncovalently bound 17 kDa molecules. The structure of TNFα is described further in, for example, Jones et al., Nature, 338:225-228 (1989). The term TNFα is intended to include human TNFα, a recombinant human TNFα (rhTNF-α), or TNFα that is at least about 80% identity to the human TNFα protein. Human TNFα consists of a 35 amino acid (aa) cytoplasmic domain, a 21 aa transmembrane segment, and a 177 aa extracellular domain (ECD) (Pennica, D. et al. (1984) Nature 312:724). Within the ECD, human TNFα shares 97% aa sequence identity with rhesus TNFα, and 71% to 92% aa sequence identity with bovine, canine, cotton rat, equine, feline, mouse, porcine, and rat TNFα. TNFα can be prepared by standard recombinant expression methods or purchased commercially (R & D Systems, Catalog No. 210-TA, Minneapolis, Minn.).

"TNFα" can be an "antigen," which includes a molecule or a portion of the molecule capable of being bound by an anti-TNF-α drug. TNFα can have one or more than one epitope. In certain instances, TNFα will react, in a highly selective manner, with an anti-TNFα antibody. Preferred antigens that bind antibodies, fragments, and regions of anti-TNFα antibodies include at least 5 amino acids of human TNFα. In certain instances, TNFα is a sufficient length having an epitope of TNFα that is capable of binding anti-TNFα antibodies, fragments, and regions thereof.

The term "predicting responsiveness to an anti-TNFα drug" is intended to refer to an ability to assess the likelihood that treatment of a subject with an anti-TNFα drug will or will not be effective in (e.g., provide a measurable benefit to) the subject. In particular, such an ability to assess the likelihood that treatment will or will not be effective typically is exercised after treatment has begun, and an indicator of effectiveness (e.g., an indicator of measurable benefit) has been observed in the subject. Particularly preferred anti-TNFα drugs are biologic agents that have been approved by the FDA for use in humans in the treatment of TNFα-mediated diseases or disorders and include those anti-TNFα drugs described herein.

The term "size exclusion chromatography" or "SEC" includes a chromatographic method in which molecules in solution are separated based on their size and/or hydrodynamic volume. It is applied to large molecules or macromolecular complexes such as proteins and their conjugates. Typically, when an aqueous solution is used to transport the sample through the column, the technique is known as gel filtration chromatography.

The terms "complex," "immuno-complex," "conjugate," and "immunoconjugate" include, but are not limited to, TNFα bound (e.g., by non-covalent means) to an anti-TNFα drug, an anti-TNFα drug bound (e.g., by non-covalent means) to an autoantibody against the anti-TNFα drug, and an anti-TNFα drug bound (e.g., by non-covalent means) to both TNFα and an autoantibody against the anti-TNFα drug.

As used herein, an entity that is modified by the term "labeled" includes any entity, molecule, protein, enzyme, antibody, antibody fragment, cytokine, or related species that is conjugated with another molecule or chemical entity that is empirically detectable. Chemical species suitable as labels for labeled-entities include, but are not limited to, fluorescent dyes, e.g. Alexa Fluor® dyes such as Alexa Fluor® 647, quantum dots, optical dyes, luminescent dyes, and radionuclides, e.g. $^{125}$I.

The term "effective amount" includes a dose of a drug that is capable of achieving a therapeutic effect in a subject in need thereof as well as the bioavailable amount of a drug. The term "bioavailable" includes the fraction of an administered dose of a drug that is available for therapeutic activity. For example, an effective amount of a drug useful for treating diseases and disorders in which TNF-α has been implicated in the pathophysiology can be the amount that is capable of preventing or relieving one or more symptoms associated therewith.

The phrase "fluorescence label detection" includes a means for detecting a fluorescent label. Means for detection include, but are not limited to, a spectrometer, a fluorimeter, a photometer, and a detection device commonly incorporated with a chromatography instrument such as, but not limited to, size exclusion-high performance liquid chromatography, such as, but not limited to, an Agilent-1200 HPLC System.

The phrase "optimize therapy" includes optimizing the dose (e.g., the effective amount or level) and/or the type of a particular therapy. For example, optimizing the dose of an anti-TNFα drug includes increasing or decreasing the amount of the anti-TNFα drug subsequently administered to a subject. In certain instances, optimizing the type of an anti-TNFα drug includes changing the administered anti-TNFα drug from one drug to a different drug (e.g., a different anti-TNFα drug). In other instances, optimizing therapy includes coadministering a dose of an anti-TNFα drug (e.g., at an increased, decreased, or same dose as the previous dose) in combination with an immunosuppressive drug.

The term "co-administer" includes to administer more than one active agent, such that the duration of physiological effect of one active agent overlaps with the physiological effect of a second active agent.

The term "subject," "patient," or "individual" typically refers to humans, but also to other animals including, e.g., other primates, rodents, canines, felines, equines, ovines, porcines, and the like.

The term "course of therapy" includes any therapeutic approach taken to relieve or prevent one or more symptoms associated with a TNFα-mediated disease or disorder. The term encompasses administering any compound, drug, procedure, and/or regimen useful for improving the health of an individual with a TNFα-mediated disease or disorder and includes any of the therapeutic agents described herein. One skilled in the art will appreciate that either the course of therapy or the dose of the current course of therapy can be changed (e.g., increased or decreased) based upon the presence or concentration level of TNFα, anti-TNFα drug, and/or anti-drug antibody using the methods.

The term "immunosuppressive drug" or "immunosuppressive agent" includes any substance capable of producing an immunosuppressive effect, *e.g.*, the prevention or diminution of the immune response, as by irradiation or by administration of drugs such as anti-metabolites, anti-lymphocyte sera, antibodies, *etc.* Examples of immunosuppressive drugs include, without limitation, thiopurine drugs such as azathioprine (AZA) and metabolites thereof; anti-metabolites such as methotrexate (MTX); sirolimus (rapamycin); temsirolimus; everolimus; tacrolimus (FK-506); FK-778; anti-lymphocyte globulin antibodies, anti-thymocyte globulin antibodies, anti-CD3 antibodies, anti-CD4 antibodies, and antibody-toxin conjugates; cyclosporine; mycophenolate; mizoribine monophosphate; scoparone; glatiramer acetate; metabolites thereof; pharmaceutically acceptable salts thereof; derivatives thereof; prodrugs thereof; and combinations thereof.

The term "thiopurine drug" includes azathioprine (AZA), 6-mercaptopurine (6-MP), or any metabolite thereof that has therapeutic efficacy and includes, without limitation, 6-thioguanine (6-TG), 6-methylmercaptopurine riboside, 6-thioinosine nucleotides *(e.g.,* 6-thioinosine monophosphate, 6-thioinosine diphosphate, 6-thioinosine triphosphate), 6-thioguanine nucleotides *(e.g.,* 6-thioguanosine monophosphate, 6-thioguanosine diphosphate, 6-thioguanosine triphosphate), 6-thioxanthosine nucleotides *(e.g.,* 6-thioxanthosine monophosphate, 6-thioxanthosine diphosphate, 6-thioxanthosine triphosphate), derivatives thereof, analogues thereof, and combinations thereof.

The term "sample" includes any biological specimen obtained from an individual. Samples include, without limitation, whole blood, plasma, serum, red blood cells, white blood cells *(e.g.,* peripheral blood mononuclear cells (PBMC), polymorphonuclear (PMN) cells), ductal lavage fluid, nipple aspirate, lymph *(e.g.,* disseminated tumor cells of the lymph node), bone marrow aspirate, saliva, urine, stool *(i.e.,* feces), sputum, bronchial lavage fluid, tears, fine needle aspirate *(e.g.,* harvested by random periareolar fine needle aspiration), any other bodily fluid, a tissue sample such as a biopsy of a site of inflammation *(e.g.,* needle biopsy), cellular extracts thereof, and an immunoglobulin enriched fraction derived from one or more of these bodily fluids or tissues. The sample can be whole blood, a fractional component thereof such as plasma, serum, or a cell pellet, or an immunoglobulin enriched fraction thereof. One skilled in the art will appreciate that samples such as serum samples can be diluted prior to the analysis. The sample can be obtained by isolating PBMCs and/or PMN cells using any technique known in the art. The sample can be a tissue biopsy such as, *e.g.*, from a site of inflammation such as a portion of the gastrointestinal tract or synovial tissue.

The steps of the methods do not necessarily have to be performed in the particular order in which they are presented. A person of ordinary skill in the art would understand that other orderings of the steps of the methods are encompassed.

Brackets, "[ ]" indicate that the species within the brackets are referred to by their concentration.

## III. Description s

Disclosed assays for detecting and measuring the presence or level of autoantibodies to anti-TNFα drug therapeutics in a sample. The present method is useful for optimizing therapy and monitoring patients receiving anti-TNFα drug therapeutics to detect the presence or level of autoantibodies *(e.g.,* HACA and/or HAHA) against the drug. Disclosed are methods for selecting therapy, optimizing therapy, and/or reducing toxicity in subjects receiving anti-TNFα drugs for the treatment of TNFα-mediated disease or disorders.

Disclosed is a method for detecting the presence or level of an autoantibody to an anti-TNFα drug in a sample without interference from the anti-TNFα drug in the sample, the method comprising:

(a) contacting the sample with an acid to dissociate preformed complexes of the autoantibody and the anti-TNFα drug, wherein the sample has or is suspected of having an autoantibody to the anti-TNFα drug;
(b) contacting the sample with a labeled anti-TNFα drug following dissociation of the preformed complexes;
(c) neutralizing the acid in the sample to form labeled complexes *(i.e.,* immuno-complexes or conjugates) of the labeled anti-TNFα drug and the autoantibody *(i.e.,* wherein the labeled anti-TNFα drug and autoantibody are not covalently attached to each other);
(d) subjecting the labeled complexes to size exclusion chromatography to separate the labeled complexes *(e.g.,* from free labeled anti-TNFα drug); and
(e) detecting the labeled complexes, thereby detecting the presence or level of the autoantibody without interference from the anti-TNFα drug in the sample.

Without being bound by any particular theory, it is believed that acid dissociation changes the $K_d$ between the autoantibody (also known as an anti-drug antibody or ADA) and the anti-TNFα drug. In particular, it is theorized that acid dissociation disrupts the bonds between the ADA and the anti-TNFα drug. These bonds include, but are not limited to, hydrogen bonds, electrostatic bonds, Van der Waals forces, and/or hydrophobic bonds. The addition of acid increases the pH and thus the hydrogen ion concentration increases. The hydrogen ions can now compete for the previously mentioned non-covalent interactions. This competition lowers the $K_d$ between the ADA and the anti-TNFα drug.

Disclosed is that the anti-TNFα drug is selected from the group consisting of REMICADE™ (infliximab), ENBREL™ (etanercept), HUMIRA™ (adalimumab), CIMZIA® (certolizumab pegol), SIMPONI® (golimumab; CNTO 148), and combinations thereof.

Disclosed is that the anti-TNFα drug autoantibody includes, but is not limited to, human anti-chimeric antibodies (HACA), human anti-humanized antibodies (HAHA), and human anti-mouse antibodies (HAMA), as well as combinations thereof.

Disclosed is that steps (a) and (b) are performed simultaneously, *e.g.*, the sample is contacted with an acid and a labeled anti-TNFα drug at the same time. Step (b) can be performed prior to step (a), *e.g.,* the sample is first contacted with a labeled anti-TNFα drug, and then contacted with an acid. Steps (b) and (c) can be performed simultaneously, *e.g.*, the sample is contacted with a labeled anti-TNFα drug and neutralized *(e.g.,* by contacting the sample with one or more neutralizing agents) at the same time.

The sample can be contacted with an amount of an acid that is sufficient to dissociate preformed complexes of the autoantibody and the anti-TNFα drug, such that the labeled anti-TNFα drug, the unlabeled anti-TNFα drug, and the autoantibody to the anti-TNFα drug can equilibrate and form complexes therebetween.

The methods can comprise detecting the presence or level of the autoantibody without substantial interference from the anti-TNFα drug that is also present in the sample. The sample can be contacted with an amount of an acid that is sufficient to allow for the detection and/or measurement of the autoantibody in the presence of a high level of the anti-TNFα drug.

Disclosed is that the phrase "high level of an anti-TNFα drug" can include drug levels of from about 10 to about 100 μg/mL, about 20 to about 80 μg/mL, about 30 to about 70 μg/mL, or about 40 to about 80 μg/mL. The phrase "high level of an anti-TNFα drug" can include drug levels greater than or equal to about 10, 20, 30, 40, 50, 60, 70, 80, 90, or 100 μg/mL.

The acid can comprise an organic acid. The acid can comprise an inorganic acid. The acid can comprise a mixture of an organic acid and an inorganic acid. Non-limiting examples of organic acids include citric acid, isocitric acid, glutamic acid, acetic acid, lactic acid, formic acid, oxalic acid, uric acid, trifluoroacetic acid, benzene sulfonic acid, aminomethanesulfonic acid, camphor-10-sulfonic acid, chloroacetic acid, bromoacetic acid, iodoacetic acid, propanoic acid, butanoic acid, glyceric acid, succinic acid, malic acid, aspartic acid, and combinations thereof. Non-limiting examples of inorganic acids include hydrochloric acid, nitric acid, phosphoric acid, sulfuric acid, boric acid, hydrofluoric acid, hydrobromic acid, and combinations thereof.

The amount of an acid can correspond to a concentration of from about 0.01M to about 10M, about 0.1 M to about 5M, about 0.1 M to about 2M, about 0.2M to about 1 M, or about 0.25M to about 0.75M of an acid or a mixture of acids. The amount of an acid can correspond to a concentration of greater than or equal to about 0.01M, 0.05M, 0.1M, 0.2M, 0.3M, 0.4M, 0.5M, 0.6M, 0.7M, 0.8M, 0.9M, 1M, 2M, 3M, 4M, 5M, 6M, 7M, 8M, 9M, or 10M of an acid or a mixture of acids. The pH of the acid can be, for example, about 0.1, 0.5, 1.0, 1.5, 2.0, 2.5, 3.0, 3.5, 4.0, 4.5, 5.0, 5.5, 6.0, or 6.5.

The sample can be contacted with an acid an amount of time that is sufficient to dissociate preformed complexes of the autoantibody and the anti-TNFα drug. In certain instances, the sample is contacted *(e.g.,* incubated) with an acid for a period of time ranging from about 0.1 hours to about 24 hours, about 0.2 hours to about 16 hours, about 0.5 hours to about 10 hours, about 0.5 hours to about 5 hours, or about 0.5 hours to about 2 hours. In other instances, the sample is contacted *(e.g.,* incubated) with an acid for a period of time that is greater than or equal to about 0.1, 0.2, 0.3, 0.4, 0.5, 0.6, 0.7, 0.8, 0.9, 1, 1.5, 2, 2.5, 3, 3.5, 4, 4.5, 5, 6, 7, 8, 9, or 10 hours. The sample can be contacted with an acid at 4°C, room temperature (RT), or 37°C.

The step of neutralizing the acid can comprise raising the pH of the sample to allow the formation of complexes between the labeled anti-TNFα drug and the autoantibody to the anti-TNFα drug as well as complexes between unlabeled anti-TNFα drug and the autoantibody. The acid can be neutralized by the addition of one or more neutralizing agents such as, for example, strong bases, weak bases, buffer solutions, and combinations thereof. One skilled in the art will appreciate that neutralization reactions do not necessarily imply a resultant pH of 7. In some instances, acid neutralization results in a sample that is basic. In other instances, acid neutralization results in a sample that is acidic (but higher than the pH of the sample prior to adding the neutralizing agent). The neutralizing agent can comprise a buffer such as phosphate buffered saline *(e.g.*, 10x PBS) at a pH of about 7.3.

Step (b) can further comprise contacting an internal control with the sample together with a labeled anti-TNFα drug *(e.g.,* before, during, or after dissociation of the preformed complexes). In certain instances, the internal control comprises a labeled internal control such as, *e.g.*, Biocytin-Alexa 488. In certain other instances, the amount of the labeled internal control ranges from about 1 ng to about 25 ng, about 5 ng to about 25 ng, about 5 ng to about 20 ng, about 1 ng to about 20 ng, about 1 ng to about 10 ng, or about 1 ng to about 5 ng per 100 μL of sample analyzed. In further instances, the amount of the labeled internal control is greater than or equal to about 1 ng, 5 ng, 10 ng, 15 ng, 20 ng, or 25 ng per 100 μL of sample analyzed.

As one non-limiting example of the methods, samples such as serum samples *(e.g.*, serum from subjects receiving therapy with an anti-TNFα drug such as Remicade (IFX)) can be incubated with 0.5M citric acid, pH 3.0 for one hour at room temperature. Following the dissociation of preformed complexes between (unlabeled) anti-TNFα drug and autoantibodies to the anti-TNFα drug (e.g., anti-drug antibodies such as anti-IFX antibodies (ATI)), labeled anti-TNFα drug (e.g., IFX-Alexa 488) and an internal control can be added and the reaction mixture and (e.g., immediately) neutralized with a neutralizing agens such as 10x PBS, pH 7.3. After neutralization, the reaction mixture can be incubated for another hour at room temperature (e.g., on a plate shaker) to allow equilibration and to complete the reformation of immune complexes between either the labeled or unlabeled anti-TNFα drug and the anti-drug antibody. The samples can then be filtered and analyzed by SEC-HPLC as described herein.

The methods (e.g., comprising acid dissociation followed by homogeneous solution phase binding kinetics) can significantly increase the IFX drug tolerance such that the ATI can be measured in the presence of IFX up to about 60 μg/mL. See, Example 14 and Figures 27-28. In other words, the methods can detect the presence or level of autoantibodies to anti-TNFα drugs such as ATI as well as autoantibodies to other anti-TNFα drugs in the presence of high levels of anti-TNFα drugs (e.g., IFX), but without substantial interference therefrom.

Disclosed is a method for optimizing therapy and/or reducing toxicity to an anti-TNFα drug in a subject receiving a course of therapy with the anti-TNFα drug, the method comprising:

(a) detecting the presence or level of an autoantibody to the anti-TNFα drug in a sample from the subject without interference from the anti-TNFα drug in the sample, the method comprising:

(i) contacting the sample with an acid to dissociate preformed complexes of the autoantibody and the anti-TNFα drug, wherein the sample has or is suspected of having an autoantibody to the anti-TNFα drug;
(ii) contacting the sample with a labeled anti-TNFα drug following dissociation of the preformed complexes;
(iii) neutralizing the acid in the sample to form labeled complexes (i.e., immuno-complexes or conjugates) of the labeled anti-TNFα drug and the autoantibody (i.e., wherein the labeled anti-TNFα drug and autoantibody are not covalently attached to each other);
(iv) subjecting the labeled complexes to size exclusion chromatography to separate the labeled complexes (e.g., from free labeled anti-TNFα drug); and
(v) detecting the labeled complexes (e.g., thereby detecting the presence or level of the autoantibody without interference from the anti-TNFα drug in the sample); and

(b) determining a subsequent dose of the course of therapy for the subject or whether a different course of therapy should be administered to the subject based upon the presence or level of the autoantibody,

thereby optimizing therapy and/or reducing toxicity to the anti-TNFα drug.

The subsequent dose of the course of therapy can be increased, decreased, or maintained based upon the presence or level of the autoantibody. As a non-limiting example, a subsequent dose of the course of therapy is decreased when a high level of the autoantibody is detected in the sample. The different course of therapy can comprise a different anti-TNFα drug, the current course of therapy along with an immunosuppressive agent, or switching to a course of therapy that is not an anti-TNFα drug (e.g., discontinuing use of an anti-TNFα therapeutic antibody). As a non-limiting example, a different course of therapy is administered when a high level of the autoantibody is detected in the sample.

Steps (i) and (ii) can be performed simultaneously, e.g., the sample is contacted with an acid and a labeled anti-TNFα drug at the same time. Step (ii) can be performed prior to step (i), e.g., the sample is first contacted with a labeled anti-TNFα drug, and then contacted with an acid. Steps (ii) and (iii) can be performed simultaneously, e.g., the sample is contacted with a labeled anti-TNFα drug and neutralized (e.g., by contacting the sample with one or more neutralizing agents) at the same time.

An anti-TNFα drug can be labeled with any of a variety of detectable group(s). An anti-TNFα drug can be labeled with a fluorophore or a fluorescent dye. Non-limiting examples of fluorophores or fluorescent dyes include those listed in the Molecular Probes Catalogue (see, R. Haugland, The Handbook-A Guide to Fluorescent Probes and Labeling Technologies, 10th Edition, Molecular probes, Inc. (2005)). Such exemplary fluorophores or fluorescent dyes include, but are not limited to, Alexa Fluor® dyes such as Alexa Fluor® 350, Alexa Fluor® 405, Alexa Fluor® 430, Alexa Fluor® 488, Alexa Fluor® 514, Alexa Fluor® 532, Alexa Fluor® 546, Alexa Fluor® 555, Alexa Fluor® 568, Alexa Fluor® 594, Alexa Fluor® 610, Alexa Fluor® 633, Alexa Fluor® 635, Alexa Fluor® 647, Alexa Fluor® 660, Alexa Fluor® 680, Alexa Fluor® 700, Alexa Fluor® 750, and/or Alexa Fluor® 790, as well as other fluorophores including, but not limited to, Dansyl Chloride (DNS-Cl), 5-(iodoacetamida)fluoroscein (5-IAF), fluoroscein 5-isothiocyanate (FITC), tetramethylrhodamine 5-(and 6-)isothiocyanate (TRITC), 6-acryloyl-2-dimethylaminonaphthalene (acrylodan), 7-nitrobenzo-2-oxa-1,3,-diazol-4-yl chloride (NBD-Cl), ethidium bromide, Lucifer Yellow, 5-carboxyrhodamine 6G hydrochloride, Lissamine rhodamine B sulfonyl chloride, Texas Red™ sulfonyl chloride, BODIPY™, naphthalamine sulfonic acids (e.g., 1-anilinonaphthalene-8-sulfonic acid (ANS), 6-(p-toluidinyl)naphthalen-e-2-sulfonic acid (TNS), and the like), Anthroyl fatty acid, DPH, Parinaric acid, TMA-DPH, Fluorenyl fatty acid, fluorescein-phosphatidylethanolamine, Texas Red-phosphatidylethanolamine, Pyrenyl-phophatidylcholine, Fluorenyl-phosphotidylcholine, Merocyanine 540,l-(3-sulfonatopropyl)-4-[β-[2[(di-n-butylamino)-6 naphthyl]vinyl]pyridinium betaine (Naphtyl Styryl), 3,3'dipropylthiadicarbocyanine (diS-C$_3$-(5)), 4-(p-dipentyl aminostyryl)-1-methylpyridinium (di-5-ASP), Cy-3 Iodo Acetamide, Cy-5-N-Hydroxysuccinimide, Cy-7-Isothiocyanate, rhodamine 800, IR-125, Thiazole Orange, Azure B, Nile Blue, Al Phthalocyanine, Oxaxine 1, 4', 6-diamidino-2-phenylindole (DAPI), Hoechst 33342, TOTO, Acridine Orange, Ethidium Homodimer, N(ethoxycarbonylmethyl)-6-methoxyquinolinium (MQAE), Fura-2, Calcium Green, Carboxy SNARF-6, BAPTA, coumarin, phytofluors, Coronene, metal-ligand complexes, IRDye® 700DX, IRDye® 700, IRDye® 800RS, IRDye® 800CW, IRDye® 800, Cy5, Cy5.5, Cy7, DY 676, DY680, DY682, DY780, and mixtures thereof. Additional suitable fluorophores include enzyme-cofactors; lanthanide, green fluorescent protein, yellow fluorescent protein, red fluorescent protein, or mutants and derivates thereof. The second member of the specific binding pair can have a detectable group attached thereto.

Typically, the fluorescent group is a fluorophore selected from the category of dyes comprising polymethines, pthalocyanines, cyanines, xanthenes, fluorenes, rhodamines, coumarins, fluoresceins and BODIPY™.

The fluorescent group can be a near-infrared (NIR) fluorophore that emits in the range of between about 650 to about 900 nm. Use of near infrared fluorescence technology is advantageous in biological assays as it substantially eliminates or reduces background from auto fluorescence of biosubstrates. Another benefit to the near-IR fluorescent technology is that the scattered light from the excitation source is greatly reduced since the scattering intensity is proportional to the inverse fourth power of the wavelength. Low background fluorescence and low scattering result in a high signal to noise ratio, which is essential for highly sensitive detection. Furthermore, the optically transparent window in the near-IR region (650 nm to 900 nm) in biological tissue makes NIR fluorescence a valuable technology *for in vivo* imaging and subcellular detection applications that require the transmission of light through biological components. The fluorescent group is preferably selected form the group consisting of IRDye® 700DX, IRDye® 700, IRDye® 800RS, IRDye® 800CW, IRDye® 800, Alexa Fluor® 660, Alexa Fluor® 680, Alexa Fluor® 700, Alexa Fluor® 750, Alexa Fluor® 790, Cy5, Cy5.5, Cy7, DY 676, DY680, DY682, and DY780. The near infrared group can be IRDye® 800CW, IRDye® 800, IRDye® 700DX, IRDye® 700, or Dynomic DY676.

Fluorescent labeling is accomplished using a chemically reactive derivative of a fluorophore. Common reactive groups include amine reactive isothiocyanate derivatives such as FITC and TRITC (derivatives of fluorescein and rhodamine), amine reactive succinimidyl esters such as NHS-fluorescein, and sulfhydryl reactive maleimide activated fluors such as fluorescein-5-maleimide, many of which are commercially available. Reaction of any of these reactive dyes with an anti-TNFα drug results in a stable covalent bond formed between a fluorophore and an anti-TNFα drug.

In certain instances, following a fluorescent labeling reaction, it is often necessary to remove any nonreacted fluorophore from the labeled target molecule. This is often accomplished by size exclusion chromatography, taking advantage of the size difference between fluorophore and labeled protein.

Reactive fluorescent dyes are available from many sources. They can be obtained with different reactive groups for attachment to various functional groups within the target molecule. They are also available in labeling kits that contain all the components to carry out a labeling reaction. In one preferred aspect, Alexa Fluor® 647 C2 maleimide is used from Invitrogen (Cat. No. A-20347).

Specific immunological binding of an anti-drug antibody (ADA) to an anti-TNFα drug can be detected directly or indirectly. Direct labels include fluorescent or luminescent tags, metals, dyes, radionuclides, and the like, attached to the antibody. In certain instances, an anti-TNFα drug that is labeled with iodine-125 ($^{125}$I) can be used for determining the concentration levels of ADA in a sample. In other instances, a chemiluminescence assay using a chemiluminescent anti-TNFα drug that is specific for ADA in a sample is suitable for sensitive, non-radioactive detection of ADA concentration levels. In particular instances, an anti-TNFα drug that is labeled with a fluorochrome is also suitable for determining the concentration levels of ADA in a sample. Examples of fluorochromes include, without limitation, Alexa Fluor® dyes, DAPI, fluorescein, Hoechst 33258, R-phycocyanin, B-phycoerythrin, R-phycoerythrin, rhodamine, Texas red, and lissamine. Secondary antibodies linked to fluorochromes can be obtained commercially, *e.g.*, goat F(ab')$_2$ anti-human IgG-FITC is available from Tago Immunologicals (Burlingame, CA).

Indirect labels include various enzymes well-known in the art, such as horseradish peroxidase (HRP), alkaline phosphatase (AP), β-galactosidase, urease, and the like. A horseradish-peroxidase detection system can be used, for example, with the chromogenic substrate tetramethylbenzidine (TMB), which yields a soluble product in the presence of hydrogen peroxide that is detectable at 450 nm. An alkaline phosphatase detection system can be used with the chromogenic substrate p-nitrophenyl phosphate, for example, which yields a soluble product readily detectable at 405 nm. Similarly, a β-galactosidase detection system can be used with the chromogenic substrate o-nitrophenyl-β-D-galactopyranoside (ONPG), which yields a soluble product detectable at 410 nm. An urease detection system can be used with a substrate such as urea-bromocresol purple (Sigma Immunochemicals; St. Louis, MO). A useful secondary antibody linked to an enzyme can be obtained from a number of commercial sources, *e.g.*, goat F(ab')$_2$ anti-human IgG-alkaline phosphatase can be purchased from Jackson ImmunoResearch (West Grove, PA.).

A signal from the direct or indirect label can be analyzed, for example, using a spectrophotometer to detect color from a chromogenic substrate; a radiation counter to detect radiation such as a gamma counter for detection of $^{125}$I; or a fluorometer to detect fluorescence in the presence of light of a certain wavelength. For detection of enzyme-linked antibodies, a quantitative analysis of ADA levels can be made using a spectrophotometer such as an EMAX Microplate Reader (Molecular Devices; Menlo Park, CA) in accordance with the manufacturer's instructions. If desired, the assays can be automated or performed robotically, and the signal from multiple samples can be detected simultaneously.

Size exclusion chromatography can be used. The underlying principle of SEC is that particles of different sizes will elute (filter) through a stationary phase at different rates. This results in the separation of a solution of particles based on size. Provided that all the particles are loaded simultaneously or near simultaneously, particles of the same size elute together. Each size exclusion column has a range of molecular weights that can be separated. The exclusion limit defines the molecular weight at the upper end of this range and is where molecules are too large to be trapped in the stationary phase. The permeation limit defines the molecular weight at the lower end of the range of separation and is where molecules of a small enough size can penetrate into the pores of the stationary phase completely and all molecules below this molecular mass are so small that they elute as a single band.

In certain aspects, the eluent is collected in constant volumes, or fractions. The more similar the particles are in size, the more likely they will be in the same fraction and not detected separately. Preferably, the collected fractions are examined by spectroscopic techniques to determine the concentration of the particles eluted. Typically, the spectroscopy detection techniques useful in the present method include, but are not limited to, fluorometry, refractive index (RI), and ultraviolet (UV). In certain instances, the elution volume decreases roughly linearly with the logarithm of the molecular hydrodynamic volume *(i.e.,* heaver moieties come off first).

Disclosed is a kit for detecting the presence or level of an autoantibody to an anti-TNF$\alpha$ drug in a sample. The kit can comprise one or more of the following components: an acid (or mixture of acids), a labeled anti-TNF$\alpha$ drug *(e.g.,* a labeled anti-TNF$\alpha$ antibody), a labeled internal control, a neutralizing agent (or mixtures thereof), means for detection *(e.g.,* a fluorescence detector), a size exclusion-high performance liquid chromatography (SE-HPLC) instrument, and/or instructions for using the kit.

Disclosed is also a method for selecting a course of therapy *(e.g.,* selecting an appropriate anti-TNF$\alpha$ drug) for the treatment of a TNF$\alpha$-mediated disease or disorder in a subject, the method comprising:

(a) analyzing a sample obtained from the subject to determine the presence, level, or genotype of one or more markers in the sample;
(b) applying a statistical algorithm to the presence, level, or genotype of the one or more markers determined in step (a) to generate a disease activity/severity index; and
(c) selecting an appropriate course of therapy *(e.g.,* anti-TNF$\alpha$ therapy) for the subject based upon the disease activity/severity index.

A method is disclosed for optimizing therapy and/or reducing toxicity in a subject receiving a course of therapy for the treatment of a TNF$\alpha$-mediated disease or disorder, the method comprising:

(a) analyzing a sample obtained from the subject to determine the presence, level, or genotype of one or more markers in the sample;
(b) applying a statistical algorithm to the presence, level, or genotype of the one or more markers determined in step (a) to generate a disease activity/severity index; and
(c) determining a subsequent dose of the course of therapy for the subject or whether a different course of therapy should be administered to the subject based upon the disease activity/severity index.

The course of therapy can comprise an anti-TNF$\alpha$ antibody. In certain instances, the anti-TNF$\alpha$ antibody is a member selected from the group consisting of REMICADE™ (infliximab), ENBREL™ (etanercept), HUMIRA™ (adalimumab), CIMZIA® (certolizumab pegol), SIMPONI® (golimumab; CNTO 148), and combinations thereof. The course of therapy can comprise an anti-TNF$\alpha$ antibody along with an immunosuppressive agent.

The level of one or more markers can comprise a total level, an activation level, or combinations thereof. In particular instances, the one or more markers is a member selected from the group consisting of an inflammatory marker, a growth factor, a serology marker, a cytokine and/or chemokine, a marker of oxidative stress, a cell surface receptor, a signaling pathway marker, a genetic marker, an anti-TNF$\alpha$ antibody, an anti-drug antibody (ADA), and combinations thereof.

In some instances, the inflammatory marker is a member selected from the group consisting of CRP, SAA, VCAM, ICAM, calprotectin, lactoferrin, IL-8, Rantes, TNF$\alpha$, IL-6, IL-1 $\beta$, S100A12, M2-pyruvate kinase (PK), IFN, IL-2, TGF, IL-13, IL-15, IL-12, and combinations thereof. In other instances, the growth factor is a member selected from the group consisting of GM-CSF, VEGF, EGF, keratinocyte growth factor (KGF; FGF7), and combinations thereof. In yet other instances, the serology marker is a member selected from the group consisting of an anti-neutrophil antibody, an anti-microbial antibody, an anti-*Saccharomyces cerevisiae* antibody, and combinations thereof. In further instances, the cytokine is a member selected from the group consisting of TNFα, IL-6, IL-1β, IFN-γ, IL-10, and combinations thereof. In other instances, the cell surface receptor is CD64. In yet other instances, the signaling pathway marker is a signal transduction molecule. In other instances, the genetic marker is a mutation in an inflammatory pathway gene.

Step (a) can comprise determining the presence, level, and/or genotype of at least two, three, four, five, six, seven, eight, nine, ten, fifteen, twenty, thirty, forty, fifty, or more markers in the sample. In certain instances, the sample is selected from the group consisting of serum, plasma, whole blood, stool, peripheral blood mononuclear cells (PBMC), polymorphonuclear (PMN) cells, and a tissue biopsy.

The statistical algorithm can comprise a learning statistical classifier system. In some instances, the learning statistical classifier system is selected from the group consisting of a random forest, classification and regression tree, boosted tree, neural network, support vector machine, general chi-squared automatic interaction detector model, interactive tree, multiadaptive regression spline, machine learning classifier, and combinations thereof. In certain instances, the statistical algorithm comprises a single learning statistical classifier system. In certain other instances, the statistical algorithm comprises a combination of at least two learning statistical classifier systems. In some instances, the at least two learning statistical classifier systems are applied in tandem. Non-limiting examples of statistical algorithms and analysis suitable for use in the method are described in International Application No. PCT/US2011/056777, filed October 18, 2011.

The method further can comprise sending the results from the selection or determination of step (c) to a clinician. Step (c) can comprise selecting an initial course of therapy for the subject.

Step (b) can further comprise applying a statistical algorithm to the presence, level, or genotype of one or more markers determined at an earlier time during the course of therapy to generate an earlier disease activity/severity index. In some instances, the earlier disease activity/severity index is compared to the disease activity/severity index generated in step (b) to determine a subsequent dose of the course of therapy or whether a different course of therapy should be administered. The subsequent dose of the course of therapy can be increased, decreased, or maintained based upon the disease activity/severity index generated in step (b). In some instances, the different course of therapy comprises a different anti-TNFα antibody. In other instances, the different course of therapy comprises the current course of therapy along with an immunosuppressive agent.

Methods for detecting anti-TNFα antibodies and anti-drug antibodies (ADA) are described herein and in PCT Publication No. WO 2011/056590. In particular , the presence or level of anti-drug antibodies can be determined in accordance with the methods comprising an acid dissociation step by contacting a sample with an acid prior to, during, and/or after contacting the sample with a labeled anti-TNFα drug.

Disclosed is also a method for predicting the course of a TNFα-mediated disease or disorder in a subject, the method comprising:

(a) analyzing a sample obtained from the subject to determine the presence, level, or genotype of one or more markers in the sample;
(b) applying a statistical algorithm to the presence, level, or genotype of the one or more markers determined in step (a) to generate a disease activity/severity index; and
(c) predicting the course of the TNFα-mediated disease or disorder based upon the disease activity/severity index generated in step (b).

Step (b) can further comprise applying a statistical algorithm to the presence, level, or genotype of one or more of the markers determined at an earlier time to generate an earlier disease activity/severity index. In certain instances, the earlier disease activity/severity index is compared to the disease activity/severity index generated in step (b) to predict the course of the TNFα-mediated disease or disorder.

Once the diagnosis or prognosis of a subject receiving anti-TNFα drug therapy has been determined or the likelihood of response to an anti-TNFα drug has been predicted in a subject diagnosed with a disease and disorder in which TNFα has been implicated in the pathophysiology, *e.g.*, but not limited to, shock, sepsis, infections, autoimmune diseases, RA, Crohn's disease, transplant rejection and graft-versus-host disease, according to the methods described herein, the present method may further comprise recommending a course of therapy based upon the diagnosis, prognosis, or prediction. -Disclosed is that the method may further comprise administering to a subject a therapeutically effective amount of an anti-TNFα drug useful for treating one or more symptoms associated with the TNFα-mediated disease or disorder. For therapeutic applications, the anti-TNFα drug can be administered alone or co-administered in combination with one or more additional anti-TNFα drugs and/or one or more drugs that reduce the side-effects associated with the anti-TNFα drug (e.g., an immunosuppressive agent). As such, the present method advantageously enables a clinician to practice "personalized medicine" by guiding treatment decisions and informing therapy selection and optimization for anti-TNFα drugs such that the right drug is given to the right patient at the right time.

## IV. Disease Activity/Severity Index

In certain aspects, the present method provides an algorithmic-based analysis of one or a plurality of *(e.g.,* two, three, four, five, six, seven, or more) biomarkers to improve the accuracy of selecting therapy, optimizing therapy, reducing toxicity, and/or monitoring the efficacy of therapeutic treatment to anti-TNF$\alpha$ drug therapy.

As a non-limiting example, the disease activity/severity index can comprise detecting, measuring, or determining the presence, level (concentration *(e.g.,* total) and/or activation *(e.g.,* phosphorylation)), or genotype of one or more specific biomarkers in one or more of the following categories of biomarkers:

(1) Inflammatory markers
(2) Growth factors
(3) Serology *(e.g.,* immune markers)
(4) Cytokines and chemokines
(5) Markers of oxidative stress
(6) Cell surface receptors *(e.g.,* CD64, others)
(7) Signaling pathways
(8) Other markers *(e.g.,* genetic markers such as inflammatory pathway genes).

The presence and/or level of one or both of the following markers can also be detected, measured, or determined in a patient sample *(e.g.,* a serum sample from a patient on anti-TNF$\alpha$ drug therapy): (9) anti-TNF$\alpha$ drug levels *(e.g.,* levels of free anti-TNF$\alpha$ therapeutic antibody); and/or (10) anti-drug antibody (ADA) levels *(e.g.,* levels of autoantibody to the anti-TNF$\alpha$ drug).

A single statistical algorithm or a combination of two or more statistical algorithms described herein can then be applied to the presence, concentration level, activation level, or genotype of the markers detected, measured, or determined in the sample to thereby select therapy, optimize therapy, reduce toxicity, or monitor the efficacy of therapeutic treatment with an anti-TNF$\alpha$ drug. As such, the methods find utility in determining patient management by determining patient immune status.

Understanding the clinical course of disease will enable physicians to make better informed treatment decisions for their inflammatory disease patients *(e.g.,* IBD *(e.g.,* Crohn's disease), rheumatoid arthritis (RA), others) and may help to direct new drug development in the future. The ideal biomarker(s) for use in the disease activity/severity index described herein should be able to identify individuals at risk for the disease and should be disease-specific. Moreover, the biomarker(s) should be able to detect disease activity and monitor the effect of treatment; and should have a predictive value towards relapse or recurrence of the disease. Predicting disease course, however, has now been expanded beyond just disease recurrence, but perhaps more importantly to include predictors of disease complications including surgery. The present method is particularly advantageous because it provides indicators of disease activity and/or severity and enables a prediction of the risk of relapse in those patients in remission. In addition, the biomarkers and disease activity/severity index of present method have enormous implications for patient management as well as therapeutic decision-making and would aid or assist in directing the appropriate therapy to those patients who would most likely benefit from it and avoid the expense and potential toxicity of chronic maintenance therapy in those who have a low risk of recurrence.

## A. Inflammatory Markers

Although disease course of an inflammatory disease is typically measured in terms of inflammatory activity by noninvasive tests using white blood cell count, this method has a low specificity and shows limited correlation with disease activity.

As such, a variety of inflammatory markers, including biochemical markers, serological markers, protein markers, genetic markers, and other clinical or echographic characteristics, are particularly useful in the methods for selecting therapy, optimizing therapy, reducing toxicity, and/or monitoring the efficacy of therapeutic treatment with one or more therapeutic agents such as biologics *(e.g.,* anti-TNF$\alpha$ drugs). In certain aspects, the methods described herein utilize the application of an algorithm *(e.g.,* statistical analysis) to the presence, concentration level, and/or genotype determined for one or more inflammatory markers *(e.g.,* alone or in combination with biomarkers from other categories) to aid or assist in predicting disease course, selecting an appropriate anti-TNF$\alpha$ drug therapy, optimizing anti-TNF$\alpha$ drug therapy, reducing toxicity associated with anti-TNF$\alpha$ drug therapy, or monitoring the efficacy of therapeutic treatment with an anti-TNF$\alpha$ drug.

Non-limiting examples of inflammatory markers suitable for use in the present method include biochemical, serological, and protein markers such as, *e.g.,* cytokines, chemokines, acute phase proteins, cellular adhesion molecules, S100 proteins, and/or other inflammatory markers.

## 1. Cytokines and Chemokines

The determination of the presence or level of at least one cytokine or chemokine in a sample is particularly useful in the present method. As used herein, the term "cytokine" includes any of a variety of polypeptides or proteins secreted by immune cells that regulate a range of immune system functions and encompasses small cytokines such as chemokines. The term "cytokine" also includes adipocytokines, which comprise a group of cytokines secreted by adipocytes that function, for example, in the regulation of body weight, hematopoiesis, angiogenesis, wound healing, insulin resistance, the immune response, and the inflammatory response.

In certain aspects, the presence or level of at least one cytokine including, but not limited to, TNF$\alpha$, TNF-related weak inducer of apoptosis (TWEAK), osteoprotegerin (OPG), IFN-$\alpha$, IFN-$\beta$, IFN-$\gamma$, IL-1$\alpha$, IL-1$\beta$, IL-1 receptor antagonist (IL-1ra), IL-2, IL-4, IL-5, IL-6, soluble IL-6 receptor (sIL-6R), IL-7, IL-8, IL-9, IL-10, IL-12, IL-13, IL-15, IL-17, IL-23, and IL-27 is determined in a sample. In certain other aspects, the presence or level of at least one chemokine such as, for example, CXCL1/GRO1/GROa, CXCL2/GRO2, CXCL3/GRO3, CXCL4/PF-4, CXCL5/ENA-78, CXCL6/GCP-2, CXCL7/NAP-2, CXCL9/MIG, CXCL10/IP-10, CXCL11/I-TAC, CXCL12/SDF-1, CXCL13/BCA-1, CXCL14/BRAK, CXCL15, CXCL16, CXCL17/DMC, CCL1, CCL2/MCP-1, CCL3/MIP-1$\alpha$, CCL4/MIP-1$\beta$, CCL5/RANTES, CCL6/C10, CCL7/MCP-3, CCL8/MCP-2, CCL9/CCL10, CCL11/Eotaxin, CCL12/MCP-5, CCL13/MCP-4, CCL14/HCC-1, CCL15/MIP-5, CCL16/LEC, CCL17/TARC, CCL18/MIP-4, CCL19/MIP-3$\beta$, CCL20/MIP-3$\alpha$, CCL21/SLC, CCL22/MDC, CCL23/MPIF1, CCL24/Eotaxin-2, CCL25/TECK, CCL26/Eotaxin-3, CCL27/CTACK, CCL28/MEC, CL1, CL2, and CX$_3$CL1 is determined in a sample. In certain further aspects, the presence or level of at least one adipocytokine including, but not limited to, leptin, adiponectin, resistin, active or total plasminogen activator inhibitor-1 (PAI-1), visfatin, and retinol binding protein 4 (RBP4) is determined in a sample. Preferably, the presence or level of TNF$\alpha$, IL-6, IL-8, IL-1$\beta$, IL-2, IL-12, IL-13, IL-15, IFN (e.g., IFN-$\alpha$, IFN-$\beta$, IFN-$\gamma$), IL-10, CCL5/RANTES, and/or other cytokines or chemokines is determined.

In certain instances, the presence or level of a particular cytokine or chemokine is detected at the level of mRNA expression with an assay such as, for example, a hybridization assay or an amplification-based assay. In certain other instances, the presence or level of a particular cytokine or chemokine is detected at the level of protein expression using, for example, an immunoassay (e.g., ELISA) or an immunohistochemical assay. Suitable ELISA kits for determining the presence or level of a cytokine or chemokine of interest in a serum, plasma, saliva, or urine sample are available from, e.g., R&D Systems, Inc. (Minneapolis, MN), Neogen Corp. (Lexington, KY), Alpco Diagnostics (Salem, NH), Assay Designs, Inc. (Ann Arbor, MI), BD Biosciences Pharmingen (San Diego, CA), Invitrogen (Camarillo, CA), Calbiochem (San Diego, CA), CHEMICON International, Inc. (Temecula, CA), Antigenix America Inc. (Huntington Station, NY), QIAGEN Inc. (Valencia, CA), Bio-Rad Laboratories, Inc. (Hercules, CA), and/or Bender MedSystems Inc. (Burlingame, CA).

The human IL-6 polypeptide sequence is set forth in, e.g., Genbank Accession No. NP_000591. The human IL-6 mRNA (coding) sequence is set forth in, e.g., Genbank Accession No. NM_000600. One skilled in the art will appreciate that IL-6 is also known as interferon beta 2 (IFNB2), HGF, HSF, and BSF2.

The human IL-1$\beta$ polypeptide sequence is set forth in, e.g., Genbank Accession No. NP_000567. The human IL-1$\beta$ mRNA (coding) sequence is set forth in, e.g., Genbank Accession No. NM_000576. One skilled in the art will appreciate that IL-1$\beta$ is also known as IL 1 F2 and IL-1 beta.

The human IL-8 polypeptide sequence is set forth in, e.g., Genbank Accession No. NP_000575 (SEQ ID NO:1). The human IL-8 mRNA (coding) sequence is set forth in, e.g., Genbank Accession No. NM_000584 (SEQ ID NO:2). One skilled in the art will appreciate that IL-8 is also known as CXCL8, K60, NAF, GCP1, LECT, LUCT, NAP1, 3-10C, GCP-1, LYNAP, MDNCF, MONAP, NAP-1, SCYB8, TSG-1, AMCF-I, and b-ENAP.

The human TWEAK polypeptide sequence is set forth in, e.g., Genbank Accession Nos. NP_003800 and AAC51923. The human TWEAK mRNA (coding) sequence is set forth in, e.g., Genbank Accession Nos. NM_003809 and BC104420. One skilled in the art will appreciate that TWEAK is also known as tumor necrosis factor ligand superfamily member 12 (TNFSF12), APO3 ligand (APO3L), CD255, DR3 ligand, growth factor-inducible 14 (Fn14) ligand, and UNQ181/PR0207.

## 2. Acute Phase Proteins

The determination of the presence or level of one or more acute-phase proteins in a sample is also useful in the present method. Acute-phase proteins are a class of proteins whose plasma concentrations increase (positive acute-phase proteins) or decrease (negative acute-phase proteins) in response to inflammation. This response is called the acute-phase reaction (also called acute-phase response). Examples of positive acute-phase proteins include, but are not limited to, C-reactive protein (CRP), D-dimer protein, mannose-binding protein, alpha 1-antitrypsin, alpha 1-antichymotrypsin, alpha 2-macroglobulin, fibrinogen, prothrombin, factor VIII, von Willebrand factor, plasminogen, complement factors, ferritin, serum amyloid P component, serum amyloid A (SAA), orosomucoid (alpha 1-acid glycoprotein, AGP), ceruloplasmin, haptoglobin, and combinations thereof. Non-limiting examples of negative acute-phase proteins include albumin, transferrin, transthyretin, transcortin, retinol-binding protein, and combinations thereof. Preferably, the presence or level of CRP and/or SAA is determined.

In certain instances, the presence or level of a particular acute-phase protein is detected at the level of mRNA expression with an assay such as, for example, a hybridization assay or an amplification-based assay. In certain other instances, the presence or level of a particular acute-phase protein is detected at the level of protein expression using, for example, an immunoassay (*e.g.*, ELISA) or an immunohistochemical assay. For example, a sandwich colorimetric ELISA assay available from Alpco Diagnostics (Salem, NH) can be used to determine the level of CRP in a serum, plasma, urine, or stool sample. Similarly, an ELISA kit available from Biomeda Corporation (Foster City, CA) can be used to detect CRP levels in a sample. Other methods for determining CRP levels in a sample are described in, *e.g.*, U.S. Patent Nos. 6,838,250 and 6,406,862; and U.S. Patent Publication Nos. 20060024682 and 20060019410. Additional methods for determining CRP levels include, *e.g.*, immunoturbidimetry assays, rapid immunodiffusion assays, and visual agglutination assays. Suitable ELISA kits for determining the presence or level of SAA in a sample such as serum, plasma, saliva, urine, or stool are available from, *e.g.*, Antigenix America Inc. (Huntington Station, NY), Abazyme (Needham, MA), USCN Life (Missouri City, TX), and/or U.S. Biological (Swampscott, MA).

C-reactive protein (CRP) is a protein found in the blood in response to inflammation (an acute-phase protein). CRP is typically produced by the liver and by fat cells (adipocytes). It is a member of the pentraxin family of proteins. The human CRP polypeptide sequence is set forth in, *e.g.*, Genbank Accession No. NP_000558. The human CRP mRNA (coding) sequence is set forth in, *e.g.*, Genbank Accession No. NM_000567. One skilled in the art will appreciate that CRP is also known as PTX1, MGC88244, and MGC149895.

Serum amyloid A (SAA) proteins are a family of apolipoproteins associated with high-density lipoprotein (HDL) in plasma. Different isoforms of SAA are expressed constitutively (constitutive SAAs) at different levels or in response to inflammatory stimuli (acute phase SAAs). These proteins are predominantly produced by the liver. The conservation of these proteins throughout invertebrates and vertebrates suggests SAAs play a highly essential role in all animals. Acute phase serum amyloid A proteins (A-SAAs) are secreted during the acute phase of inflammation. The human SAA polypeptide sequence is set forth in, *e.g.*, Genbank Accession No. NP_000322. The human SAA mRNA (coding) sequence is set forth in, *e.g.*, Genbank Accession No. NM_000331. One skilled in the art will appreciate that SAA is also known as PIG4, TP53I4, MGC111216, and SAA1.

## 3. Cellular Adhesion Molecules (IgSF CAMs)

The determination of the presence or level of one or more immunoglobulin superfamily cellular adhesion molecules in a sample is also useful in the present method. As used herein, the term "immunoglobulin superfamily cellular adhesion molecule" (IgSF CAM) includes any of a variety of polypeptides or proteins located on the surface of a cell that have one or more immunoglobulin-like fold domains, and which function in intercellular adhesion and/or signal transduction. In many cases, IgSF CAMs are transmembrane proteins. Non-limiting examples of IgSF CAMs include Neural Cell Adhesion Molecules (NCAMs; *e.g.*, NCAM-120, NCAM-125, NCAM-140, NCAM-145, NCAM-180, NCAM-185, *etc.*), Intercellular Adhesion Molecules (ICAMs, *e.g.*, ICAM-1, ICAM-2, ICAM-3, ICAM-4, and ICAM-5), Vascular Cell Adhesion Molecule-1 (VCAM-1), Platelet-Endothelial Cell Adhesion Molecule-1 (PECAM-1), L1 Cell Adhesion Molecule (L1CAM), cell adhesion molecule with homology to L1CAM (close homolog of L1) (CHL1), sialic acid binding Ig-like lectins (SIGLECs; *e.g.*, SIGLEC-1, SIGLEC-2, SIGLEC-3, SIGLEC-4, *etc.*), Nectins *(e.g.*, Nectin-1, Nectin-2, Nectin-3, *etc.*), and Nectin-like molecules *(e.g.*, Necl-1, Necl-2, Necl-3, Necl-4, and Necl-5). Preferably, the presence or level of ICAM-1 and/or VCAM-1 is determined.

ICAM-1 is a transmembrane cellular adhesion protein that is continuously present in low concentrations in the membranes of leukocytes and endothelial cells. Upon cytokine stimulation, the concentrations greatly increase. ICAM-1 can be induced by IL-1 and TNFα and is expressed by the vascular endothelium, macrophages, and lymphocytes. In IBD, proinflammatory cytokines cause inflammation by upregulating expression of adhesion molecules such as ICAM-1 and VCAM-1. The increased expression of adhesion molecules recruit more lymphocytes to the infected tissue, resulting in tissue inflammation *(see,* Goke et al., J., Gastroenterol., 32:480 (1997); and Rijcken et al., Gut, 51:529 (2002)). ICAM-1 is encoded by the intercellular adhesion molecule 1 gene (ICAM1; Entrez GeneID:3383; Genbank Accession No. NM_000201) and is produced after processing of the intercellular adhesion molecule 1 precursor polypeptide (Genbank Accession No. SNP_000192).

VCAM-1 is a transmembrane cellular adhesion protein that mediates the adhesion of lymphocytes, monocytes, eosinophils, and basophils to vascular endothelium. Upregulation of VCAM-1 in endothelial cells by cytokines occurs as a result of increased gene transcription *(e.g.*, in response to Tumor necrosis factor-alpha (TNFα) and Interleukin-1 (IL-1)). VCAM-1 is encoded by the vascular cell adhesion molecule 1 gene (VCAM1; Entrez GeneID:7412) and is produced after differential splicing of the transcript (Genbank Accession No. NM_001078 (variant 1) or NM_080682 (variant 2)), and processing of the precursor polypeptide splice isoform (Genbank Accession No. NP_001069 (isoform a) or NP_542413 (isoform b)).

In certain instances, the presence or level of an IgSF CAM is detected at the level of mRNA expression with an assay such as, for example, a hybridization assay or an amplification-based assay. In certain other instances, the presence or level of an IgSF CAM is detected at the level of protein expression using, for example, an immunoassay *(e.g.,* ELISA) or an immunohistochemical assay. Suitable antibodies and/or ELISA kits for determining the presence or level of ICAM-1 and/or VCAM-1 in a sample such as a tissue sample, biopsy, serum, plasma, saliva, urine, or stool are available from, *e.g.,* Invitrogen (Camarillo, CA), Santa Cruz Biotechnology, Inc. (Santa Cruz, CA), and/or Abcam Inc. (Cambridge, MA).

**4. S100 Proteins**

The determination of the presence or level of at least one S100 protein in a sample is also useful in the present method. As used herein, the term "S100 protein" includes any member of a family of low molecular mass acidic proteins characterized by cell-type-specific expression and the presence of 2 EF-hand calcium-binding domains. There are at least 21 different types of S100 proteins in humans. The name is derived from the fact that S100 proteins are 100% soluble in ammonium sulfate at neutral pH. Most S100 proteins are homodimeric, consisting of two identical polypeptides held together by non-covalent bonds. Although S100 proteins are structurally similar to calmodulin, they differ in that they are cell-specific, expressed in particular cells at different levels depending on environmental factors. S-100 proteins are normally present in cells derived from the neural crest (e.g., Schwann cells, melanocytes, glial cells), chondrocytes, adipocytes, myoepithelial cells, macrophages, Langerhans cells, dendritic cells, and keratinocytes. S100 proteins have been implicated in a variety of intracellular and extracellular functions such as the regulation of protein phosphorylation, transcription factors, $Ca^{2+}$ homeostasis, the dynamics of cytoskeleton constituents, enzyme activities, cell growth and differentiation, and the inflammatory response.

Calgranulin is an S100 protein that is expressed in multiple cell types, including renal epithelial cells and neutrophils, and are abundant in infiltrating monocytes and granulocytes under conditions of chronic inflammation. Examples of calgranulins include, without limitation, calgranulin A (also known as S100A8 or MRP-8), calgranulin B (also known as S100A9 or MRP-14), and calgranulin C (also known as S100A12).

In certain instances, the presence or level of a particular S100 protein is detected at the level of mRNA expression with an assay such as, for example, a hybridization assay or an amplification-based assay. In certain other instances, the presence or level of a particular S100 protein is detected at the level of protein expression using, for example, an immunoassay *(e.g.,* ELISA) or an immunohistochemical assay. Suitable ELISA kits for determining the presence or level of an S100 protein such as calgranulin A (S100A8), calgranulin B (S100A9), or calgranulin C (S100A12) in a serum, plasma, or urine sample are available from, *e.g.,* Peninsula Laboratories Inc. (San Carlos, CA) and Hycult biotechnology b.v. (Uden, The Netherlands).

Calprotectin, the complex of S100A8 and S100A9, is a calcium- and zinc-binding protein in the cytosol of neutrophils, monocytes, and keratinocytes. Calprotectin is a major protein in neutrophilic granulocytes and macrophages and accounts for as much as 60% of the total protein in the cytosol fraction in these cells. It is therefore a surrogate marker of neutrophil turnover. Its concentration in stool correlates with the intensity of neutrophil infiltration of the intestinal mucosa and with the severity of inflammation. In some instances, calprotectin can be measured with an ELISA using small (50-100 mg) fecal samples *(see, e.g.,* Johne et al., Scand J Gastroenterol., 36:291-296 (2001)).

**5. Other Inflammatory Markers**

The determination of the presence or level of lactoferrin in a sample is also useful in the present method. In certain instances, the presence or level of lactoferrin is detected at the level of mRNA expression with an assay such as, for example, a hybridization assay or an amplification-based assay. In certain other instances, the presence or level of lactoferrin is detected at the level of protein expression using, for example, an immunoassay *(e.g.,* ELISA) or an immunohistochemical assay. A lactoferrin ELISA kit available from Calbiochem (San Diego, CA) can be used to detect human lactoferrin in a plasma, urine, bronchoalveolar lavage, or cerebrospinal fluid sample. Similarly, an ELISA kit available from U.S. Biological (Swampscott, MA) can be used to determine the level of lactoferrin in a plasma sample. U.S. Patent Publication No. 20040137536 describes an ELISA assay for determining the presence of elevated lactoferrin levels in a stool sample. Likewise, U.S. Patent Publication No. 20040033537 describes an ELISA assay for determining the concentration of endogenous lactoferrin in a stool, mucus, or bile sample. The presence or level of anti-lactoferrin antibodies can be detected in a sample using, *e.g.,* lactoferrin protein or a fragment thereof.

The determination of the presence or level of one or more pyruvate kinase isozymes such as M1-PK and M2-PK in a sample is also useful. In certain instances, the presence or level of M1-PK and/or M2-PK is detected at the level of mRNA expression with an assay such as, for example, a hybridization assay or an amplification-based assay. In certain other instances, the presence or level of M1-PK and/or M2-PK is detected at the level of protein expression using, for example, an immunoassay *(e.g.,* ELISA) or an immunohistochemical assay. Pyruvate kinase isozymes M1/M2 are also known as pyruvate kinase muscle isozyme (PKM), pyruvate kinase type K, cytosolic thyroid hormone-binding protein (CTHBP), thyroid hormone-binding protein 1 (THBP1), or opa-interacting protein 3 (OIP3).

The determination of the presence or level of one or more growth factors in a sample can also useful in the present method. Non-limiting examples of growth factors include transforming growth factors (TGF) such as TGF-$\alpha$, TGF-$\beta$, TNF-$\beta$2, TNF-$\beta$3, *etc.,* which are described in detail below.

### 6. Exemplary Set of Inflammatory Markers

At least one or a plurality *(e.g.,* two, three, four, five, six, seven, eight, nine, ten, or more such as, *e.g.,* a panel) of the following inflammatory markers can be detected (*e.g.,* alone or in combination with biomarkers from other categories) to aid or assist in predicting disease course, and/or to improve the accuracy of selecting therapy, optimizing therapy, reducing toxicity, and/or monitoring the efficacy of therapeutic treatment to anti-TNF$\alpha$ drug therapy:

    a. CRP
    b. SAA
    c. VCAM
    d. ICAM
    e. Calprotectin
    f. Lactoferrin
    g. IL8
    h. Rantes
    i. TNFalpha
    j. IL-6
    k. IL-1 beta
    l. S100A12
    m. M2-pyruvate kinase (PK)
    n. IFN
    o. IL2
    p. TGF
    q. IL-13
    r. IL-15
    s. IL12
    t. Other chemokines and cytokines.

### B. Growth Factors

A variety of growth factors, including biochemical markers, serological markers, protein markers, genetic markers, and other clinical or echographic characteristics, are suitable for use in the methods for selecting therapy, optimizing therapy, reducing toxicity, and/or monitoring the efficacy of therapeutic treatment with one or more therapeutic agents such as biologics *(e.g.,* anti-TNF$\alpha$ drugs). In certain aspects, the methods described herein utilize the application of an algorithm *(e.g.,* statistical analysis) to the presence, concentration level, and/or genotype determined for one or more growth factors *(e.g.,* alone or in combination with biomarkers from other categories) to aid or assist in predicting disease course, selecting an appropriate anti-TNF$\alpha$ drug therapy, optimizing anti-TNF$\alpha$ drug therapy, reducing toxicity associated with anti-TNF$\alpha$ drug therapy, or monitoring the efficacy of therapeutic treatment with an anti-TNF$\alpha$ drug.

As such, the determination of the presence or level of one or more growth factors in a sample can be useful in the present method. As used herein, the term "growth factor" includes any of a variety of peptides, polypeptides, or proteins that are capable of stimulating cellular proliferation and/or cellular differentiation.

In certain aspects, the presence or level of at least one growth factor including, but not limited to, epidermal growth factor (EGF), heparin-binding epidermal growth factor (HB-EGF), vascular endothelial growth factor (VEGF), pigment epithelium-derived factor (PEDF; also known as SERPINF1), amphiregulin (AREG; also known as schwannoma-derived growth factor (SDGF)), basic fibroblast growth factor (bFGF), hepatocyte growth factor (HGF), transforming growth factor-$\alpha$ (TGF-$\alpha$), transforming growth factor-$\beta$ (TNF-$\beta$1, TGF-$\beta$2, TGF-$\beta$3, *etc.),* endothelin-1 (ET-1), keratinocyte growth factor (KGF; also known as FGF7), bone morphogenetic proteins *(e.g.,* BMP1-BMP15), platelet-derived growth factor (PDGF), nerve growth factor (NGF), $\beta$-nerve growth factor ($\beta$-NGF), neurotrophic factors *(e.g.,* brain-derived neurotrophic factor (BDNF), neurotrophin 3 (NT3), neurotrophin 4 (NT4), *etc.*), growth differentiation factor-9 (GDF-9), granulocyte-colony stimulating factor (G-CSF), granulocyte-macrophage colony stimulating factor (GM-CSF), myostatin (GDF-8), erythropoietin (EPO), and thrombopoietin (TPO) is determined in a sample. In particular, the presence or level of at least one of VEGF, EGF, bFGF, ET-1, TGF-β2 and/or TGF-β3 can be determined. These markers have been found to be significantly higher in active IBD than in controls, indicating that they may play a role in promoting healing after mucosal injury of the luminal surface of the intestine in IBD.

In certain instances, the presence or level of a particular growth factor is detected at the level of mRNA expression with an assay such as, for example, a hybridization assay or an amplification-based assay. In certain other instances, the presence or level of a particular growth factor is detected at the level of protein expression using, for example, an immunoassay *(e.g.,* ELISA) or an immunohistochemical assay. Suitable ELISA kits for determining the presence or level of a growth factor in a serum, plasma, saliva, or urine sample are available from, *e.g.,* Antigenix America Inc. (Huntington Station, NY), Promega (Madison, WI), R&D Systems, Inc. (Minneapolis, MN), Invitrogen (Camarillo, CA), CHEMICON International, Inc. (Temecula, CA), Neogen Corp. (Lexington, KY), PeproTech (Rocky Hill, NJ), Alpco Diagnostics (Salem, NH), Pierce Biotechnology, Inc. (Rockford, IL), and/or Abazyme (Needham, MA).

The human epidermal growth factor (EGF) polypeptide sequence is set forth in, *e.g.,* Genbank Accession No. NP_001954 (SEQ ID NO:19). The human EGF mRNA (coding) sequence is set forth in, *e.g.,* Genbank Accession No. NM_001963 (SEQ ID NO:20). One skilled in the art will appreciate that EGF is also known as beta-urogastrone, URG, and HOMG4.

The human vascular endothelial growth factor (VEGF) polypeptide sequence is set forth in, *e.g.*, Genbank Accession Nos. NP_001020537 (SEQ ID NO:21), NP_001020538, NP_001020539, NP_001020540, NP_001020541, NP_001028928, and NP_003367. The human VEGF mRNA (coding) sequence is set forth in, *e.g.,* Genbank Accession No. NM_001025366 (SEQ ID NO:22), NM_001025367, NM_001025368, NM_001025369, NM_001025370, NM_001033756, and NM_003376. One skilled in the art will appreciate that VEGF is also known as VPF, VEGFA, VEGF-A, and MGC70609.

At least one or a plurality (*e.g.*, two, three, four, five, six, seven, eight, nine, ten, or more such as, *e.g.,* a panel) of the following growth factors can be detected *(e.g.,* alone or in combination with biomarkers from other categories) to aid or assist in predicting disease course, and/or to improve the accuracy of selecting therapy, optimizing therapy, reducing toxicity, and/or monitoring the efficacy of therapeutic treatment to anti-TNFα drug therapy: GM-CSF; VEGF; EGF; Keratinocyte growth factor (KGF; FGF7); and other growth factors.

**C. Serology (Immune Markers)**

The determination of serological or immune markers such as autoantibodies in a sample *(e.g.,* serum sample) is also useful in the present method. Antibodies against antiinflammatory molecules such as IL-10, TGF-β, and others might suppress the body's ability to control inflammation and the presence or level of these antibodies in the patient indicates the use of powerful immunosuppressive medications such as anti-TNFα drugs. Mucosal healing might result in a decrease in the antibody titre of antibodies to bacterial antigens such as, *e.g.,* OmpC, flagellins (cBir-1, Fla-A, Fla-X, *etc.),* I2, and others (pANCA, ASCA, *etc.).*

As such, in certain aspects, the methods described herein utilize the application of an algorithm (e.g., statistical analysis) to the presence, concentration level, and/or genotype determined for one or more immune markers *(e.g.,* alone or in combination with biomarkers from other categories) to aid or assist in predicting disease course, selecting an appropriate anti-TNFα drug therapy, optimizing anti-TNFα drug therapy, reducing toxicity associated with anti-TNFα drug therapy, or monitoring the efficacy of therapeutic treatment with an anti-TNFα drug.

Non-limiting examples of serological immune markers suitable for use in the present method include anti-neutrophil antibodies, *anti-Saccharomyces cerevisiae* antibodies, and/or other anti-microbial antibodies.

**1. Anti-Neutrophil Antibodies**

The determination of ANCA levels and/or the presence or absence of pANCA in a sample is useful in the methods. As used herein, the term "anti-neutrophil cytoplasmic antibody" or "ANCA" includes antibodies directed to cytoplasmic and/or nuclear components of neutrophils. ANCA activity can be divided into several broad categories based upon the ANCA staining pattern in neutrophils: (1) cytoplasmic neutrophil staining without perinuclear highlighting (cANCA); (2) perinuclear staining around the outside edge of the nucleus (pANCA); (3) perinuclear staining around the inside edge of the nucleus (NSNA); and (4) diffuse staining with speckling across the entire neutrophil (SAPPA). In certain instances, pANCA staining is sensitive to DNase treatment. The term ANCA encompasses all varieties of anti-neutrophil reactivity, including, but not limited to, cANCA, pANCA, NSNA, and SAPPA. Similarly, the term ANCA encompasses all immunoglobulin isotypes including, without limitation, immunoglobulin A and G.

ANCA levels in a sample from an individual can be determined, for example, using an immunoassay such as an enzyme-linked immunosorbent assay (ELISA) with alcohol-fixed neutrophils. The presence or absence of a particular category of ANCA such as pANCA can be determined, for example, using an immunohistochemical assay such as an indirect fluorescent antibody (IFA) assay. Preferably, the presence or absence of pANCA in a sample is determined using an immunofluorescence assay with DNase-treated, fixed neutrophils. In addition to fixed neutrophils, antigens specific for ANCA that are suitable for determining ANCA levels include, without limitation, unpurified or partially purified neutrophil extracts; purified proteins, protein fragments, or synthetic peptides such as histone H1 or ANCA-reactive fragments thereof (see, e.g., U.S. Patent No. 6,074,835); histone H1-like antigens, porin antigens, Bacteroides antigens, or ANCA-reactive fragments thereof (see, e.g., U.S. Patent No. 6,033,864); secretory vesicle antigens or ANCA-reactive fragments thereof (see, e.g., U.S. Patent Application No. 08/804,106); and anti-ANCA idiotypic antibodies. One skilled in the art will appreciate that the use of additional antigens specific for ANCA is possible with the present method.

## 2. *Anti-Saccharomyces cerevisiae* Antibodies

The determination of ASCA (e.g., ASCA-IgA and/or ASCA-IgG) levels in a sample is useful in the present method. As used herein, the term "anti-Saccharomyces cerevisiae immunoglobulin A" or "ASCA-IgA" includes antibodies of the immunoglobulin A isotype that react specifically with *S. cerevisiae.* Similarly, the term "anti-Saccharomyces cerevisiae immunoglobulin G" or "ASCA-IgG" includes antibodies of the immunoglobulin G isotype that react specifically with *S. cerevisiae.*

The determination of whether a sample is positive for ASCA-IgA or ASCA-IgG is made using an antigen specific for ASCA. Such an antigen can be any antigen or mixture of antigens that is bound specifically by ASCA-IgA and/or ASCA-IgG. Although ASCA antibodies were initially characterized by their ability to bind *S. cerevisiae,* those of skill in the art will understand that an antigen that is bound specifically by ASCA can be obtained from *S. cerevisiae* or from a variety of other sources so long as the antigen is capable of binding specifically to ASCA antibodies. Accordingly, exemplary sources of an antigen specific for ASCA, which can be used to determine the levels of ASCA-IgA and/or ASCA-IgG in a sample, include, without limitation, whole killed yeast cells such as *Saccharomyces* or *Candida* cells; yeast cell wall mannan such as phosphopeptidomannan (PPM); oligosachharides such as oligomannosides; neoglycolipids; anti-ASCA idiotypic antibodies; and the like. Different species and strains of yeast, such as *S. cerevisiae* strain Su1, Su2, CBS 1315, or BM 156, or *Candida albicans* strain VW32, are suitable for use as an antigen specific for ASCA-IgA and/or ASCA-IgG. Purified and synthetic antigens specific for ASCA are also suitable for use in determining the levels of ASCA-IgA and/or ASCA-IgG in a sample. Examples of purified antigens include, without limitation, purified oligosaccharide antigens such as oligomannosides. Examples of synthetic antigens include, without limitation, synthetic oligomannosides such as those described in U.S. Patent Publication No. 20030105060, e.g., D-Man $\beta$(1-2) D-Man $\beta$(1-2) D-Man $\beta$(1-2) D-Man-OR, D-Man $\alpha$(1-2) D-Man $\alpha$(1-2) D-Man $\alpha$(1-2) D-Man-OR, and D-Man $\alpha$(1-3) D-Man $\alpha$(1-2) D-Man $\alpha$(1-2) D-Man-OR, wherein R is a hydrogen atom, a $C_1$ to $C_{20}$ alkyl, or an optionally labeled connector group.

Preparations of yeast cell wall mannans, e.g., PPM, can be used in determining the levels of ASCA-IgA and/or ASCA-IgG in a sample. Such water-soluble surface antigens can be prepared by any appropriate extraction technique known in the art, including, for example, by autoclaving, or can be obtained commercially (see, e.g., Lindberg et al., Gut, 33:909-913 (1992)). The acid-stable fraction of PPM is also useful in the statistical algorithms (Sendid et al., Clin. Diag. Lab. Immunol., 3:219-226 (1996)). An exemplary PPM that is useful in determining ASCA levels in a sample is derived from *S. uvarum* strain ATCC #38926.

Purified oligosaccharide antigens such as oligomannosides can also be useful in determining the levels of ASCA-IgA and/or ASCA-IgG in a sample. The purified oligomannoside antigens are preferably converted into neoglycolipids as described in, for example, Faille et al., Eur. J. Microbiol. Infect. Dis., 11:43 8-446 (1992). One skilled in the art understands that the reactivity of such an oligomannoside antigen with ASCA can be optimized by varying the mannosyl chain length (Frosh et al., Proc Natl. Acad. Sci. USA, 82:1194-1198 (1985)); the anomeric configuration (Fukazawa et al., In "Immunology of Fungal Disease," E. Kurstak (ed.), Marcel Dekker Inc., New York, pp. 37-62 (1989); Nishikawa et al., Microbiol. Immunol., 34:825-840 (1990); Poulain et al., Eur. J. Clin. Microbiol., 23:46-52 (1993); Shibata et al., Arch. Biochem. Biophys., 243:338-348 (1985); Trinel et al., Infect. Immun., 60:3845-3851 (1992)); or the position of the linkage (Kikuchi et al., Planta, 190:525-535 (1993)).

Suitable oligomannosides for use in the methods include, without limitation, an oligomannoside having the mannotetraose Man(1-3) Man(1-2) Man(1-2) Man. Such an oligomannoside can be purified from PPM as described in, e.g., Faille et al., supra. An exemplary neoglycolipid specific for ASCA can be constructed by releasing the oligomannoside from its respective PPM and subsequently coupling the released oligomannoside to 4-hexadecylaniline or the like.

## 3. Anti-Microbial Antibodies

The determination of anti-OmpC antibody levels in a sample is also useful in the present method. As used herein, the term "anti-outer membrane protein C antibody" or "anti-OmpC antibody" includes antibodies directed to a bacterial outer membrane porin as described in, e.g., PCT Patent Publication No. WO 01/89361. The term "outer membrane protein C" or "OmpC" refers to a bacterial porin that is immunoreactive with an anti-OmpC antibody.

The level of anti-OmpC antibody present in a sample from an individual can be determined using an OmpC protein or a fragment thereof such as an immunoreactive fragment thereof. Suitable OmpC antigens useful in determining anti-OmpC antibody levels in a sample include, without limitation, an OmpC protein, an OmpC polypeptide having substantially the same amino acid sequence as the OmpC protein, or a fragment thereof such as an immunoreactive fragment thereof. As used herein, an OmpC polypeptide generally describes polypeptides having an amino acid sequence with greater than about 50% identity, preferably greater than about 60% identity, more preferably greater than about 70% identity, still more preferably greater than about 80%, 85%, 90%, 95%, 96%, 97%, 98%, or 99% amino acid sequence identity with an OmpC protein, with the amino acid identity determined using a sequence alignment program such as CLUSTALW. Such antigens can be prepared, for example, by purification from enteric bacteria such as *E. coli,* by recombinant expression of a nucleic acid such as Genbank Accession No. K00541, by synthetic means such as solution or solid phase peptide synthesis, or by using phage display.

The determination of anti-I2 antibody levels in a sample is also useful in the present method. As used herein, the term "anti-I2 antibody" includes antibodies directed to a microbial antigen sharing homology to bacterial transcriptional regulators as described in, *e.g.,* U.S. Patent No. 6,309,643. The term "I2" refers to a microbial antigen that is immunoreactive with an anti-I2 antibody. The microbial I2 protein is a polypeptide of 100 amino acids sharing some similarity weak homology with the predicted protein 4 from C. *pasteurianum,* Rv3557c from *Mycobacterium tuberculosis,* and a transcriptional regulator from *Aquifex aeolicus.* The nucleic acid and protein sequences for the I2 protein are described in, *e.g.,* U.S. Patent No. 6,309,643.

The level of anti-I2 antibody present in a sample from an individual can be determined using an I2 protein or a fragment thereof such as an immunoreactive fragment thereof. Suitable I2 antigens useful in determining anti-I2 antibody levels in a sample include, without limitation, an I2 protein, an I2 polypeptide having substantially the same amino acid sequence as the I2 protein, or a fragment thereof such as an immunoreactive fragment thereof. Such I2 polypeptides exhibit greater sequence similarity to the I2 protein than to the *C. pasteurianum* protein 4 and include isotype variants and homologs thereof. As used herein, an I2 polypeptide generally describes polypeptides having an amino acid sequence with greater than about 50% identity, preferably greater than about 60% identity, more preferably greater than about 70% identity, still more preferably greater than about 80%, 85%, 90%, 95%, 96%, 97%, 98%, or 99% amino acid sequence identity with a naturally-occurring I2 protein, with the amino acid identity determined using a sequence alignment program such as CLUSTALW. Such I2 antigens can be prepared, for example, by purification from microbes, by recombinant expression of a nucleic acid encoding an I2 antigen, by synthetic means such as solution or solid phase peptide synthesis, or by using phage display.

The determination of anti-flagellin antibody levels in a sample is also useful in the present method. As used herein, the term "anti-flagellin antibody" includes antibodies directed to a protein component of bacterial flagella as described in, *e.g.,* PCT Patent Publication No. WO 03/053220 and U.S. Patent Publication No. 20040043931. The term "flagellin" refers to a bacterial flagellum protein that is immunoreactive with an anti-flagellin antibody. Microbial flagellins are proteins found in bacterial flagellum that arrange themselves in a hollow cylinder to form the filament.

The level of anti-flagellin antibody present in a sample from an individual can be determined using a flagellin protein or a fragment thereof such as an immunoreactive fragment thereof. Suitable flagellin antigens useful in determining anti-flagellin antibody levels in a sample include, without limitation, a flagellin protein such as Cbir-1 flagellin, flagellin X, flagellin A, flagellin B, fragments thereof, and combinations thereof, a flagellin polypeptide having substantially the same amino acid sequence as the flagellin protein, or a fragment thereof such as an immunoreactive fragment thereof. As used herein, a flagellin polypeptide generally describes polypeptides having an amino acid sequence with greater than about 50% identity, preferably greater than about 60% identity, more preferably greater than about 70% identity, still more preferably greater than about 80%, 85%, 90%, 95%, 96%, 97%, 98%, or 99% amino acid sequence identity with a naturally-occurring flagellin protein, with the amino acid identity determined using a sequence alignment program such as CLUSTALW. Such flagellin antigens can be prepared, *e.g.*, by purification from bacterium such as *Helicobacter Bilis, Helicobacter mustelae, Helicobacter pylori, Butyrivibrio fibrisolvens,* and bacterium found in the cecum, by recombinant expression of a nucleic acid encoding a flagellin antigen, by synthetic means such as solution or solid phase peptide synthesis, or by using phage display.

### D. Oxidative Stress Markers

The determination of markers of oxidative stress in a sample is also useful in the present method. Non-limiting examples of markers of oxidative stress include those that are protein-based or DNA-based, which can be detected by measuring protein oxidation and DNA fragmentation, respectively. Other examples of markers of oxidative stress include organic compounds such as malondialdehyde.

Oxidative stress represents an imbalance between the production and manifestation of reactive oxygen species and a biological system's ability to readily detoxify the reactive intermediates or to repair the resulting damage. Disturbances in the normal redox state of tissues can cause toxic effects through the production of peroxides and free radicals that damage all components of the cell, including proteins, lipids, and DNA. Some reactive oxidative species can even act as messengers through a phenomenon called redox signaling.

Derivatives of reactive oxidative metabolites (DROMs), ratios of oxidized to reduced glutathione (Eh GSH), and/or ratios of oxidized to reduced cysteine (Eh CySH) can be used to quantify oxidative stress. *See, e.g.,* Neuman et al., Clin. Chem., 53:1652-1657 (2007). Oxidative modifications of highly reactive cysteine residues in proteins such as tyrosine phosphatases and thioredoxin-related proteins can also be detected or measured using a technique such as, *e.g.,* mass spectrometry (MS). *See, e.g.,* Naito et al., Anti-Aging Medicine, 7 (5):36-44 (2010). Other markers of oxidative stress include protein-bound acrolein as described, *e.g.,* in Uchida et al., PNAS, 95 (9) 4882-4887 (1998), the free oxygen radical test (FORT), which reflects levels of organic hydroperoxides, and the redox potential of the reduced glutathione/glutathione disulfide couple, (Eh) GSH/GSSG. *See, e.g.,* Abramson et al., Atherosclerosis, 178(1):115-21 (2005).

### E. Cell Surface Receptors

The determination of cell surface receptors in a sample is also useful in the present method. The half-life of anti-TNFα drugs such as Remicade and Humira is significantly decreased in patients with a high level of inflammation. CD64, the high-affinity receptor for immunoglobulin (Ig) G1 and IgG3, is predominantly expressed by mononuclear phagocytes. Resting polymorphonuclear (PMN) cells scarcely express CD64, but the expression of this marker is upregulated by interferon and granulocyte-colony-stimulating factor acting on myeloid precursors in the bone marrow. Crosslinking of CD64 with IgG complexes exerts a number of cellular responses, including the internalization of immune complexes by endocytosis, phagocytosis of opsonized particles, degranulation, activation of the oxidative burst, and the release of cytokines.

As such, in certain aspects, the methods described herein utilize the application of an algorithm *(e.g.,* statistical analysis) to the presence, concentration level, and/or genotype determined for one or more cell surface receptors such as CD64 *(e.g.,* alone or in combination with biomarkers from other categories) to aid or assist in predicting disease course, selecting an appropriate anti-TNFα drug therapy, optimizing anti-TNFα drug therapy, reducing toxicity associated with anti-TNFα drug therapy, or monitoring the efficacy of therapeutic treatment with an anti-TNFα drug.

### F. Signaling Pathways

The determination of signaling pathways in a sample is also useful in the present method. Polymorphonuclear (PMN) cell activation, followed by infltration into the intestinal mucosa (synovium for RA) and migration across the crypt epithelium is regarded as a key feature of IBD. It has been estimated by fecal indium-111-labeled leukocyte excretion that migration of PMN cells from the circulation to the diseased section of the intestine is increased by 10-fold or more in IBD patients. Thus, in certain aspects, measuring activation of PMN cells from blood or tissue inflammation by measuring signaling pathways using an assay such as the Collaborative Enzyme Enhanced Reactive ImmunoAssay (CEER) is an ideal way to understand inflammatory disease. The CEER technology is described in the following patent documents: PCT Publication Nos. WO 2008/036802, WO 2009/012140, WO 2009/108637, WO 2010/132723, WO 2011/008990, and WO 2011/050069; and PCT Application No. PCT/US2011/066624.

As such, in certain aspects, the methods described herein utilize the application of an algorithm *(e.g.,* statistical analysis) to the presence, concentration level, and/or genotype determined for one or more signal transduction molecules in one or more signaling pathways *(e.g.,* alone or in combination with biomarkers from other categories) to aid or assist in predicting disease course, selecting an appropriate anti-TNFα drug therapy, optimizing anti-TNFα drug therapy, reducing toxicity associated with anti-TNFα drug therapy, or monitoring the efficacy of therapeutic treatment with an anti-TNFα drug. The total level and/or activation *(e.g.,* phosphorylation) level of one or more signal transduction molecules in one or more signaling pathways can be measured.

The term "signal transduction molecule" or "signal transducer" includes proteins and other molecules that carry out the process by which a cell converts an extracellular signal or stimulus into a response, typically involving ordered sequences of biochemical reactions inside the cell. Examples of signal transduction molecules include, but are not limited to, receptor tyrosine kinases such as EGFR *(e.g.,* EGFR/HER1/ErbB1, HER2/Neu/ErbB2, HER3/ErbB3, HER4/ErbB4), VEGFR1/FLT1, VEGFR2/FLK1/KDR, VEGFR3/FLT4, FLT3/FLK2, PDGFR *(e.g.,* PDGFRA, PDGFRB), c-KIT/SCFR, INSR (insulin receptor), IGF-IR, IGF-IIR, IRR (insulin receptor-related receptor), CSF-1R, FGFR 1-4, HGFR 1-2, CCK4, TRK A-C, c-MET, RON, EPHA 1-8, EPHB 1-6, AXL, MER, TYRO3, TIE 1-2, TEK, RYK, DDR 1-2, RET, c-ROS, V-cadherin, LTK (leukocyte tyrosine kinase), ALK (anaplastic lymphoma kinase), ROR 1-2, MUSK, AATYK 1-3, and RTK 106; truncated forms of receptor tyrosine kinases such as truncated HER2 receptors with missing amino-terminal extracellular domains *(e.g.,* p95ErbB2 (p95m), p110, p95c, p95n, *etc.),* truncated cMET receptors with missing amino-terminal extracellular domains, and truncated HER3 receptors with missing amino-terminal extracellular domains; receptor tyrosine kinase dimers *(e.g.,* p95HER2/HER3; p95HER2/HER2; truncated HER3 receptor with HER1, HER2, HER3, or HER4; HER2/HER2; HER3/HER3; HER2/HER3; HER1/HER2; HER1/HER3; HER2/HER4; HER3/HER4; *etc.);*

non-receptor tyrosine kinases such as BCR-ABL, Src, Frk, Btk, Csk, Abl, Zap70, Fes/Fps, Fak, Jak, Ack, and LIMK; tyrosine kinase signaling cascade components such as AKT *(e.g.,* AKT1, AKT2, AKT3), MEK (MAP2K1), ERK2 (MAPK1), ERK1 (MAPK3), PI3K *(e.g.,* PIK3CA (p110), PIK3R1 (p85)), PDK1, PDK2, phosphatase and tensin homolog (PTEN), SGK3, 4E-BP1, P70S6K *(e.g.,* p70 S6 kinase splice variant alpha I), protein tyrosine phosphatases *(e.g.,* PTP1B, PTPN13, BDP1, *etc.*), RAF, PLA2, MEKK, JNKK, JNK, p38, Shc (p66), Ras *(e.g.,* K-Ras, N-Ras, H-Ras), Rho, Rac1, Cdc42, PLC, PKC, p53, cyclin D1, STAT1, STAT3, phosphatidylinositol 4,5-bisphosphate (PIP2), phosphatidylinositol 3,4,5-trisphosphate (PIP3), mTOR, BAD, p21, p27, ROCK, IP3, TSP-1, NOS, GSK-3β, RSK 1-3, JNK, c-Jun, Rb, CREB, Ki67, paxillin, NF-kB, and IKK; nuclear hormone receptors such as estrogen receptor (ER), progesterone receptor (PR), androgen receptor, glucocorticoid receptor, mineralocorticoid receptor, vitamin A receptor, vitamin D receptor, retinoid receptor, thyroid hormone receptor, and orphan receptors; nuclear receptor coactivators and repressors such as amplified in breast cancer-1 (AIB1) and nuclear receptor corepressor 1 (NCOR), respectively; and combinations thereof.

The term "activation state" refers to whether a particular signal transduction molecule is activated. Similarly, the term "activation level" refers to what extent a particular signal transduction molecule is activated. The activation state typically corresponds to the phosphorylation, ubiquitination, and/or complexation status of one or more signal transduction molecules. Non-limiting examples of activation states (listed in parentheses) include: HER1/EGFR (EGFRvIII, phosphorylated (p-) EGFR, EGFR:Shc, ubiquitinated (u-) EGFR, p-EGFRvIII); ErbB2 (p-ErbB2, p95HER2 (truncated ErbB2), p-p95HER2, ErbB2:Shc, ErbB2:PI3K, ErbB2:EGFR, ErbB2:ErbB3, ErbB2:ErbB4); ErbB3 (p-ErbB3, truncated ErbB3, ErbB3:PI3K, p-ErbB3:PI3K, ErbB3:Shc); ErbB4 (p-ErbB4, ErbB4:Shc); c-MET (p-c-MET, truncated c-MET, c-Met:HGF complex); AKT1 (p-AKT1); AKT2 (p-AKT2); AKT3 (p-AKT3); PTEN (p-PTEN); P70S6K (p-P70S6K); MEK (p-MEK); ERK1 (p-ERK1); ERK2 (p-ERK2); PDK1 (p-PDK1); PDK2 (p-PDK2); SGK3 (p-SGK3); 4E-BP1 (p-4E-BP1); PIK3R1 (p-PIK3R1); c-KIT (p-c-KIT); ER (p-ER); IGF-1R (p-IGF-1R, IGF-1R:IRS, IRS:PI3K, p-IRS, IGF-1R:PI3K); INSR (p-INSR); FLT3 (p-FLT3); HGFR1 (p-HGFR1); HGFR2 (p-HGFR2); RET (p-RET); PDGFRA (p-PDGFRA); PDGFRB (p-PDGFRB); VEGFR1 (p-VEGFR1, VEGFR1:PLCγ, VEGFR1:Src); VEGFR2 (p-VEGFR2, VEGFR2:PLCγ, VEGFR2:Src, VEGFR2:heparin sulphate, VEGFR2:VE-cadherin); VEGFR3 (p-VEGFR3); FGFR1 (p-FGFR1); FGFR2 (p-FGFR2); FGFR3 (p-FGFR3); FGFR4 (p-FGFR4); TIEI (p-TIEI); TIE2 (p-TIE2); EPHA (p-EPHA); EPHB (p-EPHB); GSK-3β (p-GSK-3β); NF-kB (p-NF-kB, NF-kB-IkB alpha complex and others), IkB (p-IkB, p-P65:IkB); IKK (phospho IKK); BAD (p-BAD, BAD: 14-3-3); mTOR (p-mTOR); Rsk-1 (p-Rsk-1); Jnk (p-Jnk); P38 (p-P38); STAT1 (p-STAT1); STAT3 (p-STAT3); FAK (p-FAK); RB (p-RB); Ki67; p53 (p-p53); CREB (p-CREB); c-Jun (p-c-Jun); c-Src (p-c-Src); paxillin (p-paxillin); GRB2 (p-GRB2), Shc (p-Shc), Ras (p-Ras), GAB1 (p-GAB1), SHP2 (p-SHP2), GRB2 (p-GRB2), CRKL (p-CRKL), PLCγ (p-PLCγ), PKC *(e.g.,* p-PKCα, p-PKCβ, p-PKCδ), adducin (p-adducin), RB1 (p-RB1), and PYK2 (p-PYK2).

The following tables provide additional examples of signal transduction molecules for which total levels and/or activation *(e.g.,* phosphorylation) levels can be determined in a sample *(e.g.,* alone or in combination with biomarkers from other categories) to aid or assist in predicting disease course, selecting an appropriate anti-TNFα drug therapy, optimizing anti-TNFα drug therapy, reducing toxicity associated with anti-TNFα drug therapy, or monitoring the efficacy of therapeutic treatment with an anti-TNFα drug.

<u>Table 1</u>                    <u>Table 2</u>

| Total / | Phospho Assays | Phospho sites |
|---|---|---|
| VEGFR2 Total | VEGFR2 Phospho | Y951, 1212 |
| Erk Total | Erk Phospho | T202/Y204 |
| Akt Total | Akt Phospho | T308, S473 |
| MEK Total | MEK Phospho | S217/221 |
| MEK Total | MEK Phospho | S217/221 |
| P70S6K Total | P70S6K Phospho | T389 (T229) |
| PTEN Total | | |
| VEGFR1 (T) | VEGFR1 Phospho | |
| SGK total | SGK phospho | T320, S486 |
| CRKL Total | CRKL Phospho | Y207 |
| SRC Total | SRC Phospho | Y 416, 527 |
| FAK Total | FAK Phospho | Y397 |
| BCR Total | BCR Phospho | |
| PI3K activated | PI3K complexed | P85 Y688 |
| 4EBP1 | 4EBP1 phospho | T70, T37, T46 |
| PRAS40 | PRAS40 phospho | T246 |

| Total / | Phospho Assays | Phospho sites |
|---|---|---|
| TIE Total | TIE-2 Phospho | Y992 (S1119) |
| Jak 2 Total | JAK 2 Phospho | Y1007/1008 |
| STAT5 Total | STAT 5 Phospho | Y694/699 |
| STAT 3 Total | STAT 3 Phospho | Y705 |
| FGFR1 total | FGFR1 Phospho | Y 653, 766 |
| FGFR2 total | FGFR 2 Phospho | Y653 |
| FGFR3 total | FGFR 3 Phospho | |
| FGFR4 total | FGFR 4 Phospho | |
| Axl total | Axl Phospho | Y702 |
| BAD total | BAD Phospho | (S112) (S136) |
| RSK total | RSK Phospho | (T359/S363) |
| PDK total | PDK 1 Phospho | (S241) |
| JAK 1 and 3 total | JAK 1 and 3 Phospho | |
| TSC2 total | TSC 2 Phospho | S664, S939 |
| S6RP Total | S6RP phospho | S235/236 |

## G. Genetic Markers

The determination of the presence or absence of allelic variants *(e.g.,* SNPs) in one or more genetic markers in a sample *(e.g.,* alone or in combination with biomarkers from other categories) is also useful in the methods to aid or assist in predicting disease course, selecting an appropriate anti-TNFα drug therapy, optimizing anti-TNFα drug therapy, reducing toxicity associated with anti-TNFα drug therapy, or monitoring the efficacy of therapeutic treatment with an anti-TNFα drug.

Non-limiting examples of genetic markers include, but are not limited to, any of the inflammatory pathway genes and corresponding SNPs that can be genotyped as set forth in Table 3 (*e.g.*, a NOD2/CARD15 gene, an IL12/IL23 pathway gene, *etc.).* Preferably, the presence or absence of at least one allelic variant, *e.g.*, a single nucleotide polymorphism (SNP), in the NOD2/CARD15 gene and/or one or more genes in the IL12/IL23 pathway is determined. *See, e.g.,* Barrett et al., Nat. Genet., 40:955-62 (2008) and Wang et al., Amer. J. Hum. Genet., 84:399-405 (2009).

Table 3

| Gene | SNP |
|---|---|
| NOD2 (R702W) - SNP8 | rs2066844 |
| NOD2 (G908R) - SNP12 | rs2066845 |
| NOD2 (3020insC) - SNP13 | rs5743293 |
| ATG16L1 (T300A) | rs2241880 |
| IL23R (R381Q) | rs11209026 |
| DLG5 | rs2165047 |
| NOD2/CARD15 | rs2066847 |
| IL23R | rs11465804 |
| ATG16L1 | rs3828309 |
| MST1 | rs3197999 |
| PTGER4 | rs4613763 |
| IRGM | rs11747270 |

(continued)

| Gene | SNP |
|---|---|
| TNFSF15 | rs4263839 |
| ZNF365 | rs10995271 |
| NKX2-3 | rs11190140 |
| PTPN2 | rs2542151 |
| PTPN22 | rs2476601 |
| ITLN1 | rs2274910 |
| IL12B | rs10045431 |
| CDKAL1 | rs6908425 |
| CCR6 | rs2301436 |
| JAK2 | rs10758669 |
| C11orf30 | rs7927894 |
| LRRK2, MUC19 | rs11175593 |
| ORMDL3 | rs2872507 |
| STAT3 | rs744166 |
| ICOSLG | rs762421 |
| GCKR | rs780094 |
| BTNL2, SLC26A3, HLA-DRB1, HLA-DQA1 | rs3763313 |
| PUS10 | rs13003464 |
| CCL2, CCL7 | rs991804 |
| LYRM4 | rs12529198 |
| SLC22A23 | rs17309827 |
| IL18RAP | rs917997 |
| IL12RB2 | rs7546245 |
| IL12RB1 | rs374326 |
| CD3D | rs3212262 |
| CD3G | rs3212262 |
| CD247 | rs704853 |
| JUN | rs6661505 |
| CD3E | rs7937334 |
| IL18R1 | rs1035127 |
| CCR5 | |
| MAPK14 | rs2237093 |
| IL18 | rs11214108 |
| IFNG | rs10878698 |
| MAP2K6 | rs2905443 |
| STAT4 | rs1584945 |
| IL12A | rs6800657 |
| TYK2 | rs12720356 |

(continued)

| Gene | SNP |
|---|---|
| ETV5 | rs9867846 |
| MAPK8 | rs17697885 |
| IRGM | rs13361189 |
| IRGM | rs4958847 |
| IRGM | rs1000113 |
| IRGM | rs11747270 |
| TL1A/TNFSF15 | rs6478109 |
| TL1A/TNFSF15 | rs6478108 |
| TL1A/TNFSF15 | rs4263839 |
| PTN22 | rs2476601 |
| CCR6 | rs1456893 |
| CCR6 | rs2301436 |
| 5p13/PTGER4 | rs1373692 |
| 5p13/PTGER4 | rs4495224 |
| 5p13/PTGER4 | rs7720838 |
| 5p13/PTGER4 | rs4613763 |
| ITLN1 | rs2274910 |
| ITLN1 | rs9286879 |
| ITLN1 | rs11584383 |
| IBD5/5q31 | rs2188962 |
| IBD5/5q31 | rs252057 |
| IBD5/5q31 | rs10067603 |
| GCKR | rs780094 |
| TNFRSF6B | rs1736135 |
| ZNF365 | rs224136 |
| ZNF365 | rs10995271 |
| C11orf30 | rs7927894 |
| LRRK2;MUC19 | rs1175593 |
| IL-27 | rs8049439 |
| TLR2 | rs4696480 |
| TLR2 | rs3804099 |
| TLR2 | rs3804100 |
| TLR2 | rs5743704 |
| TLR2 | rs2405432 |
| TLR4 (D299G) | rs4986790 |
| TLR4 (T399I) | rs4986791 |
| TLR4 (S360N) | rs4987233 |
| TLR9 | rs187084 |

(continued)

| Gene | SNP |
|---|---|
| TLR9 | rs352140 |
| NFC4 | rs4821544 |
| KIF21B | rs11584383 |
| IKZF1 | rs1456893 |
| C11orf30 | rs7927894 |
| CCL2,CCL7 | rs991804 |
| ICOSLG | rs762421 |
| TNFAIP3 | rs7753394 |
| FLJ45139 | rs2836754 |
| PTGER4 | rs4613763 |
| ECM1 | rs7511649 |
| ECM1 (T130M) | rs3737240 |
| ECM1 (G290S) | rs13294 |
| GLI1 (G933D) | rs2228224 |
| GLI1 (Q1100E) | rs2228226 |
| MDR1 (3435C>T) | rs1045642 |
| MDR1 (A893S/T) | rs2032582 |
| MAGI2 | rs6962966 |
| MAGI2 | rs2160322 |
| IL26 | rs12815372 |
| IFNG,IL26 | rs1558744 |
| IFNG,IL26 | rs971545 |
| IL26 | rs2870946 |
| ARPC2 | rs12612347 |
| IL10,IL19 | rs3024493 |
| IL10,IL19 | rs3024505 |
| IL23R | rs1004819 |
| IL23R | rs2201841 |
| IL23R | rs11465804 |
| IL23R | rs10889677 |
| BTLN2 | rs9268480 |
| HLA-DRB1 | rs660895 |
| MEP1 | rs6920863 |
| MEP1 | rs2274658 |
| MEP1 | rs4714952 |
| MEP1 | rs1059276 |
| PUS10 | rs13003464 |
| PUS10 | rs6706689 |

(continued)

| Gene | SNP |
| --- | --- |
| RNF 186 | rs3806308 |
| RNF186 | rs1317209 |
| RNF186 | rs6426833 |
| FCGR2A,C | rs10800309 |
| CEP72 | rs4957048 |
| DLD,LAMB1 | rs4598195 |
| CAPN10,KIF1A | rs4676410 |
| IL23R | rs11805303 |
| IL23R | rs7517847 |
| IL12B/p40 | rs1368438 |
| IL12B/p40 | rs10045431 |
| IL12B/p40 | rs6556416 |
| IL12B/p40 | rs6887695 |
| IL12B/p40 | rs3212227 |
| STAT3 | rs744166 |
| JAK2 | rs10974914 |
| JAK2 | rs10758669 |
| NKX2-3 | rs6584283 |
| NKX2-3 | rs10883365 |
| NKX2-3 | rs11190140 |
| IL18RAP | rs917997 |
| LYRM4 | rs12529198 |
| CDKAL1 | rs6908425 |
| MAGI2 | rs2160322 |
| TNFRSF6B | rs2160322 |
| TNFRSF6B | rs2315008 |
| TNFRSF6B | rs4809330 |
| PSMG1 | rs2094871 |
| PSMG1 | rs2836878 |
| PTPN2 | rs2542151 |
| MST1/3p21 | rs9858542 |
| MST1/3p21 | rs3197999 |
| SLC22A23 | rs17309827 |
| MHC | rs660895 |
| XBP1 | rs35873774 |
| ICOSLG1 | rs762421 |
| BTLN2 | rs3763313 |
| BTLN2 | rs2395185 |

(continued)

| Gene | SNP |
|------|-----|
| BTLN2 | rs9268480 |
| ATG5 | rs7746082 |
| CUL2,CREM | rs17582416 |
| CARD9 | rs4077515 |
| ORMDL3 | rs2872507 |
| ORMDL3 | rs2305480 |

Additional SNPs useful in the present method include, e.g., rs2188962, rs9286879, rs11584383, rs7746082, rs1456893, rs1551398, rs17582416, rs3764147, rs1736135, rs4807569, rs7758080, and rs8098673. *See, e.g.,* Barrett et al., Nat. Genet., 40:955-62 (2008).

## 1. NOD2/CARD15

The determination of the presence or absence of allelic variants such as SNPs in the NOD2/CARD15 gene is particularly useful in the present method. As used herein, the term "NOD2/CARD15 variant" or "NOD2 variant" includes a nucleotide sequence of a NOD2 gene containing one or more changes as compared to the wild-type NOD2 gene or an amino acid sequence of a NOD2 polypeptide containing one or more changes as compared to the wild-type NOD2 polypeptide sequence. NOD2, also known as CARD15, has been localized to the IBD1 locus on chromosome 16 and identified by positional-cloning (Hugot et al., Nature, 411:599-603 (2001)) as well as a positional candidate gene strategy (Ogura et al., Nature, 411:603-606 (2001); Hampe et al., Lancet, 357:1925-1928 (2001)). The IBD1 locus has a high multipoint linkage score (MLS) for inflammatory bowel disease (MLS=5.7 at marker D16S411 in 16q12). *See, e.g.,* Cho et al., Inflamm. Bowel Dis., 3:186-190 (1997); Akolkar et al., Am. J. Gastroenterol., 96:1127-1132 (2001); Ohmen et al., Hum. Mol. Genet., 5:1679-1683 (1996); Parkes et al., Lancet, 348:1588 (1996); Cavanaugh et al., Ann. Hum. Genet., 62:291-8 (1998); Brant et al., Gastroenterology, 115:1056-1061 (1998); Curran et al., Gastroenterology, 115:1066-1071 (1998); Hampe et al., Am. J. Hum. Genet., 64:808-816 (1999); and Annese et al., Eur. J. Hum. Genet., 7:567-573 (1999).

The mRNA (coding) and polypeptide sequences of human NOD2 are set forth in, *e.g.,* Genbank Accession Nos. NM_022162 and NP_071445, respectively. In addition, the complete sequence of human chromosome 16 clone RP11-327F22, which includes NOD2, is set forth in, e.g., Genbank Accession No. AC007728. Furthermore, the sequence of NOD2 from other species can be found in the GenBank database.

The NOD2 protein contains amino-terminal caspase recruitment domains (CARDs), which can activate NF-kappa B (NF-kB), and several carboxy-terminal leucine-rich repeat domains (Ogura et al., J. Biol. Chem., 276:4812-4818 (2001)). NOD2 has structural homology with the apoptosis regulator Apaf-1/CED-4 and a class of plant disease resistant gene products (Ogura *et al*., *supra*). Similar to plant disease resistant gene products, NOD2 has an amino-terminal effector domain, a nucleotide-binding domain and leucine rich repeats (LRRs). Wild-type NOD2 activates nuclear factor NF-kappa B, making it responsive to bacterial lipopolysaccharides (LPS; Ogura *et al., supra*; Inohara et al., J. Biol. Chem., 276:2551-2554 (2001). NOD2 can function as an intercellular receptor for LPS, with the leucine rich repeats required for responsiveness.

Variations at three single nucleotide polymorphisms in the coding region of NOD2 have been previously described. These three SNPs, designated R702W ("SNP 8"), G908R ("SNP 12"), and 1007fs ("SNP 13"), are located in the carboxy-terminal region of the NOD2 gene (Hugot *et al*., *supra*). A further description of SNP 8, SNP 12, and SNP 13, as well as additional SNPs in the NOD2 gene suitable for use in the method , can be found in, *e.g.,* U.S. Patent Nos. 6,835,815; 6,858,391; and 7,592,437; and U.S. Patent Publication Nos. 20030190639, 20050054021, and 20070072180.

A NOD2 variant can be located in a coding region of the NOD2 locus, for example, within a region encoding several leucine-rich repeats in the carboxy-terminal portion of the NOD2 polypeptide. Such NOD2 variants located in the leucine-rich repeat region of NOD2 include, without limitation, R702W ("SNP 8") and G908R ("SNP 12"). A NOD2 variant useful in the method can also encode a NOD2 polypeptide with reduced ability to activate NF-kappa B as compared to NF-kappa B activation by a wild-type NOD2 polypeptide. As a non-limiting example, the NOD2 variant 1007fs ("SNP 13") results in a truncated NOD2 polypeptide which has reduced ability to induce NF-kappa B in response to LPS stimulation (Ogura et al., Nature, 411:603-606 (2001)).

A NOD2 variant useful in the method can be, for example, R702W, G908R, or 1007fs. R702W, G908R, and 1007fs are located within the coding region of NOD2. A method of the can be practiced with the R702W NOD2 variant. As used herein, the term "R702W" includes a single nucleotide polymorphism within exon 4 of the NOD2 gene, which occurs within a triplet encoding amino acid 702 of the NOD2 protein. The wild-type NOD2 allele contains a cytosine (c) residue at position 138,991 of the AC007728 sequence, which occurs within a triplet encoding an arginine at amino acid702. The R702W NOD2 variant contains a thymine (t) residue at position 138,991 of the AC007728 sequence, resulting in an arginine (R) to tryptophan (W) substitution at amino acid 702 of the NOD2 protein. Accordingly, this NOD2 variant is denoted "R702W" or "702W" and can also be denoted "R675W" based on the earlier numbering system of Hugot *et al.*, *supra.* In addition, the R702W variant is also known as the "SNP 8" allele or a "2" allele at SNP 8. The NCBI SNP ID number for R702W or SNP 8 is rs2066844. The presence of the R702W NOD2 variant and other NOD2 variants can be conveniently detected, for example, by allelic discrimination assays or sequence analysis.

A method can also be practiced with the G908R NOD2 variant. As used herein, the term "G908R" includes a single nucleotide polymorphism within exon 8 of the NOD2 gene, which occurs within a triplet encoding amino acid 908 of the NOD2 protein. Amino acid 908 is located within the leucine rich repeat region of the NOD2 gene. The wild-type NOD2 allele contains a guanine (g) residue at position 128,377 of the AC007728 sequence, which occurs within a triplet encoding glycine at amino acid 908. The G908R NOD2 variant contains a cytosine (c) residue at position 128,377 of the AC007728 sequence, resulting in a glycine (G) to arginine (R) substitution at amino acid 908 of the NOD2 protein. Accordingly, this NOD2 variant is denoted "G908R" or "908R" and can also be denoted "G881 R" based on the earlier numbering system of Hugot *et al.*, *supra.* In addition, the G908R variant is also known as the "SNP 12" allele or a "2" allele at SNP 12. The NCBI SNP ID number for G908R SNP 12 is rs2066845.

A method can also be practiced with the 1007fs NOD2 variant. This variant is an insertion of a single nucleotide that results in a frame shift in the tenth leucine-rich repeat of the NOD2 protein and is followed by a premature stop codon. The resulting truncation of the NOD2 protein appears to prevent activation of NF-kappaB in response to bacterial lipopolysaccharides (Ogura *et al.*, *supra*). As used herein, the term "1007fs" includes a single nucleotide polymorphism within exon 11 of the NOD2 gene, which occurs in a triplet encoding amino acid 1007 of the NOD2 protein. The 1007fs variant contains a cytosine which has been added at position 121,139 of the AC007728 sequence, resulting in a frame shift mutation at amino acid 1007. Accordingly, this NOD2 variant is denoted "1007fs" and can also be denoted "3020insC" or "980fs" based on the earlier numbering system of Hugot *et al.*, *supra.* In addition, the 1007fs NOD2 variant is also known as the "SNP 13" allele or a "2" allele at SNP 13. The NCBI SNP ID number for 1007fs or SNP 13 is rs2066847.

One skilled in the art recognizes that a particular NOD2 variant allele or other polymorphic allele can be conveniently defined, for example, in comparison to a Centre d'Etude du Polymorphisme Humain (CEPH) reference individual such as the individual designated 1347-02 (Dib et al., Nature, 380:152-154 (1996)), using commercially available reference DNA obtained, for example, from PE Biosystems (Foster City, CA). In addition, specific information on SNPs can be obtained from the dbSNP of the National Center for Biotechnology Information (NCBI).

A NOD2 variant can also be located in a non-coding region of the NOD2 locus. Non-coding regions include, for example, intron sequences as well as 5' and 3' untranslated sequences. A non-limiting example of a NOD2 variant allele located in a non-coding region of the NOD2 gene is the JW 1 variant, which is described in Sugimura et al., Am. J. Hum. Genet., 72:509-518 (2003) and U.S. Patent Publication No. 20070072180. Examples of NOD2 variant alleles located in the 3' untranslated region of the NOD2 gene include, without limitation, the JW15 and JW16 variant alleles, which are described in U.S. Patent Publication No. 20070072180. Examples of NOD2 variant alleles located in the 5' untranslated region (*e.g.,* promoter region) of the NOD2 gene include, without limitation, the JW17 and JW18 variant alleles, which are described in U.S. Patent Publication No. 20070072180.

As used herein, the term "JW1 variant allele" includes a genetic variation at nucleotide 158 of intervening sequence 8 (intron 8) of the NOD2 gene. In relation to the AC007728 sequence, the JW1 variant allele is located at position 128,143. The genetic variation at nucleotide 158 of intron 8 can be, but is not limited to, a single nucleotide substitution, multiple nucleotide substitutions, or a deletion or insertion of one or more nucleotides. The wild-type sequence of intron 8 has a cytosine at position 158. As non-limiting examples, a JW1 variant allele can have a cytosine (c) to adenine (a), cytosine (c) to guanine (g), or cytosine (c) to thymine (t) substitution at nucleotide 158 of intron 8. The JW1 variant allele can be a change from a cytosine (c) to a thymine (t) at nucleotide 158 of NOD2 intron 8.

The term "JW15 variant allele" includes a genetic variation in the 3' untranslated region of NOD2 at nucleotide position 118,790 of the AC007728 sequence. The genetic variation at nucleotide 118,790 can be, but is not limited to, a single nucleotide substitution, multiple nucleotide substitutions, or a deletion or insertion of one or more nucleotides. The wild-type sequence has an adenine (a) at position 118,790. As non-limiting examples, a JW15 variant allele can have an adenine (a) to cytosine (c), adenine (a) to guanine (g), or adenine (a) to thymine (t) substitution at nucleotide 118,790. The JW15 variant allele can be a change from an adenine (a) to a cytosine (c) at nucleotide 118,790.

As used herein, the term "JW16 variant allele" includes a genetic variation in the 3' untranslated region of NOD2 at nucleotide position 118,031 of the AC007728 sequence. The genetic variation at nucleotide 118,031 can be, but is not limited to, a single nucleotide substitution, multiple nucleotide substitutions, or a deletion or insertion of one or more nucleotides. The wild-type sequence has a guanine (g) at position 118,031. As non-limiting examples, a JW16 variant allele can have a guanine (g) to cytosine (c), guanine (g) to adenine (a), or guanine (g) to thymine (t) substitution at nucleotide 118,031. The JW16 variant allele can be a change from a guanine (g) to an adenine (a) at nucleotide 118,031.

The term "JW17 variant allele" includes a genetic variation in the 5' untranslated region of NOD2 at nucleotide position 154,688 of the AC007728 sequence. The genetic variation at nucleotide 154,688 can be, but is not limited to, a single nucleotide substitution, multiple nucleotide substitutions, or a deletion or insertion of one or more nucleotides. The wild-type sequence has a cytosine (c) at position 154,688. As non-limiting examples, a JW17 variant allele can have a cytosine (c) to guanine (g), cytosine (c) to adenine (a), or cytosine (c) to thymine (t) substitution at nucleotide 154,688. The JW17 variant allele can be a change from a cytosine (c) to a thymine (t) at nucleotide 154,688.

As used herein, the term "JW18 variant allele" includes a genetic variation in the 5' untranslated region of NOD2 at nucleotide position 154,471 of the AC007728 sequence. The genetic variation at nucleotide 154,471 can be, but is not limited to, a single nucleotide substitution, multiple nucleotide substitutions, or a deletion or insertion of one or more nucleotides. The wild-type sequence has a cytosine (c) at position 154,471. As non-limiting examples, a JW18 variant allele can have a cytosine (c) to guanine (g), cytosine (c) to adenine (a), or cytosine (c) to thymine (t) substitution at nucleotide 154,471. The JW18 variant allele can be a change from a cytosine (c) to a thymine (t) at nucleotide 154,471.

It is understood that the methods can be practiced with these or other NOD2 variant alleles located in a coding region or non-coding region (*e.g.*, intron or promoter region) of the NOD2 locus. It is further understood that the methods can involve determining the presence of one, two, three, four, or more NOD2 variants, including, but not limited to, the SNP 8, SNP 12, and SNP 13 alleles, and other coding as well as non-coding region variants.

**V. Examples**

The present method will be described in greater detail by way of specific examples. The following examples are offered for illustrative purposes. Those of skill in the art will readily recognize a variety of noncritical parameters which can be changed or modified to yield essentially the same results.

**Example 1. Novel Mobility Shift Assay for Measuring Levels of anti-TNFα Biologics.**

This example illustrates a novel homogeneous assay for measuring anti-TNFα drug concentration in a patient sample *(e.g.,* serum) using size exclusion chromatography to detect the binding of the anti-TNFα drug to fluorescently labeled TNFα. The assay is advantageous because it obviates the need for wash steps, uses fluorophores that allow for detection on the visible and/or IR spectra which decreases background and serum interference issues, increases the ability to detect anti-TNFα drugs in patients with a low titer due to the high sensitivity of fluorescent label detection, and occurs as a liquid phase reaction, thereby reducing the chance of any changes in the epitope by attachment to a solid surface such as an ELISA plate.

In one exemplary method , TNFα is labeled with a fluorophore *(e.g.,* $Alexa_{647}$), wherein the fluorophore can be detected on either or both the visible and IR spectra. The labeled TNFα is incubated with human serum in a liquid phase reaction to allow the anti-TNFα drug present in the serum to bind. The labeled TNFα can also be incubated with known amounts of the anti-TNFα drug in a liquid phase reaction to create a standard curve. Following incubation, the samples are loaded directly onto a size exclusion column. Binding of the anti-TNFα drug to the labeled TNFα results in a leftward shift of the peak compared to labeled TNFα alone. The concentration of the anti-TNFα drug present in the serum sample can then be compared to the standard curve and controls.

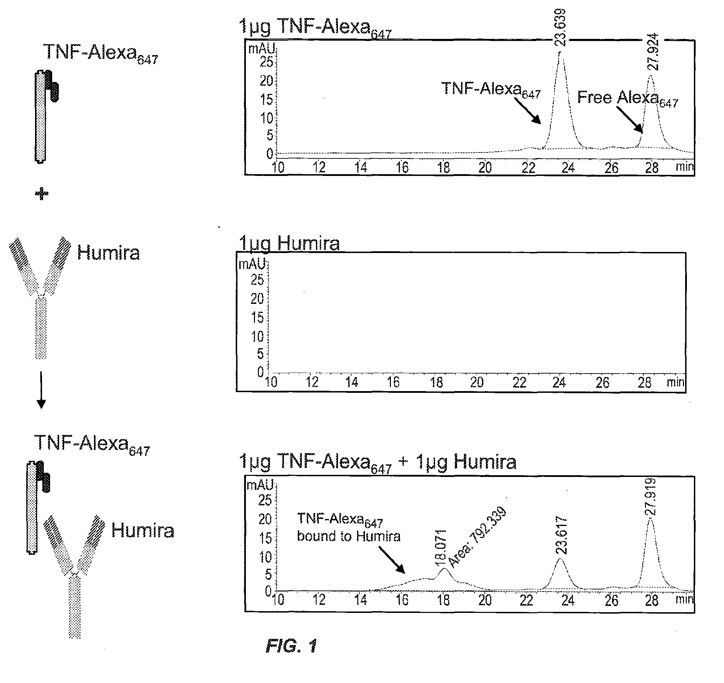

Figure 1 shows an example of the assay of the present method wherein size exclusion HPLC is used to detect the binding between $TNFα-Alexa_{647}$ and HUMIRA™ (adalimumab). As shown in Figure 1, the binding of HUMIRA™ to $TNFα.-Alex_{647}$ caused a shift of the $TNFα-Alexa_{647}$ peak to the left.

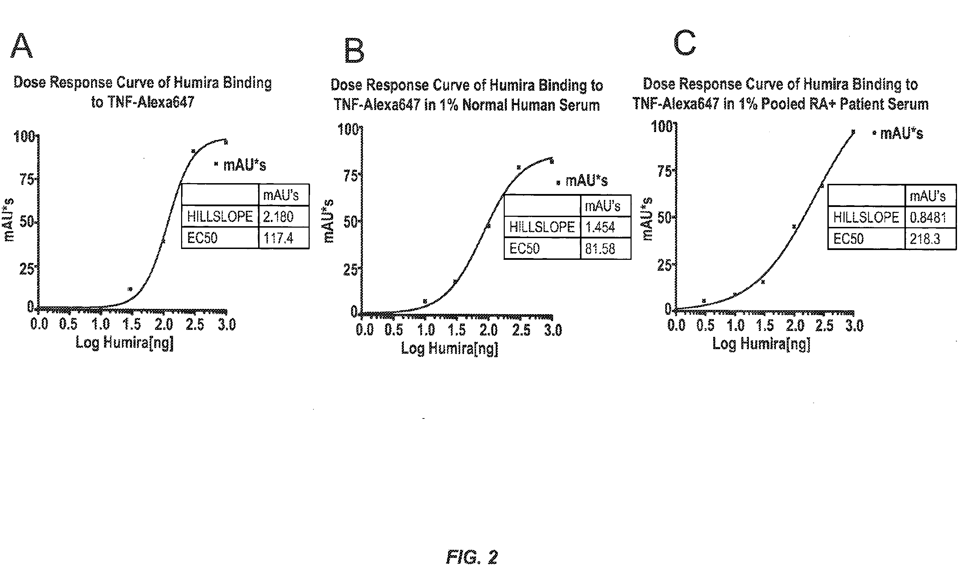

Figure 2 shows dose response curves of $HUMIRA^{TM}$ binding to $TNFα-Alexa_{647}$. In particular, Figure 2A shows that HUMIRA™ dose-dependently increased the shift of $TNFα-Alexa_{647}$ in the size exclusion chromatography assay. Figure 2B shows that the presence of 1% human serum did not have a significant effect on the shift of $TNFα-Alexa_{647}$ in the size exclusion chromatography assay. Figure 2C shows that the presence of pooled RF-positive serum did not have a significant effect on the shift of $TNFα-Alexa_{647}$ in the size exclusion chromatography assay.

As such, this example demonstrates the utility of the present method in monitoring patients receiving an anti-TNFα drug such as HUMIRA™: (1) to guide in the determination of the appropriate drug dosage; (2) to evaluate drug pharmacokinetics, *e.g.,* to determine whether the drug is being cleared from the body too quickly; and (3) to guide treatment decisions, *e.g.,* whether to switch from the current anti-TNFα drug to a different TNFα inhibitor or to another type of therapy.

**Example 2. Novel Mobility Shift Assay for Measuring HACA and HAHA Levels.**

This example illustrates a novel homogeneous assay for measuring autoantibody *(e.g.,* HACA and/or HAHA) concentrations in a patient sample *(e.g.,* serum) using size exclusion chromatography to detect the binding of these autoantibodies to fluorescently labeled anti-TNFα drug. The assay is advantageous because it obviates the need for wash steps which remove low affinity HACA and HAHA, uses fluorophores that allow for detection on the visible and/or IR spectra which decreases background and serum interference issues, increases the ability to detect HACA and HAHA in patients with a low titer due to the high sensitivity of fluorescent label detection, and occurs as a liquid phase reaction, thereby reducing the chance of any changes in the epitope by attachment to a solid surface such as an ELISA plate.

The clinical utility of measuring autoantibodies *(e.g.,* HACA, HAHA, *etc.)* that are generated against TNF$\alpha$ inhibitors is illustrated by the fact that HACAs were detected in 53%, 21%, and 7% of rheumatoid arthritis patients treated with 1 mg/kg, 3 mg/kg, and 10 mg/kg infliximab. When infliximab was combined with methotrexate, the incidence of antibodies was lower 15%, 7%, and 0%, which indicates that concurrent immunosuppressive therapy is effective in lowering anti-drug responses, but also indicates that a high dose of anti-TNF$\alpha$ antibody might lead to tolerance. In Crohn's disease, a much higher incidence was reported; after the fifth infusion, 61 % of patients had HACA. The clinical response was shortened when HACAs were present. *See,* Rutgeerts, N. Engl. J. Med., 348:601-608 (2003). A retrospective study of infliximab and HACA levels measured over a 3 year period from 2005 to 2008 in 155 patients demonstrated that HACAs were detected in 22.6% (N = 35) of patients with inflammatory bowel disease. *See,* Afif et al., "Clinical Utility of Measuring Infliximab and Human Anti-Chimeric Antibody Levels in Patients with Inflammatory Bowel Disease"; paper presented at Digestive Disease Week; May 30-June 3, 2009; Chicago, IL. The authors concluded that changing treatment based on clinical symptoms alone may lead to inappropriate management.

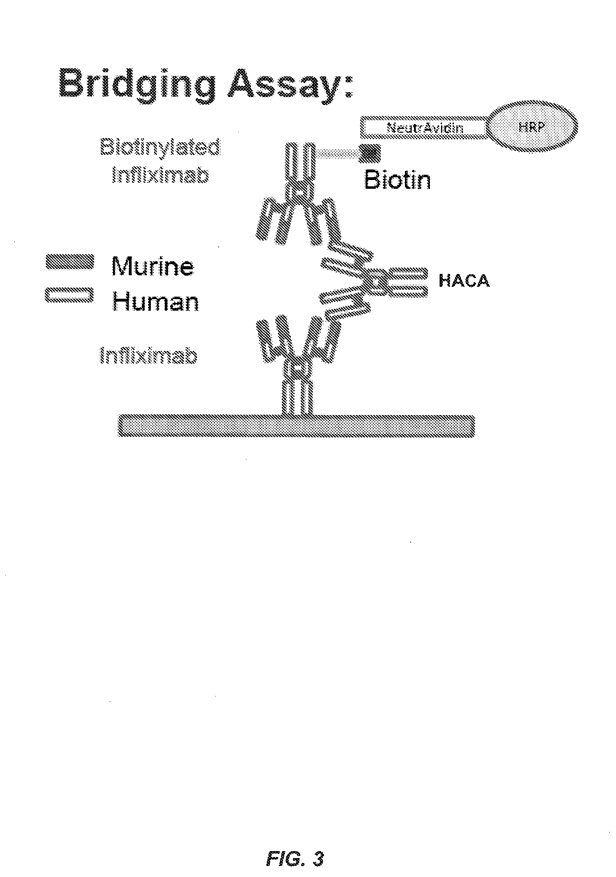

The homogeneous mobility shift assay is advantageous over current methods such as the bridging assay shown in Figure 3 for measuring autoantibody *(e.g.,* HACA and/or HAHA) concentrations in a patient sample because the inventive method is capable of measuring the concentration of autoantibodies such as HACA without non-specific binding and solid phase interference from the ELISA plate, without interference from the anti-TNF$\alpha$ drug *(e.g.,* with the bridging assay, HACA measurements must be taken at anti-TNF$\alpha$ drug trough levels), and without any dependency on the multivalency of the antibody (e.g., IgG4 antibodies are not detected using the bridging assay because IgG4 antibodies are bispecific and cannot cross-link the same antigen). As such, the present method has at least the following advantages over current methods: avoids attachment of antigens to solid surfaces (denaturation avoided); eliminates the IgG4 effect; overcomes therapeutic antibody trough issues; detects antibodies with weak affinities; eliminates non-specific binding of irrelevant IgGs; and increases the sensitivity and specificity of detection.

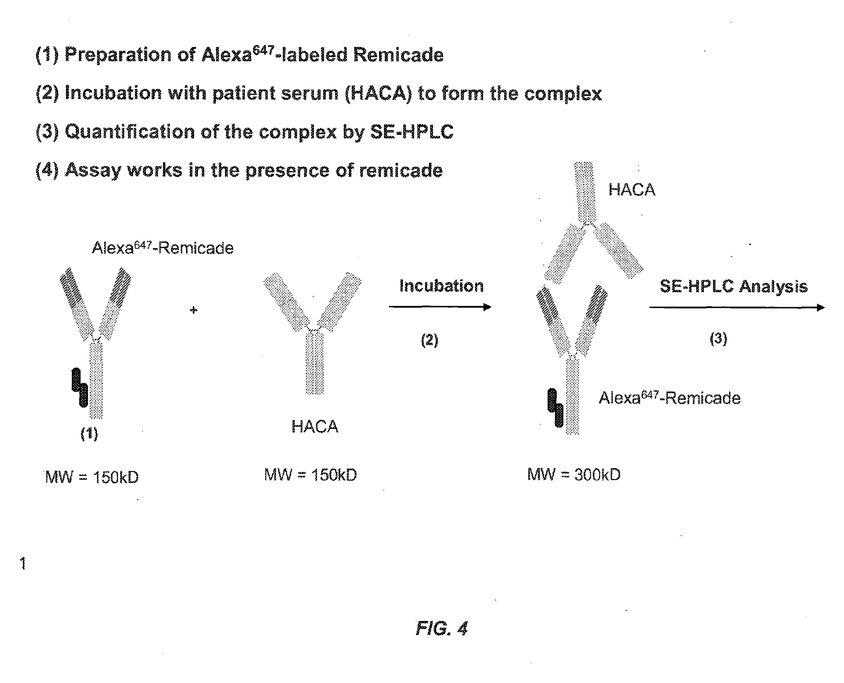

In one exemplary method , an anti-TNF$\alpha$ drug *(e.g.,* REMICADE™) is labeled with a fluorophore *(e.g.,* Alexa$_{647}$), wherein the fluorophore can be detected on either or both the visible and IR spectra. The labeled anti-TNF$\alpha$ drug is incubated with human serum in a liquid phase reaction to allow HACA and HAHA present in the serum to bind. The labeled anti-TNF$\alpha$ drug can also be incubated with known amounts of an anti-IgG antibody in a liquid phase reaction to create a standard curve. Following incubation, the samples are loaded directly onto a size exclusion column. Binding of the autoantibodies to the labeled anti-TNF$\alpha$ drug results in a leftward shift of the peak compared to labeled drug alone. The concentration of HACA and HAHA present in the serum sample can then be compared to the standard curve and controls. Figure 4 illustrates an exemplary outline of the autoantibody detection assays for measuring the concentrations of HACA/HAHA generated against REMICADE™. In certain instances, high HACA/HAHA levels indicate that the current therapy with REMICADE™ should be switched to another anti-TNF$\alpha$ drug such as HUMIRA™.

The principle of this assay is based on the mobility shift of the antibody bound Alexa$_{647}$-labeled Remicade complex versus free Alexa$_{647}$-labeled Remicade on size exclusion- high performance liquid chromatography (SE-HPLC) due to the increase in molecular weight of the complex.

The chromatography in this example was performed on an Agilent-1200 HPLC System, using a Bio-Sep 300x7.8 mm SEC-3000 column (Phenomenex) with a molecular weight fractionating range of 5,000 - 700,000 and a mobile phase of 1X PBS, pH 7.4, at a flow-rate of 0.5 mL/min with UV detection at 650 nm. A 100 $\mu$L sample volume is loaded onto the column for each analysis.

The antibody bound Alexa$_{647}$-labeled Remicade complex is formed by incubating a known amount of the antibody and Alexa$_{647}$-labeled Remicade in the 1X PBS, pH 7.3, elution buffer at room temperature for 1 hour before SE-HPLC analysis.

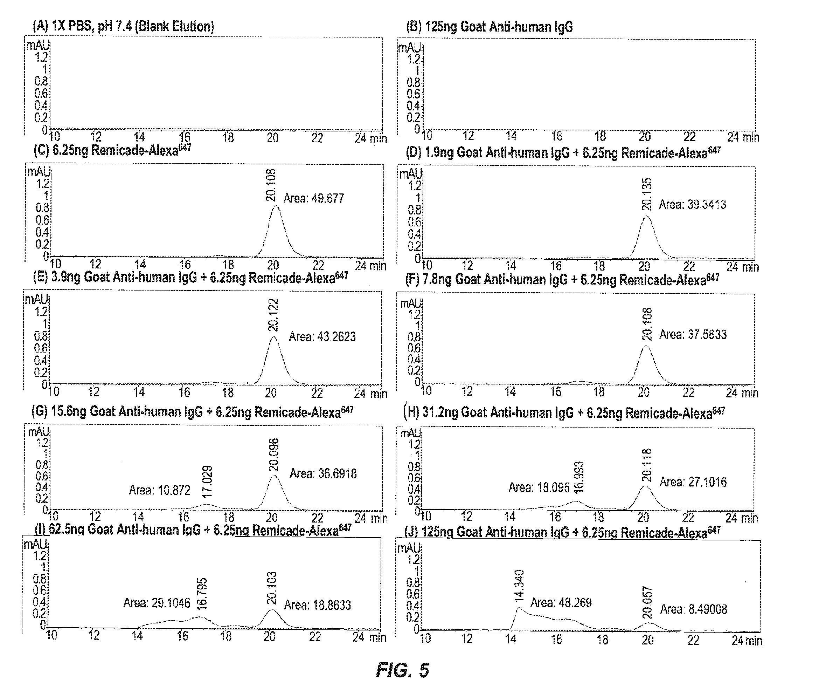

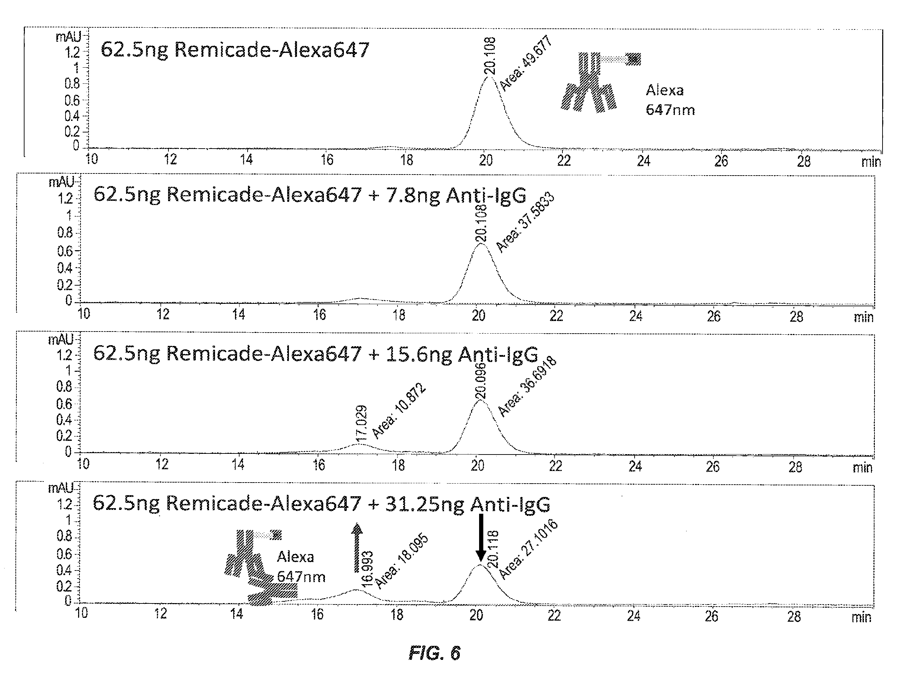

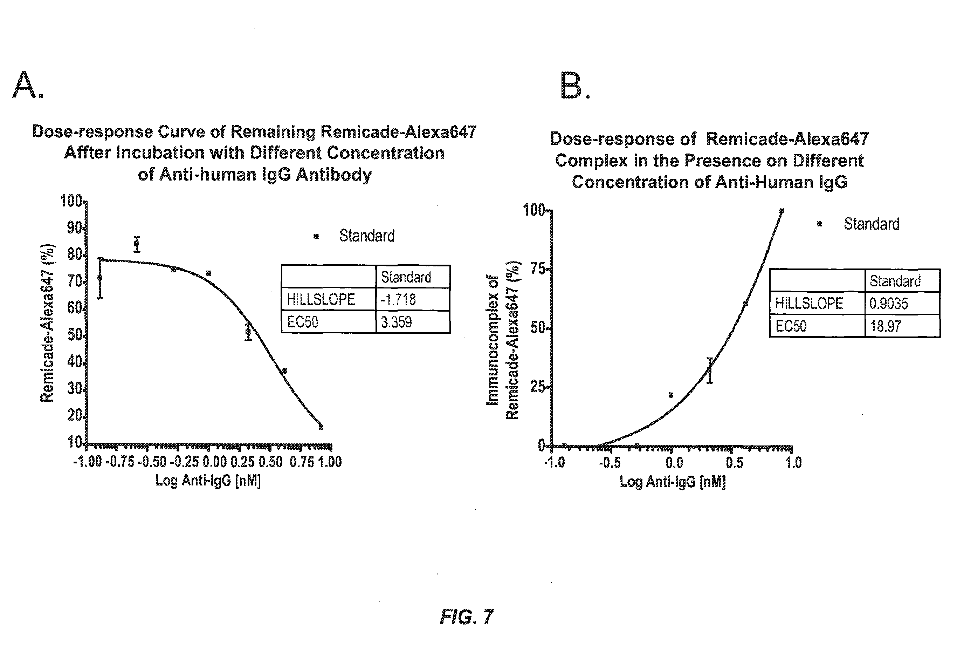

Figure 5 shows a dose response analysis of anti-human IgG antibody binding to REMICADE™-Alexa$_{647}$ as detected using the size exclusion chromatography assay. The binding of anti-IgG antibody to REMICADE™-Alexa$_{647}$ caused a shift of the REMICADE™-Alexa$_{647}$ peak to the left. Figure 6 shows a second dose response analysis of anti-human IgG antibody binding to REMICADE™-Alexa$_{647}$ as detected using the size exclusion chromatography assay. Higher amounts of anti-IgG antibody resulted in a dose-dependent increase in the formation of anti-IgG/REMICADE™-Alexa$_{647}$ complexes, as indicated by a shift of the REMICADE™-Alexa$_{647}$ peak to the left. Figure 7 shows dose response curves of anti-IgG antibody binding to REMICADE™-Alexa$_{647}$.

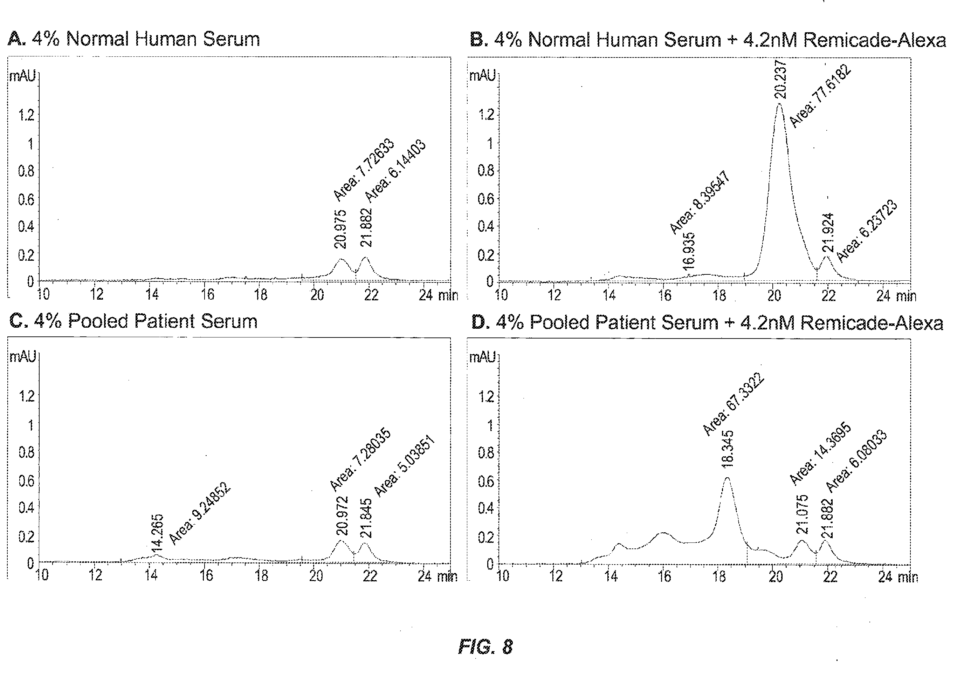

Figure 8 shows REMICADE™-Alexa$_{647}$ immunocomplex formation in normal human serum and HACA positive serum as detected using the size exclusion chromatography assay with 100 $\mu$l of injected sample. As shown in Figure 8, the binding of HACA present in patient samples to REMICADE™-Alexa$_{647}$ caused a shift of the REMICADE™-Alexa$_{647}$ peak to the left. As such, the size exclusion chromatography assay is particularly advantageous because it measures HACA in the presence of REMICADE™, can be utilized while the patient is on therapy, measures both weak and strong HACA binding, is a mix and read mobility shift assay, and can be extended to other approaches which use labeled REMICADE™ to equilibrate with HACA and REMICADE™.

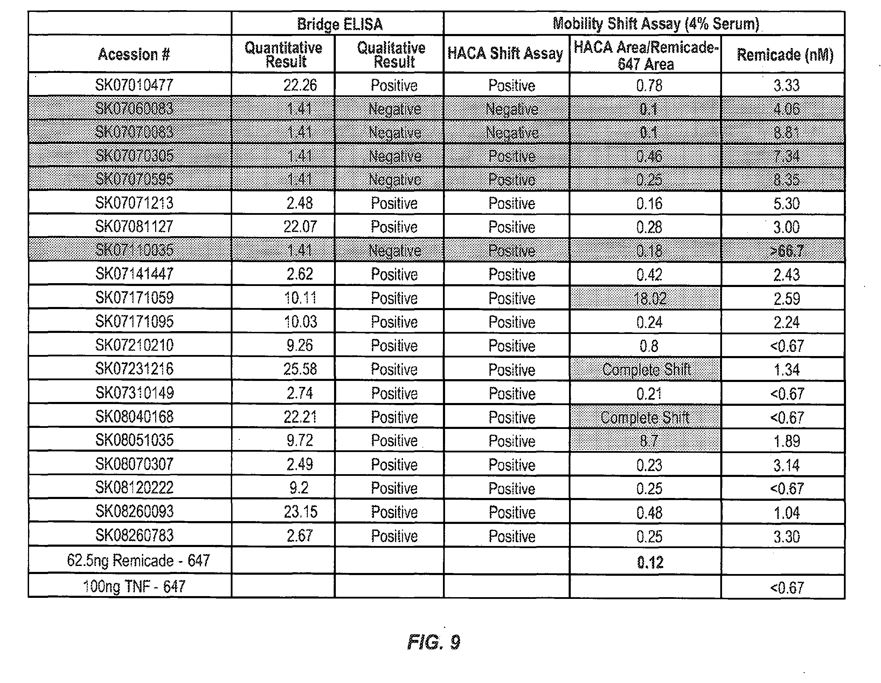

Figure 9 provides a summary of HACA measurements from 20 patient serum samples that were performed using the bridging assay or the mobility shift assay. This comparative study demonstrates that the present methods have increased sensitivity over current methods because 3 samples that were negative for HACA as measured using the bridging assay were actually HACA positive when measured using the mobility shift assay *(see,* Patient # SK07070305, SK07070595, and SK07110035).

As such, this example demonstrates the utility of the present method in monitoring patients receiving an anti-TNF$\alpha$ drug *(e.g.,* REMICADE™) to detect the presence or level of autoantibodies *(e.g.,* HACA and/or HAHA) against the drug, because such immune responses can be associated with hypersensitive reactions and dramatic changes in pharmacokinetics and biodistribution of the anti-TNF$\alpha$ drug that preclude further treatment with the drug.

In conclusion, Examples 1 and 2 demonstrate that TNF$\alpha$ and anti-TNF$\alpha$ antibodies can be efficiently labeled with Alexa$_{647}$. When labeled TNF$\alpha$.-Alex$_{647}$ was incubated with anti-TNF$\alpha$ antibodies, the retention time of the labeled TNF$\alpha$/anti-TNFa antibody complex was shifted, and the amount of anti-TNF$\alpha$ antibody that caused the shift could be quantitated with HPLC. Furthermore, when labeled anti-TNF$\alpha$ antibody was incubated with anti-human IgG antibody, the retention time of the labeled anti-TNF$\alpha$ antibody/anti-IgG antibody complex was shifted, and the amount of anti-IgG antibody that caused the shift could be quantitated with HPLC. Moreover, low serum content in the assay system was shown to have little effect on HPLC analysis. Finally, a standard curve could be generated for the anti-TNF$\alpha$ antibody and HACA/HAHA assays and could be used to quantitate patient serum anti-TNF$\alpha$ antibody or HACA/HAHA levels. Advantageously, the present method provides in certain aspects a mobility shift assay, such as a homogeneous mix and read assay developed to measure both drug and antibodies against the drug. A standard curve was generated for the anti-TNF$\alpha$ biologic Remicade and Humira and also for the HACA antibodies against Remicade. The mobility shift assay format, unlike ELISA, eliminates coating of antigens to solid surface and is not affected by non-specific binding of irrelevant IgGs. The assay format is simple, but very sensitive and can be used to detect all anti-TNF$\alpha$ biologic drugs *(e.g.,* Remicade, Humira, Enbrel and Cimzia) as well as the neutralizing antibody (anti-Remicade™) in patient serum.

**Example 3. Measurement of Human Anti-Chimeric Antibodies (HACA) and Infliximab (IFX) Levels in Patient Serum Using A Novel Mobility Shift Assay.**

**ABSTRACT**

Background: Infliximab (IFX) is a chimeric monoclonal antibody therapeutic against TNF$\alpha$ that has been shown to be effective in treating autoimmune diseases such as rheumatoid arthritis (RA) and inflammatory bowel disease (IBD). However, antibodies against IFX were found in some IFX-treated patients through the detection of human anti-chimeric antibodies (HACA), which may reduce the drug's efficacy or induce adverse effects. Monitoring of HACA and IFX levels in individual patients may help to optimize the dosing and treatment with IFX. Current methods for detecting HACA are based on solid-phase assays, which are limited by the fact that the presence of IFX in the circulation may mask the presence of HACA and, therefore, measurement can only be done at least 8 weeks following a dose of IFX. Moreover, this time-lapse further confounds the assays because of the rapid clearance of the high molecular weight immune complexes in the blood circulation. To overcome these drawbacks, we have developed and evaluated a new method to measure serum IFX and HACA levels in patients treated with IFX.

**Methods:** A novel non-radiolabeled, liquid-phase, size-exclusion (SE)-HPLC mobility shift assay was developed to measure the HACA and IFX levels in serum from patients treated with IFX. The immuno-complex *(e.g.,* TNF$\alpha$/IFX or IFX/HACA), free TNF$\alpha$ or IFX, and the ratio of bound/free can be resolved and calculated with high sensitivity. Serum concentrations of IFX or HACA were determined with standard curves generated by incubating with different concentrations of IFX or pooled HACA-positive serum. Using this novel assay, we have measured IFX and HACA levels in sera collected from IBD patients treated with IFX who had relapsed and compared the results with those obtained by the traditional Bridge ELISA assay.

**Results:** Dose-response curves were generated from the novel assay with high sensitivity. Detection of HACA was demonstrated in the presence of excess IFX. In the 117 serum samples from patients treated with IFX, 65 samples were found to have IFX levels above the detection limit and the average was 11.0+6.9 mg/mL. For HACA levels, 33 (28.2%) samples were found to be positive while the Bridge ELISA assay detected only 24 positive samples. We also identified 9 false negatives and 9 false positives from the samples determined by the Bridge assay. HACA levels were found to be increased in 11 patients during the course of IFX treatment while the IFX levels were found to be significantly decreased.

**Conclusions:** A novel non-radiolabeled, liquid-phase, mobility shift assay has been developed to measure the IFX and HACA levels in serum from patients treated with IFX. The assay has high sensitivity and accuracy, and the obtained results were reproducible. This novel assay can advantageously be used to measure HACA and IFX levels while patients are on therapy.

## INTRODUCTION

Tumor necrosis factor-alpha (TNF$\alpha$) plays a pivotal role in the pathogenesis of autoimmune diseases such as Crohn's disease (CD) and rheumatoid arthritis (RA). It is well documented that blocking TNF$\alpha$ with therapeutic antibodies such as Infliximab (human-murine chimeric monoclonal IgG1$\kappa$) or adalimumab (fully human monoclonal antibody) reduces disease activity in CD and RA. However, about 30-40% of the patients do not respond to anti-TNF$\alpha$ therapy and some patients need higher doses or dosing frequency adjustments due to lack of sufficient response. Differences of drug bioavailability and pharmacokinetics in individual patients may contribute to the failure of the treatment. Immunogenicity of the drugs, which causes patients to develop HACA/HAHA, could result in a range of adverse reactions from mild allergic response to anaphylactic shock. These problems are now recognized by many investigators, drug-controlling agencies, health insurance companies, and drug manufacturers. Furthermore, many patients with secondary response failure to one anti-TNF$\alpha$ drug benefit from a switch to other anti-TNF$\alpha$ drugs, suggesting a role of neutralizing antibodies directed specifically against the protein used for treatment (Radstake et al., Ann. Rheum. Dis., 68(11):1739-45 (2009)). Monitoring of patients for drug and HACA/HAHA levels is therefore warranted so that drug administration can be tailored to the individual patient and prolonged therapies can be given effectively and economically with little or no risk to patients (Bendtzen et al., Scand. J. Gastroenterol., 44(7):774-81 (2009)).

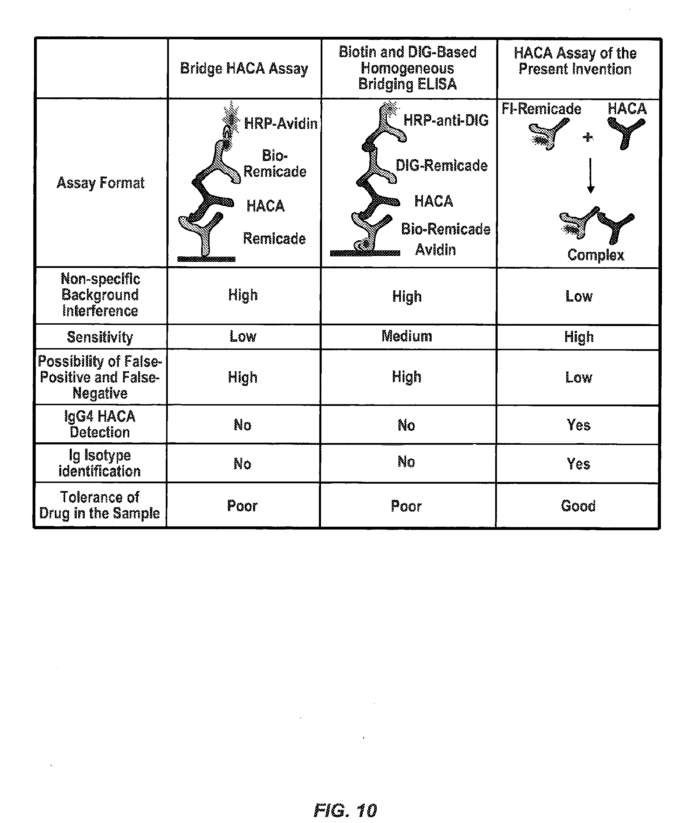

Several enzyme-linked immunoassays have been used to assess the circulating levels of drugs and HACA/HAHA. Figure 10 provides a summary of the current assays available for the measurement of HACA in comparison to the novel HACA assay of the present method. One of the limitations of current methodologies is that antibody levels are difficult to measure when there is a measurable amount of drug in the circulation. In contrast to current solid-phase methods for detecting HACA in which measurements can only be performed at least 8 weeks following a dose of IFX, the novel assay of the present method is a non-radiolabeled, liquid-phase, size-exclusion (SE)-HPLC assay that is capable of measuring HACA and IFX levels in serum from patients while being treated with IFX.

The following are rationales for measuring the serum concentrations of anti-TNF$\alpha$ biologic drugs and antibodies against TNF$\alpha$ biologic drugs in patients: (1) for PK studies in clinical trials; (2) it may be required by the FDA during clinical trials to monitor a patient's immune response to the biologic drug; (3) to monitor a patient's response to the biologic drug by measuring HACA or HAHA to guide the drug dosage for each patient; and (4) for use as a guide for switching to a different biologic drug when the initial drug fails.

## METHODS

*SE-HPLC analysis of Infliximab (IFX) levels in patient serum.* Human recombinant TNF$\alpha$ was labeled with a fluorophore ("Fl" such as, *e.g.,* Alexa Fluor$^{®}$ 488) according to the manufacture's instructions. Labeled TNF$\alpha$ was incubated with different amounts of IFX or patient serum for one hour at room temperature. Samples of 100 $\mu$L volume were analyzed by size-exclusion chromatography on an HPLC system. FLD was used to monitor the free TNF$\alpha$-Fl and the bound TNF$\alpha$-Fl immuno-complex based on their retention times. Serum IFX levels were calculated from the standard curve.

*SE-HPLC analysis of HACA levels in patient serum.* Purified IFX was labeled with Fl. Labeled IFX was incubated with different dilutions of pooled HACA-positive serum or diluted patient serum for one hour at room temperature. Samples of 100 $\mu$L volume were analyzed by size-exclusion chromatography on an HPLC system. FLD was used to monitor the free IFX-F1 and the bound IFX-Fl immuno-complex based on their retention times. The ratio of bound and free IFX- Fl was used to determine the HACA level.

*Mobility Shift Assay Procedure to Measure HACA in Serum.* The principle of this assay is based on the mobility shift of the HACA bound Fl-labeled Infliximab (IFX) complex versus free Fl-labeled IFX on size exclusion-high performance liquid chromatography (SE-HPLC) due to the increase in molecular weight of the complex. The chromatography is performed in an Agilent-1200 HPLC System, using a Bio-Sep 300x7.8 mm SEC-3000 column (Phenomenex) with a molecular weight fractionating range of 5,000-700,000 and a mobile phase of 1X PBS, pH 7.3, at a flow-rate of 0.5-1.0 mL/min with FLD detection. A 100 $\mu$L sample volume is loaded onto the column for each analysis. The HACA bound Fl-labeled IFX complex is formed by incubating serum from IFX treated patient and Fl-labeled IFX in the 1X PBS, pH 7.4, elution buffer at room temperature for 1 hour before SE-HPLC analysis. The assay was also run in the presence of varying interference agents, such as rheumatoid factor and TNF-$\beta$, in order to validate the assay.

## RESULTS

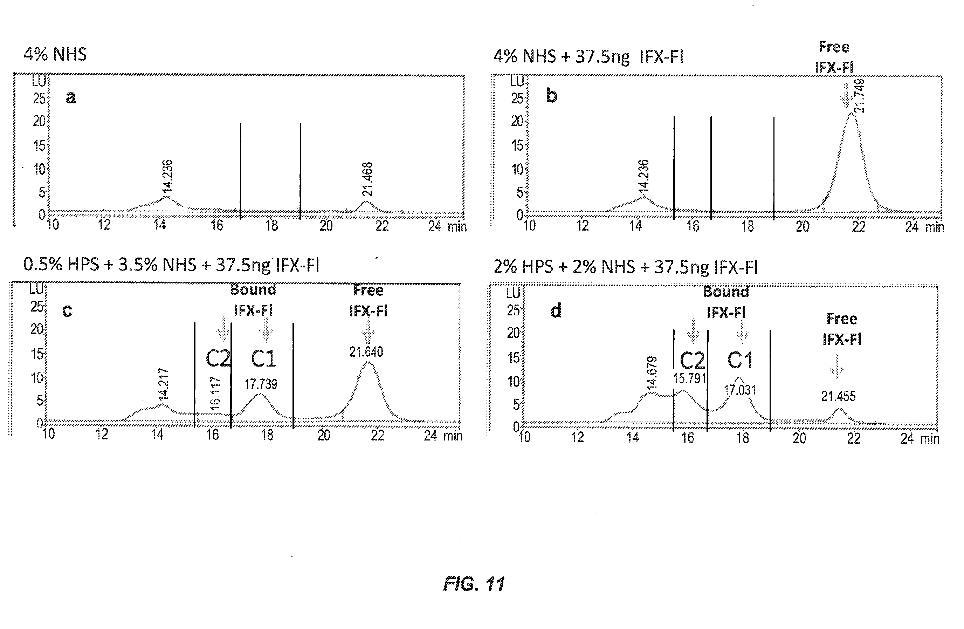

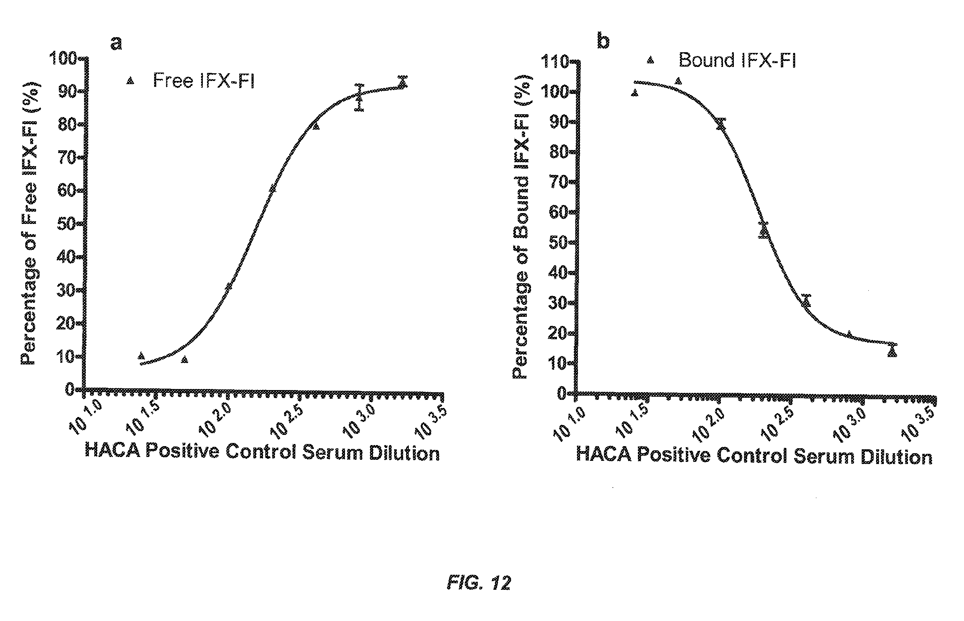

Figure 11 shows the separation of the HACA bound IFX-Fl complex from the free IFX-Fl due to the mobility shift of the high molecular weight complex. As seen in panels c and d, the retention time of the fluorescent peak shifted from 21.8 min to 15.5-19.0 min. The more the HACA is present in the reaction mixture, the less the free IFX-FI remains in the chromatogram and the more the immuno-complex is formed. Figure 12 shows the dose-response curves of the fluorescent peak shift caused by the addition of HACA. Using the HACA positive sample, we could detect the peak shift with 1:1000 dilutions of the serum.

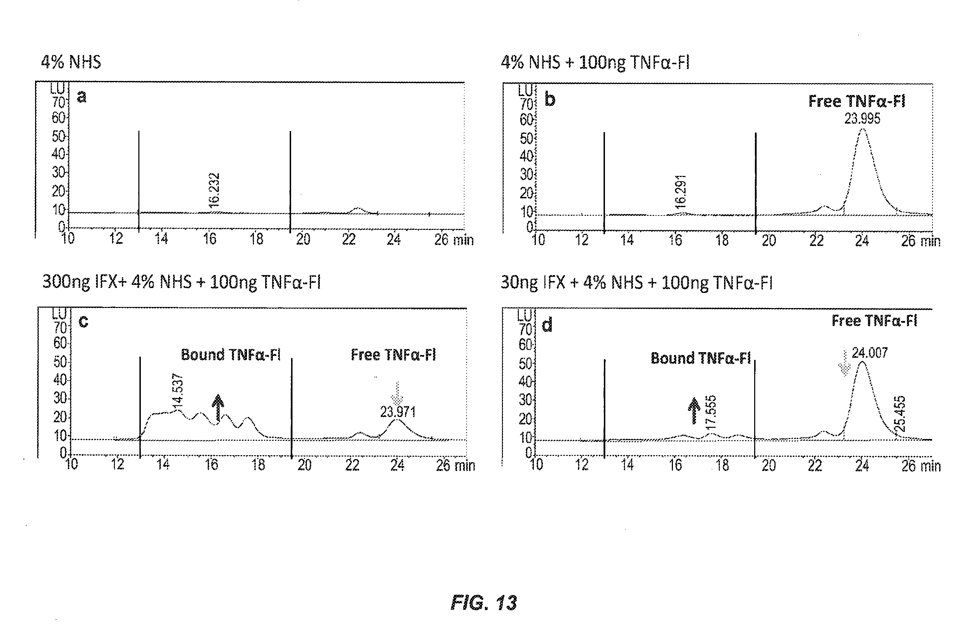

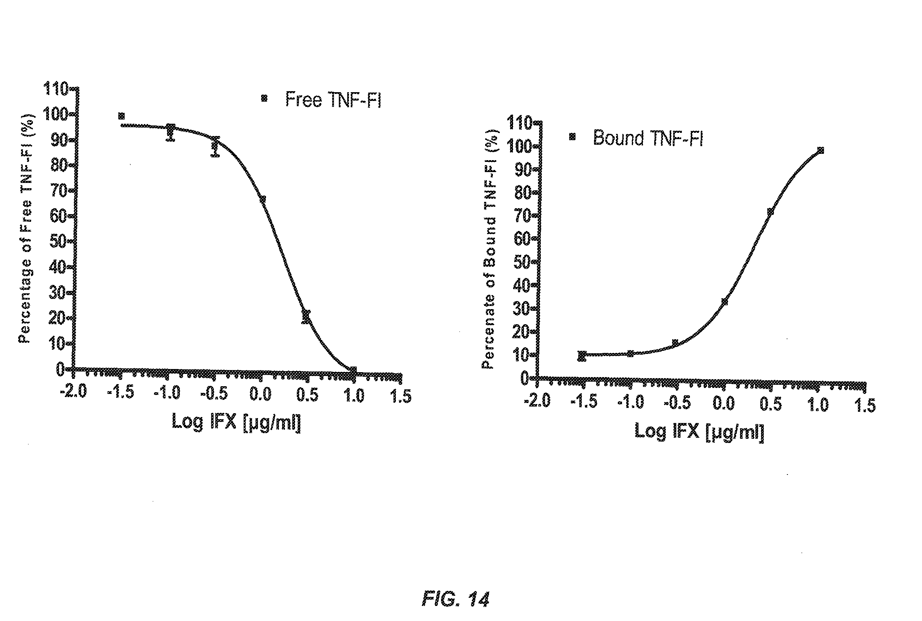

Figure 13 shows the separation of the IFX bound TNF$\alpha$-FI complex from the free TNF$\alpha$-FI due to the mobility shift of the high molecular weight complex. As seen in panels c and d, the retention time of the fluorescent peak shifted from 24 min to 13-19.5 min. The more the IFX is present in the reaction mixture, the less the free TNF$\alpha$-FI remains in the chromatogram and the more the immuno-complex is formed. Figure 14 shows the dose-response curves of the TNF$\alpha$-FI peak shift caused by the addition of IFX. Based on the added IFX, the detection limit is 10 ng/mL of IFX in serum.

The novel mobility shift assay was validated by testing serum samples from HACA positive and negative patients measured by the Bridge assay (Table 4). Using this assay, we have analyzed serum samples from 50 healthy subjects and 117 IBD patients treated with IFX. All 50 healthy subject samples have an IFX level below the limit of detection, whereas 65 of the patient samples have an average IFX concentration of 11.0 $\mu$g/ml. Table 5 shows the HACA levels in the serum of healthy controls and IBD patients treated with IFX measured by the Bridge assay and the mobility shift assay. The Bridge assay detected less HACA-positive patients than the mobility shift assay and more false negatives as well as more false positives.

Table 4. Correlation of Relative HACA Levels in Patient Serum from Strong Positive and Negative on Bridge Assay to SE-HPLC Assay.

|  | Bridge assay | HPLC shift assay | Correlation |
|---|---|---|---|
| **Positive** | 82 | 81 | 99% |
| **Negative** | 12 | 12 | 100% |

Table 5. Patient Sample Analysis on Serum Levels of HACA with Bridge Assay (Cut Off 1.69 $\mu$g/ml) and HPLC Shift Assay (Cut Off 0.19, Ratio of Bound and Free IFX).

|  | Subjects (n) | HACA Positive | | Bridge Assay | |
|---|---|---|---|---|---|
|  |  | Bridge Assay | HPLC Assay | False Negative | False Positive |
| **Healthy Control** | 50 | N/A | 0 | N/A | N/A |
| **Patient treated with IFX** | 117 | 24 (20.5%) | 33 (28.2%) | 9 (High IFX) | 9 |

False negative results are caused by patient serum containing high levels of IFX which interferes with the Bridge assay on HACA determination while the SE-HPLC assay is not affected. False positive results are caused by patient serum containing high levels of non-specific interference substance which may interfere with the Bridge assay.

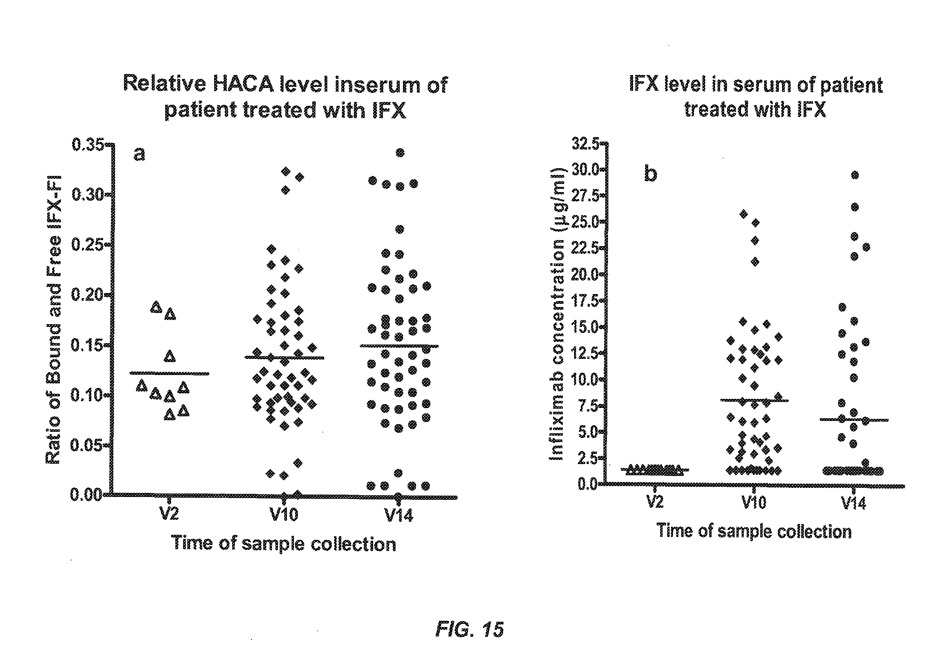

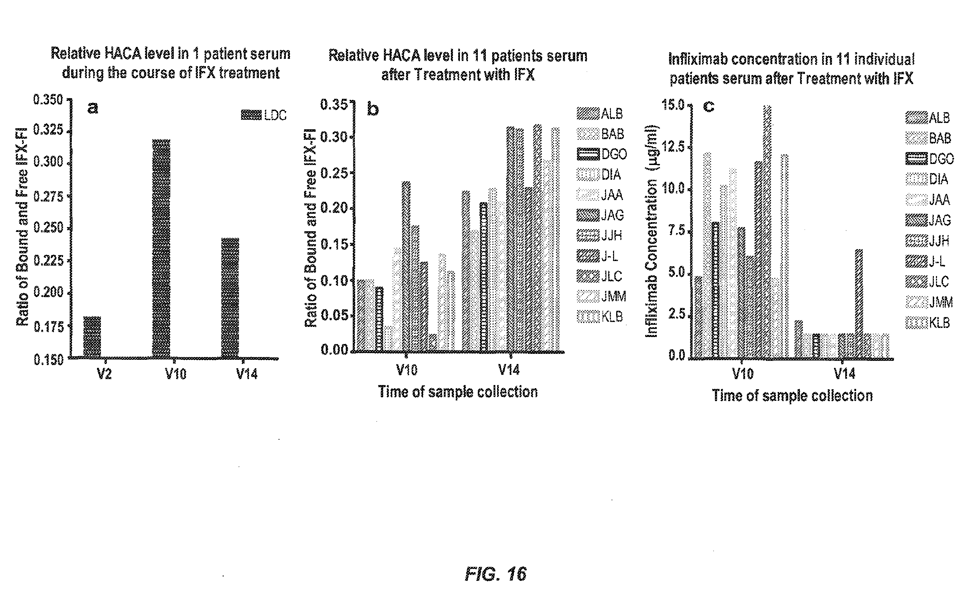

Figure 15 shows the relationship of the HACA level and IFX concentration in IBD patients during the course of IFX treatment. HACA could be detected as early as V10 (30 Weeks) and continued to increase in some patients during IFX treatment. Figure 16 shows that HACA can be detected in the presence of IFX using the assay. A higher level of HACA in the serum was associated with a lower level of IFX that could be detected (e.g., reduced the bioavailability). As such, early detection of HACA while on treatment with IFX can guide the physician and/or patient to switch to other anti-TNF drugs or increase the dose of IFX.

The assays were validated in terms of intra-and inter-assay precision (based on the CV parameter) and susceptibility to interference agents. This analysis is presented below:

Infliximab assay / HACA assay precision tables

| Infliximab assay | | HACA assay | |
|---|---|---|---|
| Parameter | CV% | Parameter | CV% |
| Intra-assay Precision | 2.89 | Intra-assay Precision | 3.96 |
| Inter-assay Precision | | Inter-assay Precision | |
| Run to Run | 4.57 | Run to Run | 4.15 |
| Analyst to Analyst | 6.06 | Analyst to Analyst | 5.84 |
| Instrument to Instrument | 2.73 | Instrument to Instrument | 6.88 |

| Infliximab assay | | | |
|---|---|---|---|
| Interference Agent | Typical Range | Concentration tested | Interference |
| IgG., IgA, IgM | 0.4-16 mg/mL | 10, 2.0, 1.5 mg/mL | NA |
| ATI | 3.71-150 U/mL (0-60 $\mu$g/mL) | 100 U/mL ($\sim$ 55 $\mu$g/mL) | Interferes with detection of low concentration IFX samples (<5 $\mu$g/mL) |
| Rheumatoid Factor | >30 IU/mL (RA positive patients) | Up to 387 IU/mL | NA |
| TNF-$\alpha$ | 6.2-6.6 pg/mL | 0.0125 ng/mL-40 $\mu$g/mL | 100 ng/ml |
| TNFR1/TNFR2 | 1.9/4.5 ng/mL | 0.1-1000ng/mL | NA |
| Hemolyzed Serum | >20 HI | 100-300 HI | NA |
| The following agents were also tested and did not show interference: Azathioprine, Methotrexate, TNF-$\beta$, Lipemic serum, Hemoglobin | | | |

| HACA assay | | | |
|---|---|---|---|
| Interference Agent | Typical Range | Concentration tested | Interference |
| IgG, IgA, IgM | 0.4-16 mg/mL | 10,2.0,1.5 mg/mL | NA |
| Infliximab | 0-100 $\mu$g/mL | 0.78-100 $\mu$g/mL | NA |
| Rheumatoid Factor | >30 IU/mL (RA positive patients) | Up to 774 IU/mL | NA |
| TNF-$\alpha$ | 6.2-6.6 pg/mL | 0.0125 ng/mL-40 $\mu$g/mL | 250 ng/mL |
| TNFR1/TNFR2 | 1.9/4.5 ng/mL | 0.1-1000 ng/mL | NA |
| Hemolyzed Serum | >20 HI | 100-300 HI | NA |
| The following agents were also tested and did not show interference: Azathioprine, Methotrexate, TNF-$\beta$, Lipemic serum, Hemoglobin | | | |

## CONCLUSION

Anti-TNF$\alpha$ biologic drugs can be readily labeled with a fluorophore ("Fl") and the 5 mobility shift assay format used for measuring HACA/HAHA is a homogeneous assay without the coating of antigens to a solid surface and multiple washing and incubation steps like a typical ELISA. Incubation of Fl-labeled IFX with HACA-positive serum results in the formation of an immune complex which elutes at a different position compared to free Fl-labeled IFX in SE-HPLC and thus the amount of HACA can be quantitated. The presence of other serum components has little effect on the mobility shift assay. The mobility shift assay format, unlike ELISA, is not affected by non-specific binding of irrelevant IgGs and detects the IgG4 isotype. Healthy serum samples do not cause mobility shift of the Fl-labeled IFX and 28.2% of the patients treated with IFX were found to have HACA by the assay. As such, the assay format described herein is very sensitive and can be applied to detect all biologic drugs *(e.g.,* Remicade, Humira, Enbrel and Cimzia) as well as their antibodies *(e.g.,* anti-Remicade, anti-Humira, anti-Enbrel and anti-Cimzia) in patient serum. Notably, since HACA can be detected in the presence of IFX using the mobility shift assay , early detection of HACA while on treatment with IFX can guide the physician and/or patient to switch to other anti-TNF drugs or increase the subsequent dose of IFX.

We have developed a novel non-radiolabeled, liquid-phase, SE-HPLC assay to measure the IFX and HACA levels in serum samples obtained from patients treated with IFX. The novel assay has high sensitivity, accuracy, and precision, and the results are highly reproducible, which makes this assay suitable for routine testing of a large number of human serum samples. The new assay format, unlike ELISA, eliminates coating of antigens to solid surfaces and is not affected by non-specific binding of irrelevant IgGs. These advantages of the assay format described herein reduce the false negative and false positive results of the test. Advantageously, the assay format is very sensitive and can be used to detect all biologic drugs as well as their antibodies present in the serum while the patient is on therapy.

**Example 4. Differentiation Between Neutralizing and Non-Neutralizing Human Anti-Chimeric Antibodies (HACA) in Patient Serum Using Novel Mobility Shift Assays.**

This example illustrates novel homogeneous assays for measuring autoantibody *(e.g.,* HACA) concentrations in a patient sample *(e.g.,* serum) and for determining whether such autoantibodies are neutralizing or non-neutralizing autoantibodies using size exclusion chromatography to detect the binding of these autoantibodies to fluorescently labeled anti-TNF$\alpha$ drug in the presence of fluorescently labeled TNF$\alpha$. These assays are advantageous because they obviate the need for wash steps which remove low affinity HACA, use distinct fluorophores that allow for detection on the visible and/or IR spectra which decreases background and serum interference issues, increase the ability to detect neutralizing or non-neutralizing HACA in patients with a low titer due to the high sensitivity of fluorescent label detection, and occur as a liquid phase reaction, thereby reducing the chance of any changes in the epitope by attachment to a solid surface such as an ELISA plate.

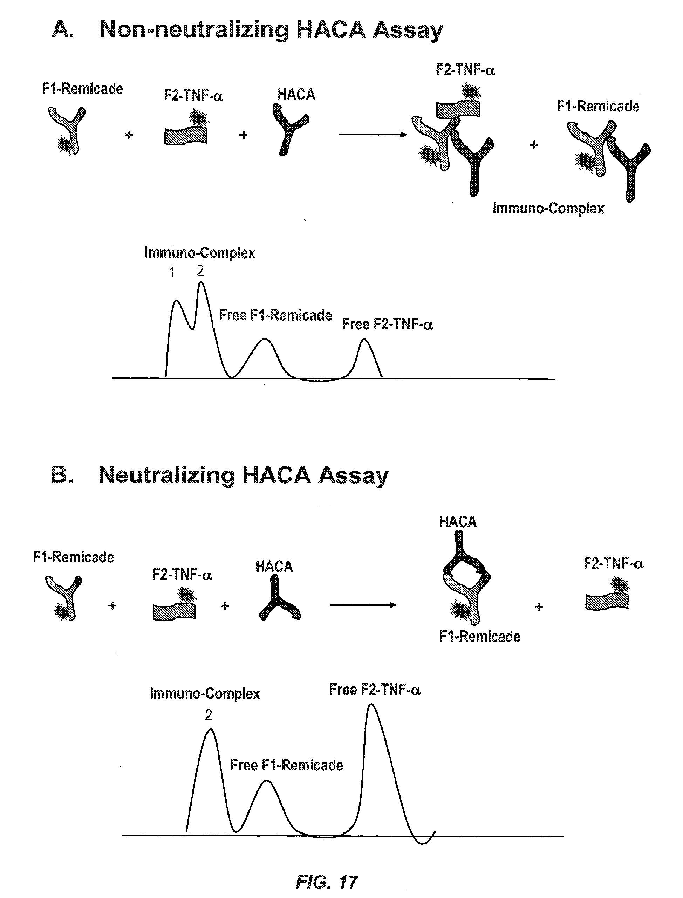

In one exemplary method, an anti-TNF$\alpha$ drug *(e.g.,* REMICADE™) is labeled with a fluorophore "F1" (*see, e.g.,* Figure 17A), wherein the fluorophore can be detected on either or both the visible and IR spectra. Similarly, TNF$\alpha$ is labeled with a fluorophore "F2" (*see, e.g.,* Figure 17A), wherein the fluorophore can also be detected on either or both the visible and IR spectra, and wherein "F1" and "F2" are different fluorophores. The labeled anti-TNF$\alpha$ drug is incubated with human serum in a liquid phase reaction and the labeled TNF$\alpha$ is added to the reaction to allow the formation of complexes *(i.e.,* immuno-complexes) between the labeled anti-TNF$\alpha$ drug, labeled TNF$\alpha$, and/or HACA present in the serum. Following incubation, the samples are loaded directly onto a size exclusion column. Binding of both the autoantibody *(e.g.,* HACA) and the labeled TNF$\alpha$ to the labeled anti-TNF$\alpha$ drug results in a leftward shift of the peak *(e.g.,* "Immuno-Complex 1" in Figure 17A) compared to a binary complex between the autoantibody and the labeled anti-TNF$\alpha$ drug *(e.g.,* "Immuno-Complex 2" in Figure 17A), the labeled drug alone, or the labeled TNF$\alpha$ alone. The presence of this ternary complex of autoantibody *(e.g.,* HACA), labeled TNF$\alpha$, and labeled anti-TNF$\alpha$ drug indicates that the autoantibody present in the serum sample is a non-neutralizing form of the autoantibody *(e.g.,* HACA), such that the autoantibody does not interfere with the binding between the anti-TNF$\alpha$ antibody and TNF$\alpha$. In one particular method , as shown in Figure 17A, if non-neutralizing HACA is present in the serum, a shift will be observed for both F1-REMICADE™ and F2-TNF$\alpha$, resulting in an increase in both the Immuno-Complex 1 and Immuno-Complex 2 peaks and a decrease in the free F1-REMICADE™ and free F2-TNF$\alpha$ peaks. However, the presence of the binary complex between the autoantibody *(e.g.,* HACA) and the labeled anti-TNF$\alpha$ drug *(e.g.,* "Immuno-Complex 2" in Figure 17B) in the absence of the ternary complex of autoantibody *(e.g.,* HACA), labeled TNF$\alpha$, and labeled anti-TNF$\alpha$ drug indicates that the autoantibody present in the serum sample is a neutralizing form of the autoantibody *(e.g.,* HACA), such that the autoantibody interferes with the binding between the anti-TNF$\alpha$ antibody and TNF$\alpha$. In one particular method , as shown in Figure 17B, if neutralizing HACA is present in the serum, a shift will be observed for F1-REMICADE™, resulting in an increase in the Immuno-Complex 2 peak, a decrease in the free F1-REMICADE™ peak, and no change in the free F2-TNF$\alpha$ peak. In certain instances, the presence of neutralizing HACA indicates that the current therapy with REMICADE™ should be switched to another anti-TNF$\alpha$ drug such as HUMIRA™.

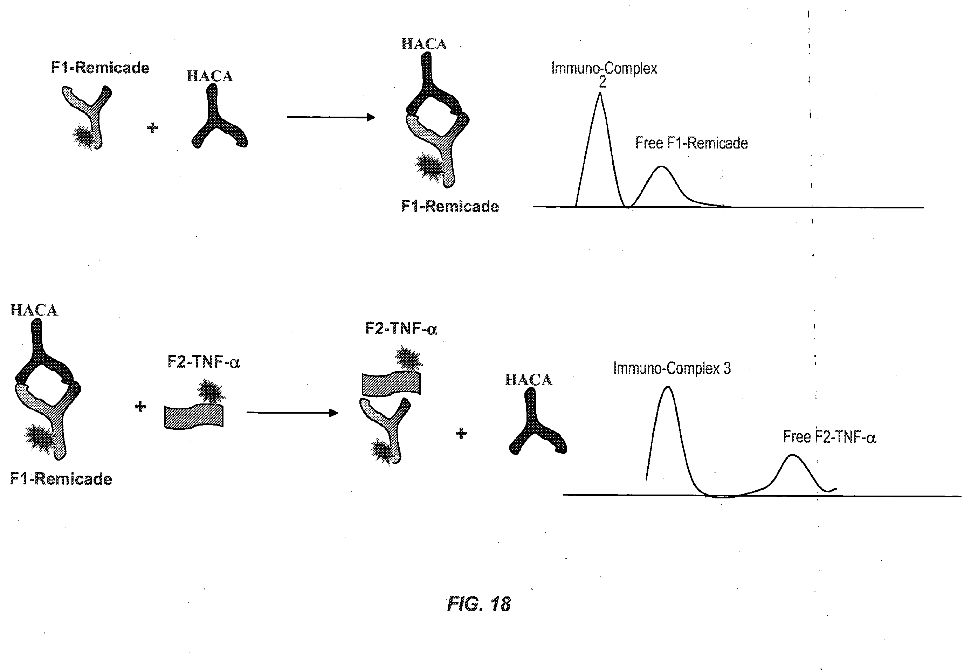

In an alternative method , the labeled anti-TNF$\alpha$ drug is first incubated with human serum in a liquid phase reaction to allow the formation of complexes *(i.e.,* immuno-complexes) between the labeled anti-TNF$\alpha$ drug and HACA present in the serum. Following incubation, the samples are loaded directly onto a first size exclusion column. Binding of the autoantibody *(e.g.,* HACA) to the labeled anti-TNF$\alpha$ drug results in a leftward shift of the peak *(e.g.,* "Immuno-Complex 2" in Figure 18) compared to the labeled drug alone. The labeled TNF$\alpha$ is then added to the reaction to determine whether it is capable of displacing (e.g., competing with) the autoantibody (e.g., HACA) for binding to the labeled anti-TNFα drug, to thereby allow the formation of complexes (i.e., immuno-complexes) between the labeled anti-TNFα drug and the labeled TNFα. Following incubation, the samples are loaded directly onto a second size exclusion column. Binding of the labeled anti-TNFα drug to the labeled TNFα results in a leftward shift of the peak (e.g., "Immuno-Complex 3" in Figure 18) compared to the labeled TNFα alone. Disruption of the binding between the autoantibody (e.g., HACA) and the labeled anti-TNFα drug by the addition of the labeled TNFα indicates that the autoantibody present in the serum sample is a neutralizing form of the autoantibody (e.g., HACA), such that the autoantibody interferes with the binding between the anti-TNFα antibody and TNFα. In certain instances, the presence of neutralizing HACA indicates that the current therapy with REMICADE™ should be switched to another anti-TNFα drug such as HUMIRA™.

**Example 5. Analysis of Human Anti-Drug Antibodies (ADA) to Adalimumab in Patient Serum Using a Novel Homogeneous Mobility Shift Assay.**

**Background and Aim:** Monoclonal antibodies against TNF-α such as infliximab (IFX), adalimumab (HUMIRA™), and certolizumab have been shown to be effective in treating inflammatory bowel disease (IBD) and other inflammatory disorders. Anti-drug antibodies (ADA) may reduce the drug's efficacy and/or induce adverse effects. However, ADAs have been found not only in patients treated with the chimeric antibody infliximab, but also in patients treated with the humanized antibody adalimumab. Monitoring of ADA and drug levels in individual patients may help optimize treatment and dosing of the patient. We have developed a non-radio labeled liquid-phase homogeneous mobility shift assay to accurately measure in the serum both HACA (Human Anti-Chimeric Antibody) and IFX from patients. This assay method overcomes a major limitation of the current solid-phase assays for detecting HACA, namely the inability to accurately detect HACA in the presence of IFX in circulation. In the present study, we have evaluated this new method for measuring serum ADA and drug levels in patients treated with the humanized antibody drug, adalimumab.

**Methods:** The mobility shift assay was based on the shift in retention time of a free antigen versus antigen-antibody immunocomplex on size-exclusion separation. Fluorophore-labeled adalimumab or TNF-α and internal control were mixed with serum samples to measure the mobility shift of free adalimumab and TNF-α in the presence of ADA or drug. The changes in the ratio of free adalimumab or TNF-α to internal control are indicators of immunocomplex formation. Serum concentrations of ADA or adalimumab were determined with standard curves generated by incubating with different concentrations of anti-human IgG antibody or purified adalimumab. Using the mobility shift assay, we measured adalimumab and ADA levels in sera collected from IBD patients treated with adalimumab who had lost response.

**Results:** Dose-response curves were generated with anti-human IgG antibody for the measurement of mobility shift of labeled adalimumab. The detection limit of the assay was 1 ng of anti-human IgG. Sera from fifty healthy controls were tested for ADA and all of the samples had ADA levels below the detection limit (i.e., no shift of the free labeled-adalimumab). Detection of ADA was also demonstrated in the presence of exogenously added adalimumab. To measure the drug concentration in patients treated with adalimumab, we generated a standard curve with different amounts of adalimumab on the mobility shift of labeled TNF-α, and the detection limit of adalimumab was 10 ng.

**Conclusions:** The non-radio labeled liquid-phase homogeneous mobility shift assay of the present method has been applied to measure ADA and adalimumab levels in serum samples from patients treated with adalimumab. The assay is found to be reproducible with high sensitivity and accuracy, and can be used to evaluate ADA levels in serum samples from patients treated with adalimumab.

**Example 6. Analysis of Anti-Drug Antibodies (ADA) to Adalimumab in Patient Serum Using A Novel Proprietary Mobility Shift Assay.**

**ABSTRACT**

**Background:** Anti-TNF-α drugs such as infliximab (IFX) and adalimumab (ADL) have been shown to be effective in treating inflammatory bowel disease (IBD). However, induction of ADA in the treated patients may reduce the drug's efficacy and/or induce adverse effects. Indeed, ADAs have been found not only in patients treated with IFX, but also in patients treated with ADL. Monitoring of ADA and drug levels in individual patients may help to optimize treatment and dosing of the patient. We have developed a proprietary mobility shift assay to accurately measure in the serum both HACA (Human Anti-Chimeric Antibody) and IFX from IFX-treated patients. This assay overcomes the major limitation of the current solid-phase assays for detecting HACA, namely the inability to accurately detect HACA in the presence of IFX in circulation. In the present study, we have evaluated this new assay to measure serum ADA and drug levels in patients treated with the fully human antibody drug, ADL.

**Methods:** The mobility shift assay was based on the shift in retention time of the antigen-antibody immunocomplex versus free antigen on size-exclusion chromatography. Fluorophore-labeled ADL or TNF-α and internal control were mixed with serum samples to measure the mobility shift of labeled ADL and TNF-α in the presence of ADA or drug. The changes in the ratio of free ADL or TNF-α to internal control are the indicators of the immunocomplex formation. Serum concentrations of ADA or ADL were determined with standard curves generated by incubating with different concentrations of anti-human IgG antibody or purified ADL. Using this assay, we measured ADL and ADA levels in sera collected from IBD patients treated with ADL.

**Results:** Dose-response curves were generated with anti-human IgG antibody for the measurement of mobility shift of labeled ADL. The detection limit of the assay was 10 ng of anti-human IgG. Sera from 100 healthy controls were tested for the ADA and all of the samples had an ADA level below detection limit (no shift of free labeled ADL). Detection of ADA was demonstrated in five out of 114 IBD patient samples treated with ADL. To measure the drug concentration in patients treated with ADL, we generated a standard curve with different amounts of ADL on the shift of labeled TNF-α with the detection limit of 10 ng.

**Conclusions:** We have applied our proprietary non-radio labeled liquid-phase homogeneous mobility shift assay to measure the ADA and ADL levels in serum from patients treated with ADL. The assays are reproducible with high sensitivity and accuracy, and are useful for evaluating ADA levels in serum samples from patients treated with ADL.

## INTRODUCTION

Anti-tumor necrosis factor-alpha (TNF-α) biologics such as infliximab (IFX), etanercept, adalimumab (ADL) and certolizumab pegol have been shown to reduce disease activity in a number of autoimmune diseases, including Crohn's Disease (CD) and rheumatoid arthritis (RA). However, some patients do not respond to anti-TNF-α therapy, while others need higher or more frequent dosage due to lack of sufficient response, or develop infusion reactions.

Immunogenicity of therapeutic antibodies which causes the patients to develop antibodies against the drugs may contribute to the failure of the treatments and infusion reactions. Chimeric antibodies like IFX have a higher potential of inducing antibody generation compared to fully humanized antibodies such as ADL. The prevalence of antibodies to IFX (HACA) in RA patients varies from 12% to 44% and seems to be inversely proportional to the level of IFX in patient serum and therapeutic response. While the fully humanized ADL is supposed to be less immunogenic than murine or chimeric antibodies, several studies have reported the formation of human anti-humanized antibodies (HAHA) and showed the prevalence of antibody generation from 1% to 87% in RA and CD patients (Aikawa et al., Immunogenicity of Anti-TNF-alpha agents in autoimmune diseases. Clin. Rev. Allergy Immunol., 38(2-3):82-9 (2010)).

Many patients with secondary response failure to one anti-TNF-α drug may benefit from switching to another anti-TNF-α drug or increasing dosage and/or dosing frequency. Monitoring of patients for drug and anti-drug antibody (ADA) levels is therefore warranted so that drug administration can be tailored to the individual patient. This approach allows dose adjustment when warranted or cessation of medication when ADA levels are present. (Bendtzen et al., Individual medicine in inflammatory bowel disease: monitoring bioavailability, pharmacokinetics and immunogenicity of anti-tumour necrosis factor-alpha antibodies. Scand. J. Gastroenterol., 44(7):774-81 (2009); Afif et al., Clinical utility of measuring infliximab and human anti-chimeric antibody concentrations in patients with inflammatory bowel disease. Am. J. Gastroenterol., 105(5):1133-9 (2010)).

A number of assays have been developed to measure HACA and HAHA. One of the limitations of the current methodologies is that ADA levels cannot be reliably measured when there is a high level of drugs in the circulation.

We have developed a proprietary non-radiolabeled, liquid-phase, mobility shift assay to measure the ADA and ADL levels in serum from patients treated with ADL which is not affected by the presence of the drug in the serum.

## METHODS

Fluorophore (Fl)-labeled ADL was incubated with patient serum to form the immunocomplex. A Fl-labeled small peptide was included as an internal control in each reaction. Different amounts of anti-human IgG were used to generate a standard curve to determine the serum ADA level. Free Fl-labeled ADL was separated from the antibody bound complex based on its molecular weight by size-exclusion chromatography. The ratio of free Fl-labeled ADL to internal control from each sample was used to extrapolate the HAHA concentration from the standard curve. A similar methodology was used to measure ADL levels in patient serum samples with Fl-labeled TNF-α.

## RESULTS

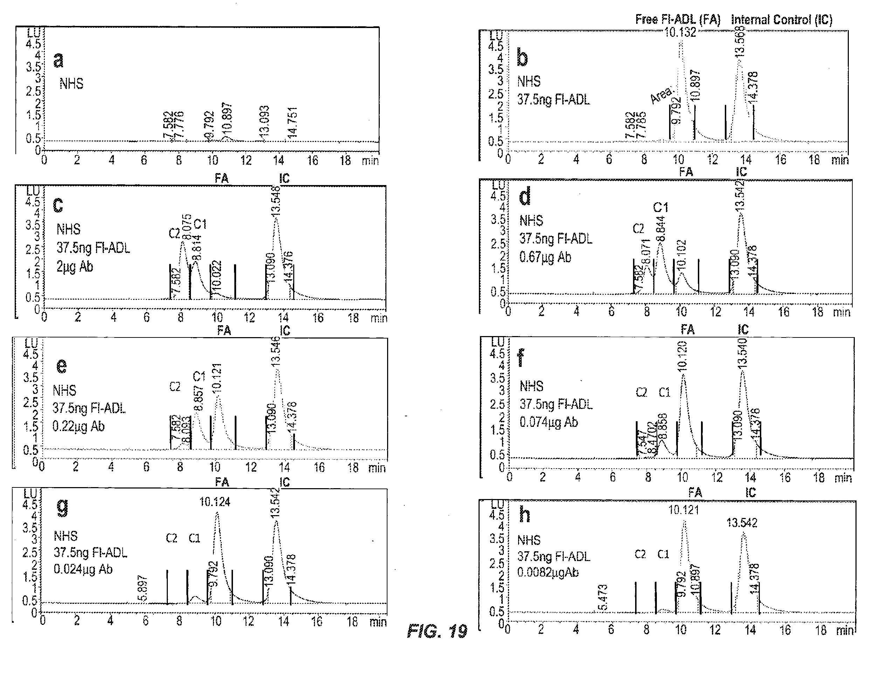

Figure 19 shows the separation of the anti-human IgG bound Fl-ADL complex from the free Fl-ADL due to the mobility shift of the high molecular weight complex. As seen in panels c to h, the retention time of the fluorescent peak shifted from 10.1 min to 7.3-9.5 min. The more the anti-human IgG is added in the reaction mixture, the less the free ADL remains in the chromatogram and the more the immunocomplex is formed (h to c). The retention time for the internal control is 13.5 min.

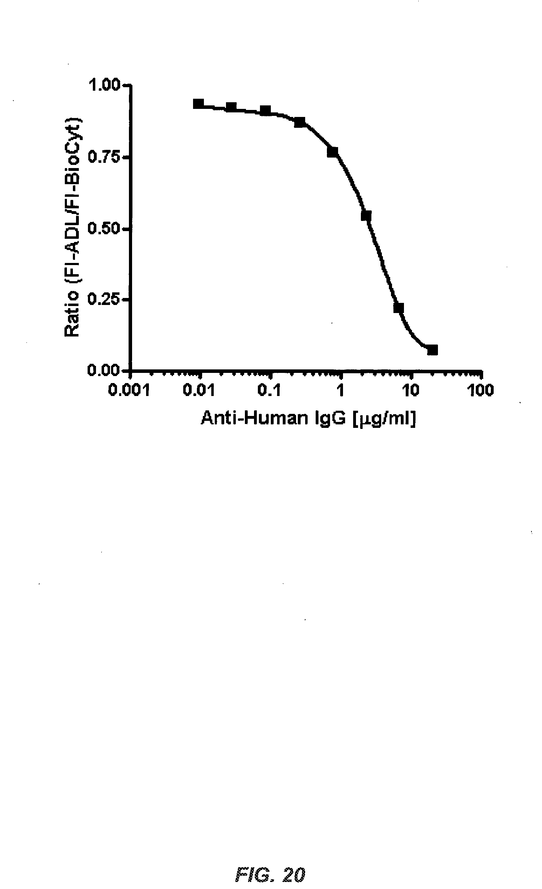

Figure 20 shows the dose-response curve of the fluorescent peak shift caused by the addition of anti-human IgG. Increasing the concentration of anti-human IgG reduces the ratio of free ADL to internal control due to the formation of the immunocomplex. The assay sensitivity is 10ng/ml of anti-human IgG. The internal control "Fl-BioCyt" corresponds to an Alexa Fluor® 488-biocytin (BioCyt) which combines the green-fluorescent Alexa Fluor® 488 fluorophore with biotin and an aldehyde-fixable primary amine (lysine) (Invitrogen Corp.; Carlsbad, CA).

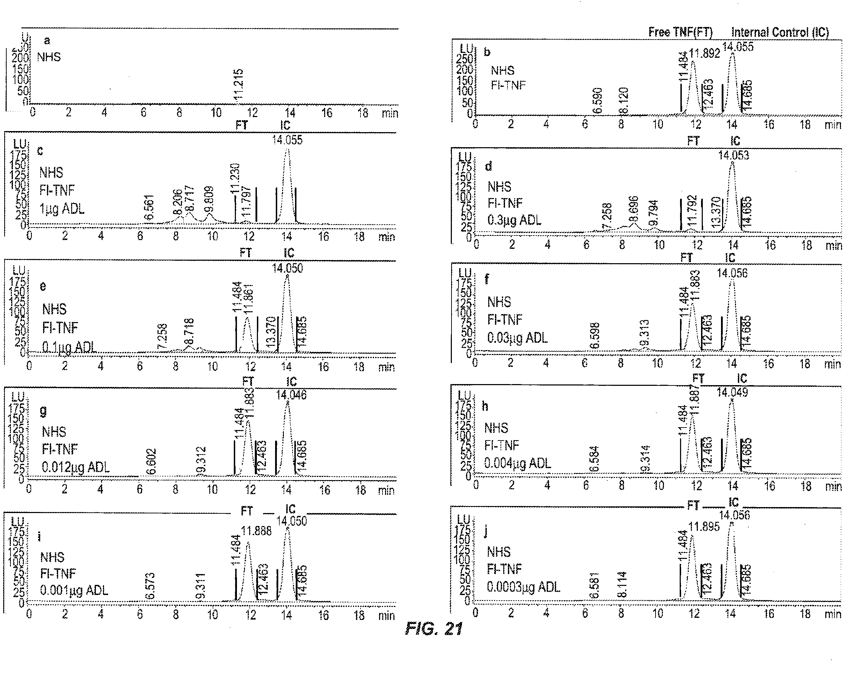

Figure 21 shows the separation of the ADL bound TNF-$\alpha$-Fl complex from the free TNF-$\alpha$-Fl due to the mobility shift of the high molecular weight complex. As seen in panels c and j, the retention time of the fluorescent peak shifted from 11.9 min to 6.5- 10.5 min. The more the ADL is added in the reaction mixture, the less the free TNF-$\alpha$-Fl peak remains in the chromatogram and the more the immuno-complex is formed.

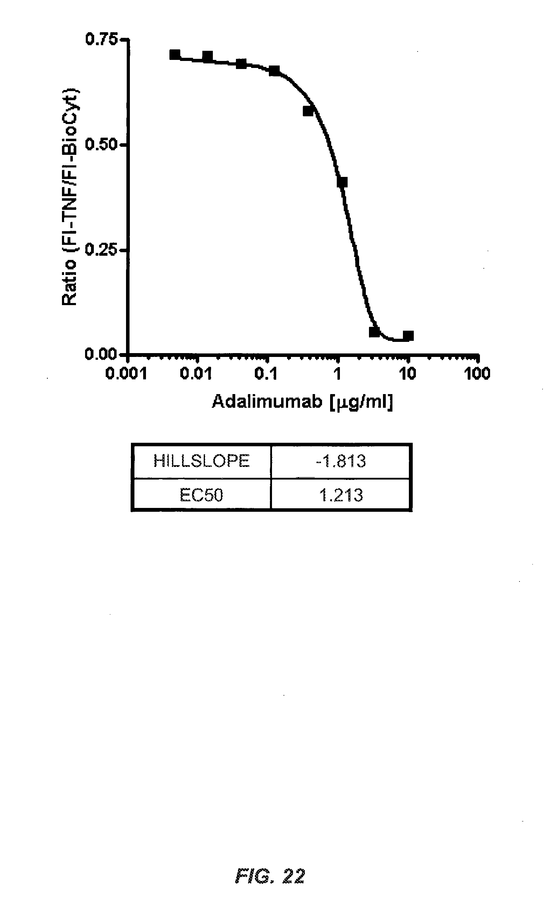

Figure 22 shows the dose-response curves of the TNF-$\alpha$-Fl peak shift caused by the addition of ADL. Based on the added ADL, the detection limit is 10 ng/mL of ADL in serum.

Table 6 shows that serum samples from 100 healthy subjects and 114 IBD patients treated with ADL were analyzed for ADA and ADL levels using the mobility shift assay. All 100 healthy subject samples had ADA levels below the limit of detection (no shift of the free F1-ADL), whereas 5 out of the 114 patient samples had an ADA concentration of 0.012 to >20 $\mu$g/ml. The mean of ADL levels in 100 healthy subject samples was 0.76±1.0 $\mu$g/ml (range 0 to 9.4 $\mu$g/ml). The mean of ADL levels in 114 serum samples from patients treated with ADL was 10.8+17.8 $\mu$g/ml (range 0 - 139 $\mu$g/ml). Four out of five ADA positive samples had undetectable levels of ADL.

Table 6. Patient Serum Levels of ADA and ADL Measured by the Mobility Shift Assay

|  | Subjects (n) | Sex (M/F) | Age (Years) (Mean) | ADA Positive | ADL level ($\mu$g/ml) |
|---|---|---|---|---|---|
| **Healthy Control** | 100 | 38/62 | 18-62 (37.1) | 0 | 0.76±1.00 |
| **IBD Patient Treated with ADL** | 114 | 51/63 | 20-69 (39.9) | 5(4.3%) | 10.80±17.80 |

## CONCLUSIONS

The mobility shift assay format used for measuring HACA/IFX is a homogeneous assay without the coating of antigens to a solid surface, and without multiple washing and incubation steps like a typical ELISA. This assay can be applied to measure ADA and anti-TNF drugs. The sensitivity of the assay (in $\mu$g/ml range) is higher for both ADA and ADL measurement with patient serum compared to ELISA methods (in mg/ml range). Healthy control serum samples did not cause mobility shift of the Fl-labeled ADL, and 4.3% of the patients treated with ADL were found to have ADA by this assay. Although healthy control serum samples caused mobility shift of the Fl-labeled TNF-$\alpha$, which may have been due to the presence of soluble free receptor of TNF-$\alpha$, the average of ADL in patients treated with ADL was much higher (10.8 vs. 0.76 mg/ml). Early detection of ADA and monitoring of ADL drug level while the patient is receiving ADL treatment will allow the physician to optimize the dosing of ADL or switch to another anti-TNF-$\alpha$ drug when appropriate and, thereby, optimizing the overall management of the patient's disease.

Table 7. Patient Serum Levels of ADA and ADL Measured by the Mobility Shift Assay

|  | Subjects (n) | Sex (M/F) | Age (Mean) | ADL Level ($\mu$g/ml) | ADA Positive |
|---|---|---|---|---|---|
| Healthy Control | 100 | 38/62 | 18-62 (37.1) | 0.76 ± 1.00 | 0 |
| IBD Patient Treated with ADL | 114 | 51/63 | 20-69 (39.9) | 10.80 ± 17.80 | 0-4 $\mu$g/ml ADL: 4 of 42 (9.52%) |
| ▪ Using this mobility shift assay we analyzed serum samples from 100 healthy subjects, and 114 IBD patients treated with ADL ,for ADA and ADL levels. All 100 healthy subject samples had ADA levels below the limit of detection (no shift of the free F1-ADL), whereas 4 out of the 42 patient samples with 0-4$\mu$g/mL ADL had an average ADA concentration of 0.012 to >20 $\mu$g/ml. Mean ADL levels in 100 healthy subject samples was 0.76+1.0 mg/ml (range 0 to 9.4 mg/ml). Mean ADL levels in 114 serum samples from patients treated with ADL was 10.8+17.8 mg/ml (range 0 - 139 mg/ml). Four out of four ADA positive samples had undetectable levels of ADL. For the detection of ADA, the 114 IBD patients treated with ADL were divided into two categories, 0-4$\mu$g/ml of ADL and >4$\mu$g/ml of ADL. Patients with greater than 4$\mu$g/ml of ADL will be tested with a larger amount of ADL-FI to address the competition of circulating ADL with ADL-FI. | | | | | |

Healthy control serum samples do not cause mobility shift of the Fl-labeled ADL. In a preliminary study, 9.52% of patients with 0.4 $\mu$g/ml ADL were found to have ADA in this assay.

**Example 7: Determining the Concentration Levels of REMICADE™ and Human Anti-Drug Antibodies.**

This example describes a method for determining the levels of Anti-TNF$\alpha$ Drugs, e.g. REMICADE™ (infliximab), in a serum sample as well as for determining the levels of a human anti-drug antibody, e.g. a human anti-chimeric antibody (HACA) to REMICADE™ (infliximab).

**Step 1: Determining concentration level of REMICADE™ (infliximab) in a sample.**

TNF$\alpha$ can be labeled with a fluorophore (e.g. Alexa$_{647}$), wherein the fluorophore can be detected by, either or both of, the visible and fluorescent spectra. The labeled TNF$\alpha$ is incubated with human serum in a liquid phase reaction to allow the anti-TNF$\alpha$ drug present in the serum to bind. The labeled TNF$\alpha$ can also be incubated with known amounts of the anti-TNF$\alpha$ drug in a liquid phase reaction to create a standard curve. Following incubation, the samples are loaded directly onto a size exclusion column. Binding of the anti-TNF$\alpha$ drug to the labeled TNF$\alpha$ results in a leftward shift of the peak compared to labeled TNF$\alpha$ alone. The concentration of the anti-TNF$\alpha$ drug present in the serum sample can then be compared to the standard curve and controls.

**SE-HPLC analysis of REMICADE™ (infliximab) levels in patient serum.** Human recombinant TNF$\alpha$ was labeled with a fluorophore, Alexa Fluor® 488, according to the manufactureR's instructions. Labeled TNF$\alpha$ was incubated with different amounts of REMICADE™ or patient serum for one hour at room temperature. Samples of 100 $\mu$L volume were analyzed by size-exclusion chromatography on an HPLC system. Fluorescence label detection was used to monitor the free labeled TNF$\alpha$ and the bound labeled TNF$\alpha$ immuno-complex based on their retention times. Serum REMICADE™ levels were calculated from the standard curve.

The following equations are relevant to this assay:

$$\textbf{Equation I:} \quad \text{labeled-TNF}\alpha + \text{REMICADE}^{\text{TM}} \rightarrow (\text{labeled-TNF}\alpha \bullet \text{REMICADE}^{\text{TM}})_{\text{complex}}$$

$$\textbf{Equation II:} \quad [\text{REMICADE}^{\text{TM}}]_{\text{without-labeled-TNF}\alpha\text{-present}} = [(\text{labeled-TNF}\alpha \bullet \text{REMICADE}^{\text{TM}})_{\text{complex}}]\;.$$

$$\textbf{Equation III:} \quad [\text{REMICADE}^{\text{TM}}] = [(\text{labeled-TNF}\alpha \bullet \text{REMICADE}^{\text{TM}})_{\text{complex}}]/[\text{labeled-TNF}\alpha] \times [\text{labeled-TNF}\alpha]$$

In **Step 1,** a known amount of the labeled-TNF$\alpha$ is contacted with a REMICADE™-containing serum sample. The labeled-TNF$\alpha$ and the REMICADE™ form a complex, (labeled-TNF$\alpha$•REMICADE™)$_{\text{complex}}$, **See Equation I.** Because almost all of the REMICADE™ will form a complex with the labeled-TNF$\alpha$, the concentration of REMICADE™ present before introduction of the labeled-TNF$\alpha$ is equal to the measured concentration of labeled-TNF$\alpha$•REMICADE™$_{\text{complex}}$, **See Equation II.** The concentration level of REMICADE™ is calculated by multiplying the ratio of [(label-TNF•REMICADE™)$_{\text{complex}}$/[labeled-TNF$\alpha$] by [labeled-TNF$\alpha$], **See Equation III.** The ratio, [(label-TNF$\alpha$•REMICADE™)$_{\text{complex}}$/[labeled-TNF$\alpha$], is obtained by integrating the area-under-the curve for the (label-TNF$\alpha$•REMICADE™)$_{\text{complex}}$ peak, from a plot of signal intensity as a function of elution time from the size exclusion HPLC, and dividing this number by the resultant integration of the area-under-the-curve for the labeled-TNF$\alpha$ peak from the plot. The [labeled-TNF$\alpha$] is known a priori.

**Step 2: Determining level of human anti-chimeric antibody, HACA.**

An anti-TNF$\alpha$ drug, e.g., REMICADE™ can be labeled with a fluorophore, e.g., Alexa$_{647}$, wherein the fluorophore can be detected by, either or both of, the visible and fluorescent spectra. The labeled anti-TNF$\alpha$ drug is incubated with human serum in a liquid phase reaction to allow any HACA present in the serum to bind. The labeled anti-TNF$\alpha$ drug can also be incubated with known amounts of an anti-IgG antibody or pooled positive patient serum in a liquid phase reaction to create a standard curve. Following incubation, the samples are loaded directly onto a size exclusion column. Binding of the autoantibodies to the labeled anti-TNF$\alpha$ drug results in a leftward shift of the peak compared to labeled drug alone. The concentration of HACA present in the serum sample can then be compared to the standard curve and controls.

**SE-HPLC analysis of HACA levels in patient serum.** Purified REMICADE™ was labeled with a fluorophore. Labeled REMICADE™ was incubated with different dilutions of pooled HACA-positive serum or diluted patient serum for one hour at room temperature. Samples of 100 $\mu$L volume were analyzed by size-exclusion chromatography on an HPLC system. Fluorescence label detection was used to monitor the free labeled REMICADE™ and the bound labeled REMICADE™ immuno-complex based on their retention times. The ratio of bound and free labeled REMICADE™ was used to determine the HACA level as described below.

**Mobility Shift Assay Procedure to Measure HACA in Serum.** The principle of this assay is based on the mobility shift of the complex of an anti-drug antibody, *e.g.* HACA, with $Alexa_{647}$-labeled REMICADE™ relative to free $Alexa_{647}$-labeled REMICADE™, on size exclusion-high performance liquid chromatography (SE-HPLC) due to the increase in molecular weight of the complex. The chromatography is performed in an Agilent-1200 HPLC System, using a Bio-Sep 300x7.8 mm SEC-3000 column (Phenomenex) with a molecular weight fractionating range of 5,000-700,000 and a mobile phase of 1X PBS, pH 7.3, at a flow-rate of 0.5 - 1.0 mL/min with fluorescence label detection, *e.g.* UV detection at 650 nm. In front of the Agilent-1200 HPLC System with a Bio-Sep 300x7.8 mm SEC-3000 column is a analytical pre-column which is a BioSep 75x7.8 mm SEC-3000. A 100 $\mu$L sample volume is loaded onto the column for each analysis. The complex of HACA and labeled REMICADE™ complex is formed by incubating serum from a REMICADE™-treated patient and labeled REMICADE™ in the 1X PBS, pH 7.3, elution buffer at room temperature for 1 hour before SE-HPLC analysis.

The following equations are relevant to this assay:

$$\text{Equation IV: REMICADE}^{TM} + \text{labeled-REMICADE}^{TM} + \text{HACA} \rightarrow (\text{REMICADE}^{TM}\bullet\text{HACA})_{complex} + (\text{Labeled-REMICADE}^{TM}\bullet\text{HACA})_{complex}$$

$$\text{Equation V: } [\text{REMICADE}^{TM}]/[\text{REMICADE}^{TM}\bullet\text{HACA}_{complex}] = [\text{labeled-REMICADE}^{TM}]/[\text{Labeled-REMICADE}^{TM}\bullet\text{HACA}_{complex}]$$

$$\text{Equation VI: } [\text{HACA}] = [\text{REMICADE}^{TM}\bullet\text{HACA}]_{complex} + [\text{labeled-REMICADE}^{TM}\bullet\text{HACA}]_{complex}$$

$$\text{Equation VII: } [\text{REMICADE}^{TM}\bullet\text{HACA}_{complex}] = [\text{REMICADE}^{TM}] \times [\text{labeled-REMICADE}^{TM}\bullet\text{HACA}_{complex}]/[\text{labeled-REMICADE}^{TM}]$$

$$\text{Equation VIII: } [\text{labeled-REMICADE}^{TM}\bullet\text{HACA}_{complex}] = [\text{labeled-REMICADE}^{TM}] \times [\text{labeled-REMICADE}^{TM}\bullet\text{HACA}_{complex}]/[\text{labeled-REMICADE}^{TM}]$$

$$\text{Equation IX: } [\text{REMICADE}^{TM}]_{effective-amount} = [\text{REMICADE}^{TM}] - [\text{HACA}]$$

**Determining the concentration levels of human anti-TNF$\alpha$ drug antibodies, *e.g.* HACA.** A known concentration of Labeled-REMICADE™ is added to a serum sample. HACA forms a complex with either REMICADE™ or Labeled-REMICADE™, **See Equation IV.** The [REMICADE™] is determined in Step 1 above. By integrating the area-under-the-curve for the labeled-REMICADE™•HACA$_{complex}$ and dividing this number by the resultant integration for the the area-under-the-curve for the free Labeled-REMICADE™, the ratio of [labeled-REMICADE™•HACA$_{complex}$] to [labeled-REMICADE™] is obtained. The ratio of [REMICADE™] to [REMICADE™•HACA$_{complex}$] is equal to the ratio of [labeled-REMICADE™] to [labeled-REMICADE™•HACA)$_{complex}$], **See Equation V.** Because HACA equilibrates and forms a complex with both REMICADE™ and Labeled-REMICADE™, the total amount of HACA equals the sum of the amount of REMICADE™•HACA$_{complex}$ and the amount of labeled-REMICADE™•HACA$_{complex}$, See **Equation VI.** Because the ratio of [REMICADE™] to [REMICADE™•HACA$_{complex}$] is equal to the ratio of [labeled-REMICADE™] to [labeled-REMICADE™•HACA$_{complex}$], both the [REMICADE™-HACA]$_{complex}$ and the [labeled-REMICADE™-HACA$_{complex}$] are determined by multiplying the ratio of the [labeled-REMICADE™•HACA$_{complex}$)]/ [labeled-REMICADE™] by, respectively, the concentration amount of REMICADE™, determined in **Step 1,** and the concentration amount of labeled-REMICADE™, known *a priori,* See **Equations VII and VIII.** Therefore, the total amount of HACA equals the sum of (1) the [REMICADE™], from step 1, multiplied by [labeled-REMICADE™•HACA)$_{complex}$]/[labeled-REMICADE™], and (2) the [labeled REMICADE™], known *a priori,* multiplied by [labeled-REMICADE™•HACA)$_{complex}$]/ [labeled-REMICADE™].

**Determining the effective concentration levels of REMICADE™.** Because HACA complexes with REMICADE™, the effective amount of REMICADE™ available in a serum sample is the amount of REMICADE™, measured from **Step 1,** minus the amount of HACA, measured from **Step 2, See Equation IX.**

**Exemplary calculation.** In patient JAG on V10, the [REMICADE™] was determined to be 7.5 μg/ml, See Figure 16c. This result was obtained by following **Step 1** and using **Equtions I-III.** 7.5 μg/ml equals 30 ng/ 4 μL. Since 4 μL of sample was used in the measurement in **Step 2,** a total of 30.0 ng of REMICADE™ was present in the sample analyzed. The ratio of [labeled-REMICADE™•HACA]$_{complex}$/[labeled-REMICADE™] for patient JAG on V10 was 0.25, See Figure 16b. The [labeled-REMICADE™] introduced into the sample was 37.5 ng/ 100μL. Since 100μL of the labeled-REMICADE™ was used in the measurement in **Step 2,** a total of 37.5 ng of labeled-REMICADE™ was present in the sample analyzed. Using **Equation VII,** the total amount of REMICADE™•HACA$_{complex}$ was 30 ng multiplied by 0.25, which is equal to 7.5 ng labeled-REMICADE™•HACA$_{compelx}$. Using **Equation VIII,** the total amount of labeled-REMICADE™•HACA$_{complex}$ was 37.5 ng multiplied by 0.25, which is equal to 9.4 ng labeled-REMICADE™•HACA$_{compelx}$. Using **Equation VI,** the total amount of HACA equals the sum of 9.4 ng and 7.5 ng, which equals 16.9 ng HACA. The 16.9 ng HACA was present in 4 μL of sample. The [HACA] was 16.9 ng/4μL, which equals 4.23 μg/ml. Using **Equation IX,** the effective amount of REMICADE™ is equal to 7.5 μg/ml REMICADE™, determined from **Step 1,** minus 4.23 μg/ml HACA, determined from **Step 2.** In this exemplary calculation, the effective [REMICADE™] was equal to 3.27μg/ml.

**Example 8: Determining the Concentration Levels of HUMIRA™ and Human Anti-Drug Antibodies.**

This example describes a method for determining the levels of HUMIRA™ in a serum sample as well as for determining the levels of human anti-human antibodies (HAHA).

**Step 1: Determining concentration level of HUMIRA™ in a sample.**

TNFα can be labeled with a fluorophore (*e.g.* Alexa$_{647}$), wherein the fluorophore can be detected by, either or both of, the visible and fluorescent spectra. The labeled TNFα is incubated with human serum in a liquid phase reaction to allow the anti-TNFα drug present in the serum to bind. The labeled TNFα can also be incubated with known amounts of the anti-TNFα drug in a liquid phase reaction to create a standard curve. Following incubation, the samples are loaded directly onto a size exclusion column. Binding of the anti-TNFα drug to the labeled TNFα results in a leftward shift of the peak compared to labeled TNFα alone. The concentration of the anti-TNFα drug present in the serum sample can then be compared to the standard curve and controls.

**SE-HPLC analysis of HUMIRA™ levels in patient serum.** Human recombinant TNFα was labeled with a fluorophore, Alexa Fluor® 488, according to the manufacturer's instructions. Labeled TNFα was incubated with different amounts of HUMIRA™ or patient serum for one hour at room temperature. Samples of 100 μL volume were analyzed by size-exclusion chromatography on an HPLC system. Fluorescence label detection was used to monitor the free labeled TNFα and the bound labeled TNFα immuno-complex based on their retention times. Serum HUMIRA™ levels were calculated from the standard curve.

The following equations are relevant to this assay:

$$\text{Equation X:} \quad \text{labeled-TNF}\alpha + \text{HUMIRA}^{\text{TM}} \rightarrow (\text{labeled-TNF}\alpha \bullet \text{HUMIRA}^{\text{TM}})_{\text{complex}}$$

$$\text{Equation XI:} \quad [\text{HUMIRA}^{\text{TM}}] = [(\text{labeled-TNF}\alpha \bullet \text{HUMIRA})_{\text{complex}}]$$

**Equation XII:** $[\text{HUMIRA}^{TM}] = [(\text{label-TNF}\alpha \bullet \text{HUMIRA}^{TM})_{\text{complex}}]/[\text{labeled-TNF}\alpha] \times [\text{labeled-TNF}\alpha]$

In Step 1, a known amount of the labeled-TNFα is contacted with a HUMIRA™-containing serum sample. The labeled-TNFα and the HUMIRA™ form a complex, (labeled-TNFα•HUMIRA™)$_{\text{complex}}$, **See Equation X.** Because almost all of the HUMIRA™ will form a complex with the labeled-TNFα, the [HUMIRA™] present before introduction of the labeled-TNFα is equal to the measured [(labeled-TNFα•HUMIRA™)$_{\text{complex}}$], **See Equation XI.** The [HUMIRA™] is calculated by multiplying the ratio of [(label-TNFα•HUMIRA™)$_{\text{complex}}$/[Labeled-TNFα] by [labeled-TNFα], **See Equation XII.** By integrating the area-under-the-curve for the labeled-TNFα and the area-under-the-curve for the (labeled-TNFα•HUMIRA™)$_{\text{complex}}$ and dividing the resultant integration for (labeled-TNFα•HUMIRA™)$_{\text{complex}}$ by the resultant integration for the labeled-TNFα, the ratio of [(label-TNFα•HUMIRA™)$_{\text{complex}}$] to [labeled-TNFα] is obtained. The [labeled-TNFα] is known *a priori.*

**Step 2: Determining level of human anti-human antibody, *e.g.* HAHA.** An anti-TNFα drug, *e.g.,* HUMIRA™, can be labeled with a fluorophore, *e.g.,* Alexa$_{647}$, wherein the fluorophore can be detected by, either or both of, the visible and fluorescent spectra. The labeled anti-TNFα drug is incubated with human serum in a liquid phase reaction to allow any HAHA present in the serum to bind. The labeled anti-TNFα drug can also be incubated with known amounts of an anti-IgG antibody or pooled positive patient serum in a liquid phase reaction to create a standard curve. Following incubation, the samples are loaded directly onto a size exclusion column. Binding of the autoantibodies to the labeled anti-TNFα drug results in a leftward shift of the peak compared to labeled drug alone. The concentration of HAHA present in the serum sample can then be compared to the standard curve and controls.

**SE-HPLC analysis of HAHA levels in patient serum.** Purified HUMIRA™ was labeled with a fluorophore. Labeled HUMIRA™ was incubated with different dilutions of pooled HAHA-positive serum or diluted patient serum for one hour at room temperature. Samples of 100 μL volume were analyzed by size-exclusion chromatography on an HPLC system. Fluorescence label detection was used to monitor the free labeled HUMIRA™ and the bound labeled HUMIRA™ immuno-complex based on their retention times. The ratio of bound and free labeled HUMIRA™ was used to determine the HAHA level as described below.

**Mobility Shift Assay Procedure to Measure HAHA in Serum.** The principle of this assay is based on the mobility shift of the antibody, e.g. HAHA, bound Alexa$_{647}$-labeled HUMIRA™ complex versus free Alexa$_{647}$-labeled HUMIRA™ on size exclusion-high performance liquid chromatography (SE-HPLC) due to the increase in molecular weight of the complex. The chromatography is performed in an Agilent-1200 HPLC System, using a Bio-Sep 300x7.8 mm SEC-3000 column (Phenomenex) with a molecular weight fractionating range of 5,000-700,000 and a mobile phase of 1X PBS, pH 7.3, at a flow-rate of 0.5-1.0 mL/min with fluorescence label detection, e.g. UV detection at 650 nm. In front of the Agilent-1200 HPLC System with a Bio-Sep 300x7.8 mm SEC-3000 column is a analytical pre-column which is a BioSep 75x7.8 mm SEC-3000. A 100 μL sample volume is loaded onto the column for each analysis. A 100 μL sample volume is loaded onto the column for each analysis. The HAHA bound labeled HUMIRA™ complex is formed by incubating serum from a HUMIRA-treated patient and labeled HUMIRA™ in the 1X PBS, pH 7.3, elution buffer at room temperature for 1 hour before SE-HPLC analysis.

**Equation XIII:** $\text{HUMIRA}^{TM} + \text{labeled-HUMIRA}^{TM} + \text{HAHA} \rightarrow (\text{HUMIRA}^{TM} \bullet \text{HAHA})_{\text{complex}} + (\text{labeled-HUMIRA}^{TM} \bullet \text{HAHA})_{\text{complex}}$

**Equation XIV:** $[\text{HUMIRA}^{TM}]/[\text{HUMIRA}^{TM} \bullet \text{HAHA}_{\text{complex}}] = [\text{labeled-HUMIRA}^{TM}]/[\text{labeled-HUMIRA} \bullet \text{HAHA}_{\text{complex}}]$

**Equation XV:** $[\text{HAHA}] = [\text{HUMIRA}^{TM} \bullet \text{HAHA}_{\text{complex}}] + [\text{labeled-HUMIRA}^{TM} \bullet \text{HAHA}_{\text{complex}}]$

**Equation XVI:** $[\text{HUMIRA}^{TM} \bullet \text{HAHA}_{\text{complex}}] = [\text{HUMIRA}^{TM}] \times [\text{labeled-HUMIRA}^{TM} \bullet \text{HAHA}_{\text{complex}}]/[\text{labeled-HUMIRA}^{TM}]$

**Equation XVII:** $[\text{labeled-HUMIRA}^{TM} \bullet \text{HAHA}_{complex}] = [\text{labeled-HUMIRA}^{TM}] \times [\text{labeled-HUMIRA}^{TM} \bullet \text{HAHA}_{complex}]/[\text{labeled-HUMIRA}^{TM}]$

**Equation XVIII:** $[\text{HUMIRA}^{TM}]_{effective\text{-}amount} = [\text{HUMIRA}^{TM}] - [\text{HAHA}]$

Calculation for **Step 2:** A known concentration of labeled-HUMIRA™ is added to a serum sample. HAHA forms a complex with either HUMIRA™ or Labeled-HUMIRA™, **See Equation XIII.** The [HUMIRA™] is determined in Step 1 as described above. By integrating the area-under-the-curve for the Labeled-HUMIRA™•HAHA$_{complex}$ and the area-under-the-curve for the Labeled-HUMIRA™ and dividing the resultant integration for the Labeled-HUMIRA™•HAHA$_{complex}$ by the resultant integration for the Labeled-HUMIRA™, the ratio of the [Labeled-HUMIRA™•HAHA$_{complex}$] to [Labeled-HUMIRA™] is obtained. The ratio of the [HUMIRA™] to the [HUMIRA™•HAHA$_{complex}$] is equal to the ratio of the [Labeled-HUMIRA™] to the [Labeled-HUMIRA™•HAHA$_{complex}$], **See Equation XIV.** Because HAHA equilibrates and forms a complex with both HUMIRA and Labeled-HUMIRA™, the total amount of HAHA equals the sum of the amount of HUMIRA™•HAHA$_{complex}$ and the Labeled-HUMIRA™•HAHA$_{complex}$, **See Equation XV.** Because the ratio of [HUMIRA™] to [HUMIRA™•HAHA$_{complex}$] is equal to the ratio of [Labeled-HUMIRA] to [Labeled-HUMIRA™•HAHA$_{complex}$], the concentration of both the [HUMIRA™-HAHA$_{complex}$] and the [Labeled-HUMIRA™-HAHA$_{complex}$] are determined by multiplying the ratio of the [Labeled-HUMIRA•HAHA$_{complex}$]/ [Labeled-HUMIRA] by the [HUMIRA™], determined in **Step 1,** and the [Labeled-HUMIRA™], known *a priori,* respectively, **See Equations XVI and XVII.** Because HAHA complexes with HUMIRA™, the effective amount of HUMIRA™ available in a serum sample is the amount of HUMIRA, measured from **Step 1,** minus the amount of HAHA, measured from **Step 2, See Equation XVIII.**

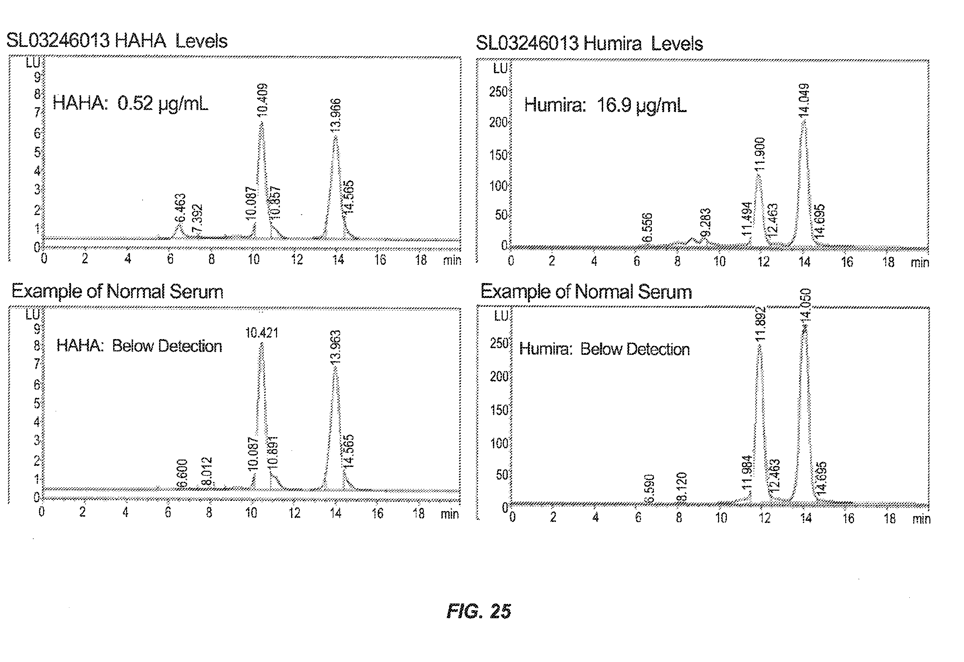

**Exemplary calculation**. In patient SL03246013, see Figure 25, the [HUMIRA™] was determined to be 16.9 $\mu$g/ml, see Figure 25. This result was obtained by following **Step 1** and using **Equtions X-XII.** 16.9 $\mu$g/ml equals 67.6 ng/ 4 $\mu$L. Since 4 $\mu$L of sample was used in the measurement in Step 2, a total of 67.6 ng of HUMIRA™ was present in the sample analyzed. The ratio of [labeled-HUMIRA™•HAHA]$_{complex}$/[labeled-HUMIRA™] for patient SL03246013 was 0.055, see Figure 25. The [labeled-HUMIRA™] introduced into the sample was 37.5 ng/ 100$\mu$L. Since 100$\mu$L of the labeled-HUMIRA™ was used in the measurement in **Step 2,** a total of 37.5 ng of labeled-HUMIRA™ was present in the sample analyzed. Using **Equation XVI,** the total amount of HUMIRA™•HAHA$_{complex}$ was 67.6 ng multiplied by 0.055, which is equal to 3.71 ng labeled-HUMIRA™•HAHA$_{compelx}$. Using **Equation XVII,** the total amount of labeled-HUMIRA™•HAHA$_{complex}$ was 37.5 ng multiplied by 0.055, which is equal to 2.06 ng labeled-HUMIRA™•HAHA$_{compelx}$. Using **Equation XV,** the total amount of HAHA equals the sum of 3.71 ng and 2.06 ng, which equals 5.77 ng HAHA. The 5.77 ng HAHA was present in 4 $\mu$L of sample. The [HAHA] was 5.77 ng/4$\mu$L, which equals 1.44 $\mu$g/ml. Using **Equation XVIII,** the effective amount of HUMIRA™ is equal to 16.99 $\mu$g/ml HUMIRA™, determined from **Step 1,** minus 1.44 $\mu$g/ml HAHA, determined from **Step 2.** In this exemplary calculation, the effective [HUMIRA™] was equal to 15.46 $\mu$g/ml.

**Example 9: Determining the Amount of a Complex of HACA or HAHA with Either REMICADE™, Labeled-REMICADE™, HUMIRA, or Labeled-HUMIRA.**

This example describes a method for determining the amount of a complex of HACA or HAHA with either REMICADE™, Labeled-REMICADE™, HUMIRA, or Labeled-HUMIRA™ with reference to an internal standard.

By using an internal control, e.g. Biocytin-Alexa 488, serum artifacts and variations from one experiment to another experiment can be identified and properly analyzed. The amount of internal control, e.g. Biocytin-Alexa 488, is from about 50 to about 200 pg per 100 $\mu$L analyzed.

Fluorophore (Fl)-labeled HUMIRA™ was incubated with patient serum to form the immunocomplex. A Fl-labeled small peptide, e.g. Biocytin-Alexa 488, was included as an internal control in each reaction. In one instance, different amounts of anti-human IgG were used to generate a standard curve to determine the serum HAHA levels. In another instance, titrated pooled positive patient serum that has been calibrated with purified HAHA was used to generate a standard curve to determine the serum HAHA levels. In yet another instance, the method described in Example 7 was used to generate a standard curve to determine the serum HAHA levels. Free labeled HUMIRA was separated from the antibody bound complex based on its molecular weight by size-exclusion chromatography. The ratio of free labeled HUMIRA to an internal control from each sample was used to extrapolate the HAHA concentration from the standard curve. A similar methodology was used to measure HUMIRA levels in patient serum samples with labeled TNF-$\alpha$.

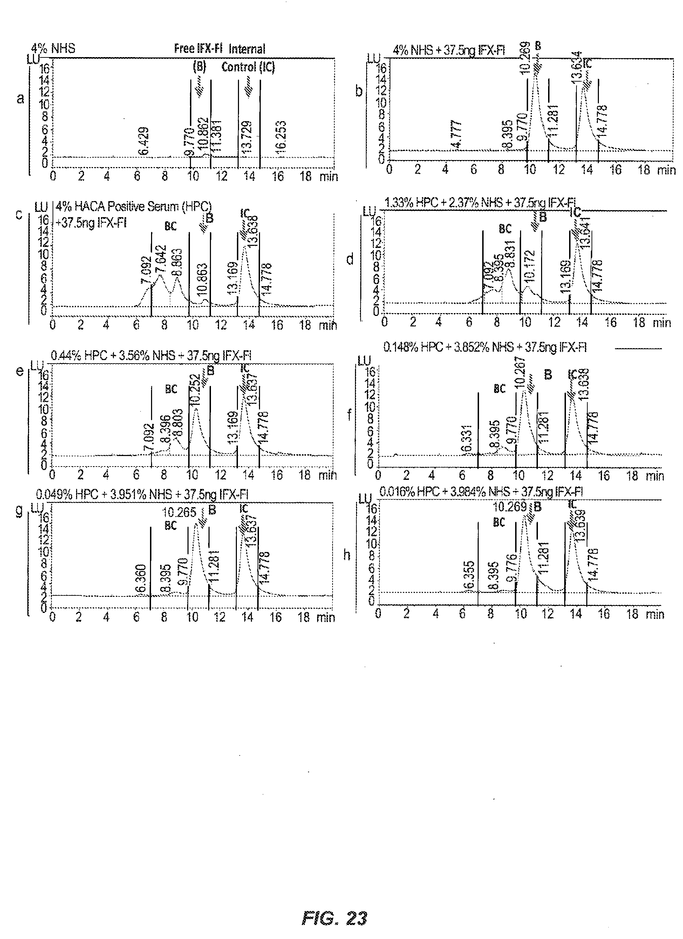

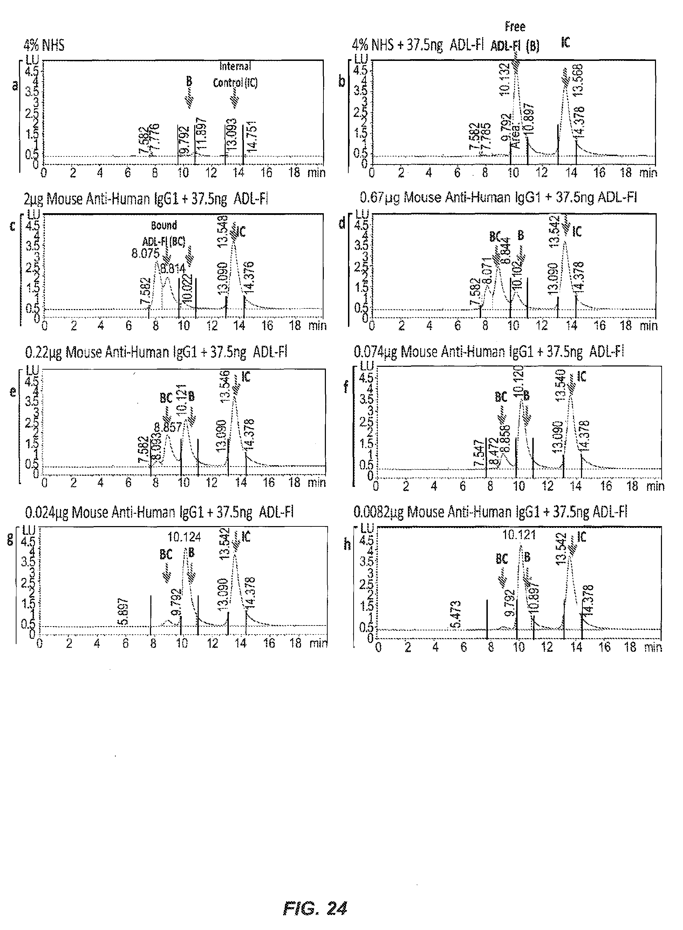

The initial ratio of the Labeled-Drug, *i.e.* Labeled-REMICADE™ or Labeled-HUMIRA, to the internal control is equal to 100. As depicted in Figures 23 and 24, when the ratio of the Labeled-Drug to the internal control falls below 95, the labeled-drug is inferred to be complexed with an anti-Drug binding compound, *e.g.* HACA, HAHA. The ratio of the [Labeled-drug] to [internal control] is obtained by integrating the areas-under-the-curve for the Labeled-Drug and for the internal control and then dividing the resultant integration for the Labeled-Drug by the resultant integration for the internal control.

**Example 10: Determining the Ratio of Complexed Anti-TNF$\alpha$ Drugs to Uncomplexed Anti-TNF$\alpha$ Drugs.**

The ratio of the complexed anti-TNF$\alpha$ drug to uncomplexed anti-TNF$\alpha$ drug is obtained by integrating the areas-under-the-curve for both the complexed anti-TNF$\alpha$ drug and the uncomplexed anti-TNF$\alpha$ drug and then dividing the resultant integration for the complexed anti-TNF$\alpha$ drug by the resultant integration for the uncomplexed anti-TNF$\alpha$ drug.

The uncomplexed anti-TNF$\alpha$ drug can be REMICADE™ having levels between about 0 ng and 100 ng in a sample. The amount of labeled-REMICADE™ is about 37.5 ng.

By using an internal control, *e.g.* Biocytin-Alexa 488, serum artifacts and variations from one experiment to another experiment can be identified and properly analyzed. The amount of internal control, *e.g.* Biocytin-Alexa 488, is from about 50 to about 200 pg per 100 $\mu$L analyzed.

The ratio of the labeled anti-TNF$\alpha$ drug, *e.g.* REMICADE™ or HUMIRA™, to the labeled internal control is obtained by integrating the the areas-under-the-curve for both the labeled anti-TNF$\alpha$ drug and the labeled internal control and then dividing the resultant integration for the labeled anti-TNF$\alpha$ drug by the resultant integration for the labeled internal control.

The ratio of [(labeled-anti-TNF$\alpha$ Drug•Autoantibody)$_{complex}$]/[internal control] is obtained by integrating the area-under-the curve for the (labeled-anti-TNF$\alpha$ drug•Autoantibody)$_{complex}$ peak from a plot of signal intensity as a function of elution time from the size exclusion HPLC, and dividing this number by the resultant integration of the area-under-the-curve for the internal control peak from the plot. The labeled anti-TNF$\alpha$ drug can be labeled REMICADE™. The labeled anti-TNF$\alpha$ drug can be labeled HUMIRA™.

**Example 11: Determining the Ratio of free and complexed labeled TNF$\alpha$.**

This example describes a method for determining the amount of a complex of labeled-TNF$\alpha$ with either REMICADE™ or HUMIRA™ with reference to an internal standard.

By using an internal control, *e.g.* Biocytin-Alexa 488, serum artifacts and variations from one experiment to another experiment can be identified and properly analyzed. The amount of internal control, *e.g.* Biocytin-Alexa 488, is from about 1 to about 25 ng per 100 $\mu$L analyzed.

The uncomplexed labeled TNF$\alpha$ can have levels between about 50 ng and 150 ng in a sample. In certain instances, the amount of labeled-TNF$\alpha$ is about 100.0 ng.

Fluorophore (Fl)-labeled TNF$\alpha$ was incubated with patient serum to form the immunocomplex. A Fl-labeled small peptide, *e.g.* Biocytin-Alexa 488, was included as an internal control in each reaction. A standard curve was created by spiking in known concentrations of purified anti-TNF$\alpha$ drug and then extrapolating from the curve to determine the concentration in units of $\mu$g/mL.

The initial ratio of the Labeled-TNF$\alpha$ to the internal control is equal to 100. When the ratio of the Labeled-TNF$\alpha$ to the internal control falls below 95, the labeled-TNF$\alpha$ is inferred to be complexed with an anti-TNF$\alpha$ drug, *e.g.* Remicade™, Humira™. The ratio of the [Labeled-TNF$\alpha$] to [internal control] is obtained by integrating the areas-under-the-curve for the Labeled-TNF$\alpha$ and for the internal control and then dividing the resultant integration for the Labeled-TNF$\alpha$ by the resultant integration for the internal control.

**Example 12: Optimizing Anti-TNF$\alpha$ Drug Therapy by Measuring Anti-TNF$\alpha$ Drug and/or Anti-Drug Antibody (ADA) Levels.**

This example describes methods for optimizing anti-TNF$\alpha$ drug therapy, reducing toxicity associated with anti-TNF$\alpha$ drug therapy, and/or monitoring the efficacy of therapeutic treatment with an anti-TNF$\alpha$ drug by measuring the amount (*e.g.*, concentration level) of anti-TNF$\alpha$ drug (*e.g.*, level of free anti-TNF$\alpha$ therapeutic antibody) and/or anti-drug antibody (ADA) (*e.g.*, level of autoantibody to the anti-TNF$\alpha$ drug) in a sample from a subject receiving anti-TNF$\alpha$ drug therapy. Accordingly, the methods set forth in the present example provide information useful for guiding treatment decisions, *e.g.*, by determining when or how to adjust or modify (*e.g.*, increase or decrease) the subsequent dose of an anti-TNF$\alpha$ drug, by determining when or how to combine an anti-TNF$\alpha$ drug (*e.g.*, at an increased, decreased, or same dose) with one or more immunosuppressive agents such as methotrexate (MTX) or azathioprine, and/or by determining when or how to change the current course of therapy (*e.g.*, switch to a different anti-TNF$\alpha$ drug).

For purposes of illustration only, the following scenarios provide a demonstration of how the methods advantageously enable therapy to be optimized and toxicity (*e.g.*, side-effects) to be minimized or reduced based upon the level of anti-TNFα drug (*e.g.*, level of free anti-TNFα therapeutic antibody) and/or ADA (*e.g.*, level of autoantibody to the anti-TNFα drug) in a sample from a subject receiving anti-TNFα drug therapy. The levels of the anti-TNFα drug and ADA can be measured with the novel assays described herein.

Scenario #1: High level of anti-TNFα drug with low level of anti-drug antibody (ADA).

Drug levels = 10-50 ng/10μl; ADA levels = 0.1-2 ng/10μl. Patient samples having this profile include samples from patients BAB and JAA on visit 10 ("V10"). *See,* Figure 16b.

Patients receiving anti-TNFα drug therapy and having this particular profile should be treated with immunosuppressive drugs like azathioprine (AZA) along with the anti-TNFα drug (*e.g.*, infliximab).

Scenario #2: Medium level of anti-TNFα drug with low level of ADA.

Drug levels = 5-20 ng/10μl; ADA levels = 0.1-2 ng/10μl. Patient samples having this profile include samples from patients DGO, JAG, and JJH on V10. *See,* Figure 16b.

Patients receiving anti-TNFα drug therapy and having this particular profile should be treated with immunosuppressive drugs like azathioprine (AZA) along with a higher dose of the anti-TNFα drug (*e.g.*, infliximab). One skilled in the art will know of suitable higher or lower doses to which the current course of therapy can be adjusted such that drug therapy is optimized, *e.g.*, a subsequent dose that is at least about 1.5, 2, 2.5, 3, 3.5, 4, 4.5, 5, 5.5, 6, 6.5, 7, 7.5, 8, 8.5, 9, 9.5, 10, 15, 20, 25, 30, 35, 40, 45, 50, or 100-fold higher or lower than the current dose.

Scenario #3: Medium level of anti-TNFα drug with medium level of ADA.

Drug levels = 5-20 ng/10μl; ADA levels = 0.5-10 ng/10 μl. Patient samples having this profile include samples from patient JMM on visit 10 ("V10") and patient J-L on visit 14 ("V 14"). *See,* Figure 16b.

Patients receiving anti-TNFα drug therapy and having this particular profile should be treated with a different drug. As a non-limiting example, a patient on infliximab (IFX) therapy and having medium levels of IFX and ADA (*i.e.,* HACA) should be switched to therapy with adalimumab (HUMIRA™).

Scenario #4: Low level of anti-TNFα drug with high level of ADA.

Drug levels = 0-5 ng/10 μl; ADA levels = 3.0-50 ng/10 μl. Patient samples having this profile include samples from all patients on V 14 in Figure 16b.

Patients receiving anti-TNFα drug therapy and having this particular profile should be treated with a different drug. As a non-limiting example, a patient on infliximab (IFX) therapy and having a low level of IFX with a high level of ADA (*i.e.,* HACA) should be switched to therapy with adalimumab (HUMIRA™).

**Example 13. Measurement of Human Anti-Chimeric Antibody (HACA) in Patient Serum Samples by HPLC Mobility Shift Assay.**

This example describes a High Performance Liquid Chromatography (HPLC) procedure intended to quantify the level of Antibodies against Remicade in patient serum samples.

The principle of the HPLC mobility assay is based on the shift in retention time of the antigen-antibody immune complex verses the free antigen in size-exclusion HPLC chromatography. Standards, controls and patient samples are acid dissociated for one hour, prior to the addition of fluorescent-labeled Remicade and a fluorescent-labeled internal control, to reduce the effect of circulating Remicade. All reactions are then neutralized and incubated for one hour to allow for formation of immune complexes. Prior to being injected over a size exclusion column, all reactions are filtered and loaded onto the HPLC system with a storage temperature of 4°C. HACA bound to Remicade is separated from free Remicade by size-exclusion chromatography. The amount of HACA is determined by the ratio of the area of free labeled Remicade peak over the area of the labeled internal control peak.

Blood can be collected by venipuncture from patients. The following additional materials can be employed: Chromasolv HPLC Water; 1.2mL Micro Titer tubes; Nunc 96 Well Sample Plate; 10XPBS pH 7.4; Remicade-AlexaFluor 488/Biocytin-AlexaFluor 488; 1L Sterile Filter Systems; Multiscreen HTS, GV 96-well Filter Plates; BioSep-SEC-S 3000 Guard Column, 75 x 7.8mm; BioSep-SEC-S 3000 Analytical Column, 300 x 7.8mm; 0.05% Na Azide/HPLC Water; Detector Waste Capillary; HPLC vials; HPLC sample inserts; Multiscreen HTS Vacuum Manifold; Agilent1200 HPLC system.

An HPLC Mobile Phase (1X solution of PBS pH 7.3 ±0.1) is prepared. 200mL of 10X PBS pH 7.4 is combined with 1750 ml of HPLC water in a graduated cylinder. The pH of the resultant is determined and adjusted with IN HCl. The total volume is increased to 2000mL with HPLC water. The resultant is filtered through a 0.22µM membrane. A Phenomenex BioSep-SEC-S 3000 guard column and BioSep-SEC-S 3000 analytical column for a HPLC system are used. UV detectors are set to record at 280nm and 210nm.

Standards, controls and patient samples are prepared. Standards, controls and patient serum samples are diluted. Serum samples, standards and controls are prepared on ice in a 0.5mL welled Nunc 96 well plate. Serum sample should be added first, followed by 0.5M Citric Acid pH 3.0, and lastly HPLC water. Standards, controls and samples are incubated for one hour at room temperature on plate shaker to allow for complete dissociation of samples. The plate is covered with foil during incubation. Remicade-AlexaFluor488/Biocytin-AlexaFluor488 is added. Specified volumes of Remciade-AlexaFluor488/Biocytin-AlexaFluor488 in HPLC water are prepared. 6 µL of HPLC water is added to appropriate wells. Remciade-AlexaFluor488/Biocytin- AlexaFluor488 is added to appropriate wells.

Other organic acids may be suitable for use with this assay including, but not limited to, ascorbic acid or acetic acid.

Neutralize Samples. Specified volume of 10X PBS pH 7.4 is added to appropriate wells. Samples are mixed by pipeting up and down six times. Standards, controls and samples are incubated for one hour at room temperature on a plate shaker to allow for complete formation of immuno-complexes. Plate is covered with foil during incubation. The incubated mixture is transferred to a 4°C refrigerator if not immediately transferring to HPLC vials.

Column Standard is prepared in new sample plate with 15 µL of Column Standard and 285 µL of Mobile Phase added to a same given well. Standards, Controls and Samples are diluted to 2% Serum. The specified volume of each standard, control and sample is transferred into the appropriate wells of a new sample plate. To the same sample plate is added the column standard it was prepared in. Specified volume of 10X PBS pH 7.4 is added to appropriate wells. Specified volume of HPLC water is added to appropriate wells. Samples are mixed by petting up and down six times. Samples are filtered through a 0.2µm Multiscreen filter plate. The collection plate is added under filter plate. 295µL of sample is transferred to the respective position on filter plate. The attached filter plate is added with sample and collection plate to the vacuum manifold. Sample are filtered through into the collection plate. Standards, controls and samples are transferred into HPLC vials.

A pipet is used to transfer 250 µL of standards, controls and samples into labeled HPLC insert vials. Standards, controls and samples are loaded onto an HPLC. HPLC Parameters may include the following: Injection volume: 100 µL; Flow Rate: 1.0 mL/min of Elution Buffer A; Stop time: 20min; Post time: Off; Minimum Pressure: 0 Bar; Maximum Pressure: 400 Bar; Thermostat: Off; DAD parameters are 210nm and 280nm with 4nm and Reference Off; Peak width (Response time): >0.1min (2s); Slit: 4nm; FLD parameters Excitation: 494nm, Emission: 519nm; One injection per vial; 100µl injection volume for each sample.

## Example 14. HACA Acid Dissociation Assay.

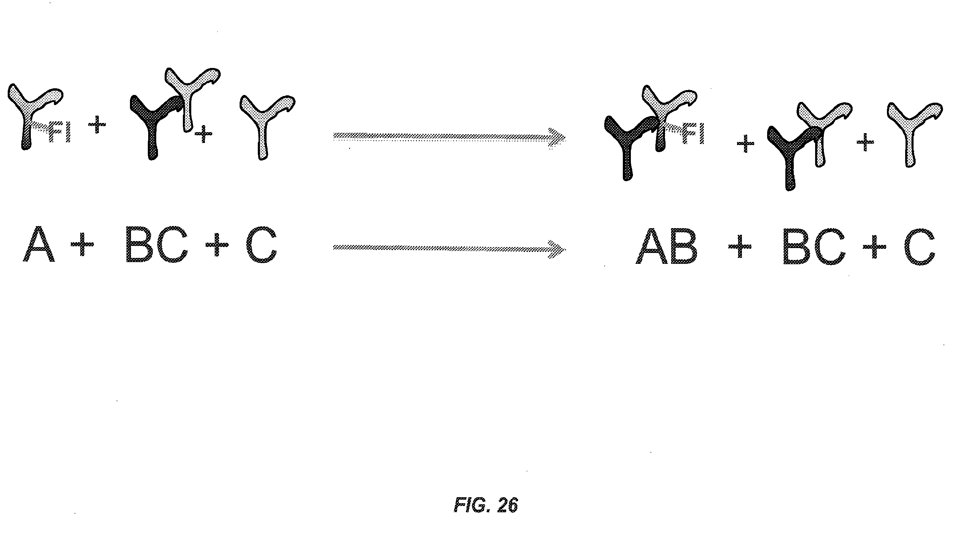

As illustrated in Figure 26, an acid dissociation step allows for the proper equilibration of the complexed species prior to measuring the concentration levels of the constituent species. High drug levels can interfere with the detection of anti-drug antibodies such as HACA. As represented in Figure 26, the acid dissociation step allows for the equilibration of the complexes of either the labeled-drug "A" or unlabeled-drug "C" with the anti-drug antibody HACA, "B." After the introduction of the acid to dissociate the BC complex, high levels of A may be added. Afterwards, the sample may be diluted and the concentration of "AB" may be measured. The concentration of "BC" after the acid dissociation step can be calculated based on the known or measured amounts of "A" and "B."

Figures 27 and 28 illustrate the percent free labeled-Infliximab as a function of Log Patient Serum percentage with and without the acid dissociation step, respectively.

The following materials can be employed in this assay: Remicade-Alexa488/Biocytin-Alexa488; Normal Human Serum; HACA Positive Control (HPC); Column Standard; 10X PBS; 1XPBS pH 7.3; Multiscreen Filter Plate; Sample Plate; IN HCl; 0.5M Citric Acid. HPC Titrations in NHS are prepared. Two fold serial dilutions are prepared by transfering 35µls of a sample into 35µl of NHS. The following solutions can be prepared for use with this example:

Solution 1: 90µl of 25% HPC/75%NHS;

Solution 2: 90µl of 12.5% HPC/87.5%NHS;

Solution 3: 90µl of 6.25%HPC/93.75%NHS.

Samples may be kept on ice before, during, and after the analysis described herein.

The following solutions are prepared:

Solution 4: A buffer solution;

Solution 5: A column standard solution;

Solution 6: 2% NHS;

Solution 7: 2% NHS + 37.5 Remicade-Alexa-488/Biocytin-Alexa488.

To these solutions are added serum samples, citric acid, HPLC water in a 96 well sample plate. Serum samples are added to respective wells. 0.5M Citric Acid pH 3.0 is added to respective wells. HPLC Water is added to respective wells.

A series of samples are prepared including the following:

Solution 8: buffer;

Solution 9: 15 μL column standard and 285 μL 1X PBS pH 7.3;

Solution 10: 2% NHS;

Solution 11: 2% NHS + 37.5 Remicade-Alexa-488/Biocytin-Alexa488;

Solution 12: 2%HPC + 0% NHS + 37.5 Remicade-Alexa-488/Biocytin-Alexa488;

Solution 13: 1%HPC + 1% NHS + 37.5 Remicade-Alexa-488/Biocytin-Alexa488;

Solution 14: 0.5%HPC + 1.5% NHS + 37.5 Remicade-Alexa-488/Biocytin-Alexa488;

Solution 15: 0.25%HPC + 1.75% NHS + 37.5 Remicade-Alexa-488/Biocytin-Alexa488;

Solution 16: 0.125%HPC + 1.875% NHS + 37.5 Remicade-Alexa-488/Biocytin-Alexa488;

Solution 17: 0.063%HPC + 1.937% NHS + 37.5 Remicade-Alexa-488/Biocytin-Alexa488;

Solution 18: 0.031%HPC + 1.969% NHS + 37.5 Remicade-Alexa-488/Biocytin-Alexa488;

Solution 19: 0.016%HPC + 1.984% NHS + 37.5 Remicade-Alexa-488/Biocytin-Alexa488;

Solution 20: 2%HPC + 0% NHS + 37.5 Remicade-Alexa-488/Biocytin-Alexa488;

Solution 21: high control;

Solution 22: medium control;

Solution 23: low control,

Solution 24: 2% NHS;

Solution 25: 2% NHS + 37.5 Remicade-Alexa-488/Biocytin-Alexa488.

All samples had 5.5 μL 0.5M pH 3 Citric Acid and 10.9 μL HPLC water added to them.

450μL of 0.074 mg/mL Remicade-Alexa488/Biocytin-Alexa488 are prepared. 6μL of HPLC water is added to three separate wells. 6μL of 0.074mg/mL Remicade-AlexaFlour488/Biocytin-AlexaFluor488 is added to remaining wells.

Neutralize samples. 27.6μL of 10XPBS pH 7.3 is added to all wells except one of the wells. Samples are mixed by pipeting up and down 6X. Samples are incubated for 1 hour at Room Temperature in the dark on plate shaker. 15μL of column standard is added the well to which the 27.6μL of 10XPBS pH 7.3 is not added. 285μL of 1XPBS pH 7.3 is added the well to which the 27.6μL of 10XPBS pH 7.3 is not added. Samples are diluted to 2% Serum.

18.4μL of each sample is transferred to corresponding wells of new sample plate. Using the same sample plate the standard was made in, 22.6μL of 10X PBS is added to all wells except the well to which the 27.6μL of 10XPBS pH

7.3 is not added. 254μL of HPLC water is added to all wells except the well to which the 27.6μL of 10XPBS pH 7.3 is not added. Samples are mixed by pipetting up and down. 295μL of standards, controls and samples are transferred to a 96 well filter plate. Using a pipet, 250μL of standards, controls and samples are transferred into HPLC insert vials.

**Example 15. Patient Case 1 of Patient Who Relapsed with Anti-TNFα Therapy.**

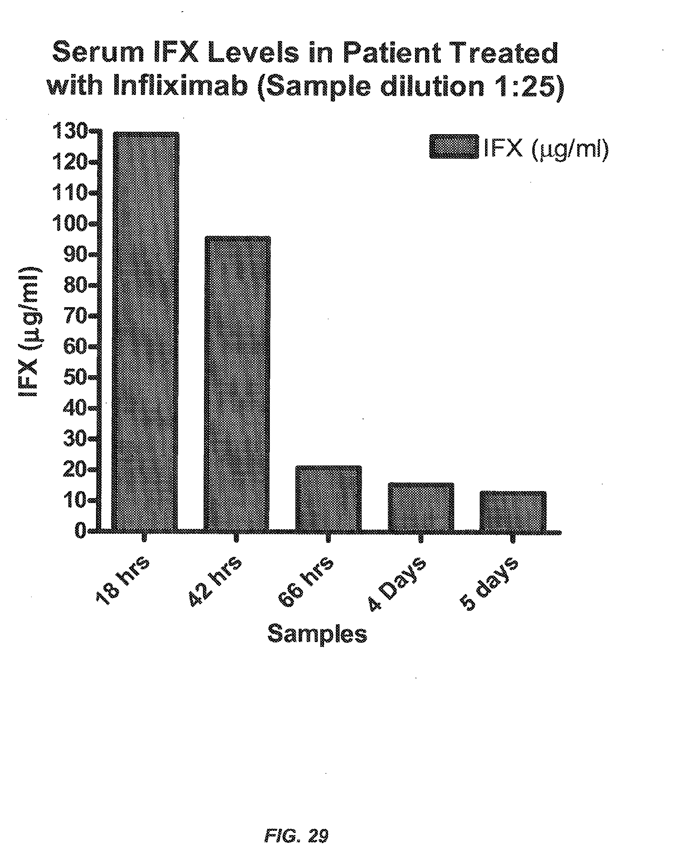

Initial testing indicated no HACA in serum and rapidly clearing IFX levels. Half life for IFX was calculated to be 46.9 hours. Dose and Frequency of IFX was increased. The patient responded. See Figure 29 for a description of the levels of IFX as a function of time.

Three months later, the patient relapsed, patient was retested and found to have low HACA and no detectable IFX. All cytokines tested were within normal range.

| Patient | HACA* (μg/mL) | IFX (μg/mL) | IFN-$\gamma$ (pg/mL) | IL-1$\beta$ (pg/mL) | IL-6 (pg/ml) | TNF-$\alpha$ (pg/mL) |
|---|---|---|---|---|---|---|
| GRD0065 | 0.34 | ND | 5.32 | 0.06 | 2.38 | 6.16 |

The suggested treatment is Azathioprine and optionally swithing to an alternative anti-TNF drug therapy. Also, continue monitoring patient to see if other anti-drug antibodies (ADA) are formed.

**Example 16. Patient Case 2 of Patient who Relapased with Anti-TNFα Therapy.**

Four months following initial testing , two samples, collected 8 days apart, were tested. HACA levels were high and IFX levels were not detectable. The recommendation is that the patient should be switched to an alternative anti-TNF therapeutic.

| Patient | Collection Date | HACA (μg/mL) | IFX (μg/mL) | IFN-$\gamma$ (pg/mL) | IL-1$\beta$ (pg/mL) | IL-6 (pg/mL) | TNF-$\alpha$ (pg/mL) |
|---|---|---|---|---|---|---|---|
| GRD0077 | Day 1 | >26 | ND | 1.57 | 0.61 | 3.38 | 0.00 |
| GRD0078 | Day 2 | >26 | ND | 1.31 | 0.24 | 2.01 | 0.00 |

**Example 17. Patient Case 3 of Patient Who Relapsed with Anti-TNFα Therapy.**

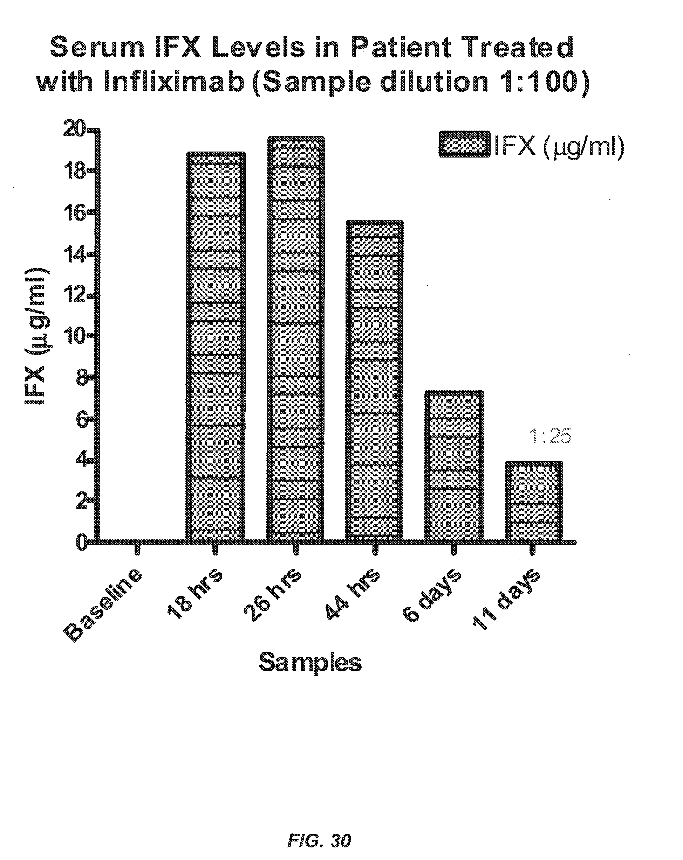

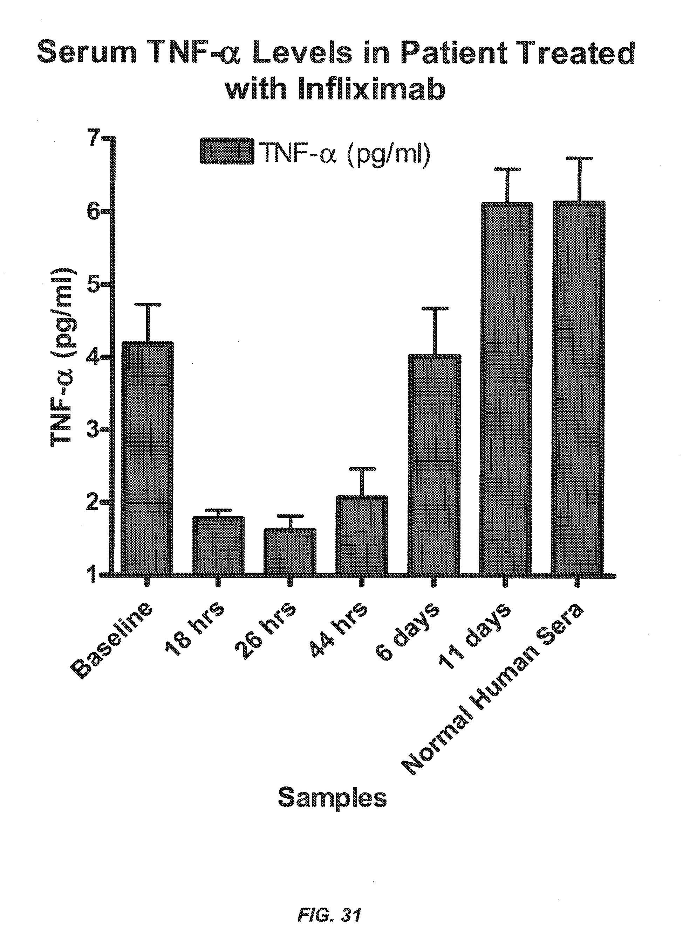

IFX concentration was calculated with a standard curve generated by reaction of different concentrations of IFX to labeled TNF-$\alpha$. Sample from 11 days was 3.8 ug/ml on 1:25 dilutions. (At least 3 half-lifes). See Figure 30 for a description of the serum levels of Infliximab as a function of time. See Figure 31 for a description of the serum levels of TNF-$\alpha$ as a function of time. The recommended treatment is to combine IFX with an immunosuppressive drug or, optionally, switch to an alternative anti-TNF drug.

**Example 18. Patient Case 4 of Patient Who Relapsed with Anti-TNFα Therapy.**

Patient was found to have high HACA and no detectable IFX. TNF-$\alpha$ levels were elevated; all other cytokines tested were within normal range. Suggested treatement is to switch to an alternative anti-TNF therapeutic.

| Patient | HACA (μg/mL) | IFX (μg/mL) | IFN-$\gamma$ (pg/mL) | IL-1$\beta$ (pg/mL) | IL-6 (pg/mL) | TNF-$\alpha$ (pg/mL) |
|---|---|---|---|---|---|---|
| GRD0009 | 21.75 | ND | 1.07 | 0.08 | 2.71 | 35.54 |

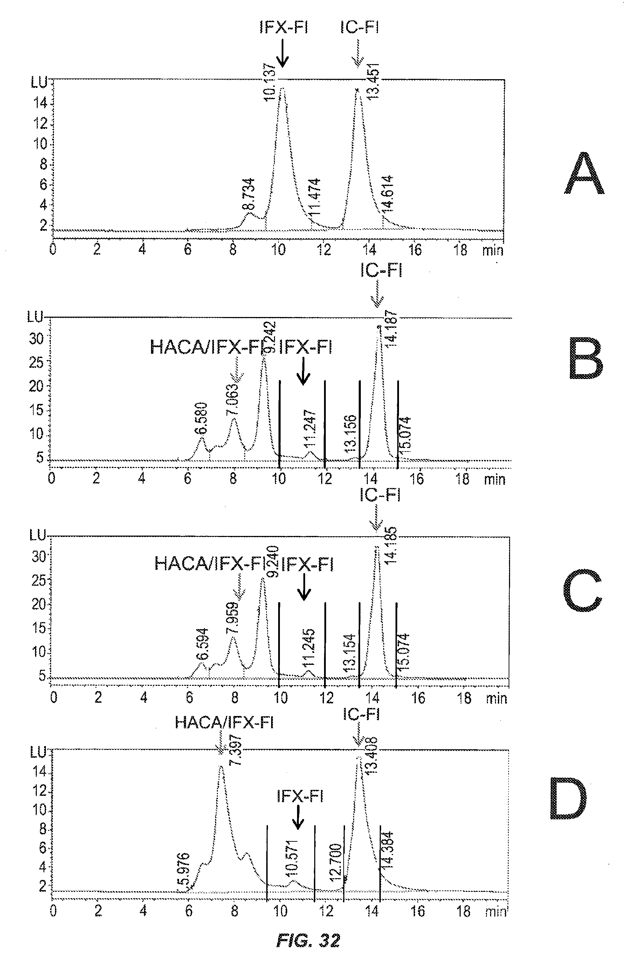

Figure 32 shows the mobility shift profiles of Fl-Labeled-IFX for Patient Case 1 (A); Patient Case 2 (B, C); and Patient Case 4 (D).

**Example 19. Patient Case 5 of Patient Who Relapsed with Anti-TNFα Therapy.**

Patient was found to have low HACA and no detectable IFX level. TNF-$\alpha$ levels were very high; all other cytokine levels tested were within normal range. Suggested therapy is to increase dose or dosing frequency of IFX or switch to an alternative anti-TNF drug along with the addition of an immunosuppressive drug. Also a suggested therapy is to continue monitoring patient to see if HACA/ADA levels increase.

| Patient | HACA (μg/mL) | IFX (μg/mL) | IFN-γ (pg/mL) | IL-1β (pg/mL) | IL-6 (pg/mL) | TNF-α (pg/mL) |
|---|---|---|---|---|---|---|
| SK12100143 | 2.80 | ND | 2.78 | 1.38 | 7.79 | **161.01** |

## Example 20. Patient Case 6 of Patient Who Relapsed with Anti-TNFα Therapy.

Patient was found to have medium HACA levels and low IFX levels. IL-1β and IL-6 levels were very high. IFN-γ was slightly elevated and TNF-α was within normal range. Suggested treatment is to switch to a different anti-TNFα drug or to therapy with a drug that targets a different mechanism (*e.g.*, an IL-6 receptor-inhibiting monoclonal antibody such as Actemra (tocilizumab)) along with the addition of an immunosuppressive drug.

| Patient | HACA (μg/mL) | IFX (μg/mL) | IFN-γ (pg/mL) | IL-1β (pg/mL) | IL-6 (pg/mL) | TNF-α (pg/mL) |
|---|---|---|---|---|---|---|
| SK07160939 | 9.42 | 11.06 | 13.31 | **366.11** | **2302.41** | 2.68 |

## Example 21. Patient Case 7 of Patient Who Relapsed with Anti-TNFα Therapy.

Patient was found to have low HACA levels. Low levels of IFX were detected. IFN-γ levels were high; all other cytokine levels tested were within normal range. Suggested treatment is to increase dose of IFX or to switch to therapy with a drug that targets a different mechanism (*e.g.*, an anti-INFγ antibody such as fontolizumab). Alternatively, suggested treatment may be to add an immunosuppressive drug.

| Patient | HACA (μg/mL) | IFX (μg/mL) | IFN-γ (pg/mL) | IL-1β (pg/mL) | IL-6 (pg/mL) | TNF-α (pg/mL) |
|---|---|---|---|---|---|---|
| SK12020346 | ND | 4.02 | **98.87** | 0.52 | 8.97 | 7.83 |

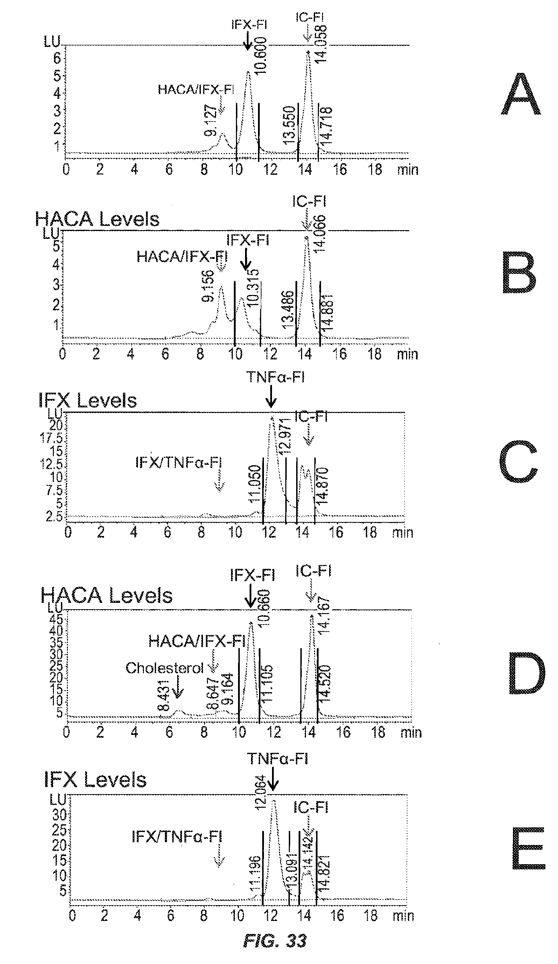

Figure 33 shows the mobility shift profiles of of Fl-Labeled-IFX for Patient Case 5 (A); Patient Case 6 (B, C); and Patient Case 7 (D, E).

## Example 22. Cytokine Levels in Different Patient Serum Groups.

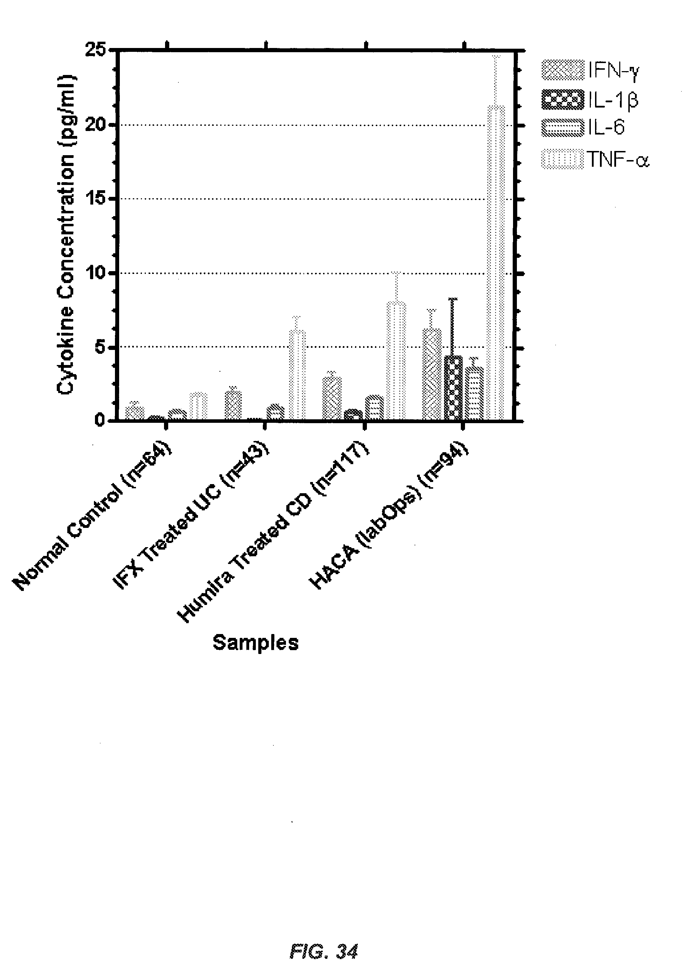

This example describes the levels of cytokines, such as, but not limited to, IFN-γ, Il-1β, IL-6, and TNFα, in normal control, infliximab treated UC, humira treated CD, and HACA positive serum samples. As illustrated in Figure 34, HACA-positive patient serum typically had higher levels of all cytokines tested (*e.g.* IFN-γ, Il-1β, IL-6, and TNFα). Based upon the presence of autoantibodies against IFX (*i.e.,* HACA) and high levels of cytokines, these patients should be switched to an alternative anti-TNF drug, optionally in combination with an immunosuppressive drug.

## Example 23: Quantification of HACA Standards by Acid Dissociation Assay.

This example describes the quantification of HACA in standard samples using the acid dissociation assay described in Example 14 with a fixed amount of Remicade™-AlexaFluor488 and varying amounts of unlabeled Remicade™. In particular, HACA concentrations ranging from 25 U/mL to 100 U/mL can be determined in the presence of unlabeled Remicade™ ranging over several orders of magnitude. Data for determination of HACA in a low-concentration standard (25 U/mL), a medium-concentration standard (50 U/mL), and a high-concentration standard (100 U/mL), are presented in Tables 8, 9, and 10, respectively. The concentration of unlabeled Remicade™ in each sample was determined using the mobility shift assay described in Example 1. Following acid dissociation and equilibration, the resulting HACA/Remicade™-AlexaFluor488 complex in a given sample was determined by SE-HPLC and total HACA was calculated according to the calculations presented in Example 7. The percent recovery of HACA in each analysis (based on the known concentration of HACA in the standard) is presented.

Table 8. Quantification of Low-Concentration HACA Standard (25 U/mL) with Varying Remicade Concentration.

| Remicade™ (µg/mL) | Mobility Shift Result | | | | | Final Concentration | | |
|---|---|---|---|---|---|---|---|---|
| | Average (U/mL) | SD | CV(%) | % Change | Recovery (%) | HACA Bound to unlabeled Remicade™ | Total HACA | Recovery (%) |
| 0 | 27.30 | 1.22 | 4.47 | NA | 109.19 | NA | 27.3 | 109.19 |
| 100 | 4.20 | 0.01 | 0.26 | -84.60 | 16.82 | 22.35 | 26.55 | 106.21 |
| 50 | 6.94 | 1.67 | 24.00 | -74.56 | 27.78 | 18.46 | 25.40 | 101.61 |
| 25 | 9.87 | 1.28 | 12.98 | -63.86 | 39.47 | 13.11 | 22.98 | 91.91 |
| 12.5 | 12.71 | 0.71 | 5.62 | -53.42 | 50.86 | 8.45 | 21.16 | 84.65 |
| 6.25 | 15.67 | 0.70 | 4.48 | -42.58 | 62.70 | 5.21 | 20.88 | 83.52 |
| 3.125 | 18.03 | 1.10 | 6.08 | -33.96 | 72.11 | 2.99 | 21.02 | 84.09 |
| 1.56 | 20.97 | 1.39 | 6.62 | -23.17 | 83.89 | 1.74 | 22.71 | 90.85 |
| 0.78 | 23.30 | 0.49 | 2.09 | -14.65 | 93.19 | 0.97 | 24.26 | 97.06 |

Table 9. Quantification of Medium-Concentration HACA Standard (50 U/mL) with Varying Remicade Concentration.

| Remicade™ (µg/mL) | Mobility Shift Result | | | | | Final Concentration | | |
|---|---|---|---|---|---|---|---|---|
| | Average (U/mL) | SD | CV(%) | % Change | Recovery (%) | HACA Bound to unlabeled Remicade™ | Total HACA | Recovery (%) |
| 0 | 54.16 | 0.80 | 1.49 | NA | 108.33 | NA | 54.16 | 108.32 |
| 100 | 7.01 | 0.80 | 11.36 | -87.06 | 14.02 | 37.25 | 44.25 | 88.51 |
| 50 | 12.22 | 0.51 | 4.14 | -77.45 | 24.43 | 32.46 | 44.68 | 89.36 |
| 25 | 19.15 | 0.19 | 1.00 | -64.65 | 38.29 | 25.44 | 44.59 | 89.17 |
| 12.5 | 25.55 | 0.81 | 3.17 | -52.83 | 51.09 | 16.97 | 42.52 | 85.04 |
| 6.25 | 31.71 | 0.33 | 1.04 | -41.46 | 63.42 | 10.53 | 42.24 | 84.49 |
| 3.125 | 38.32 | 0.46 | 1.20 | -29.25 | 76,64 | 6.38 | 44.70 | 89.40 |
| 1.56 | 42.32 | 0.02 | 0.05 | -21.87 | 84.63 | 3.51 | 45.83 | 91.65 |
| 0.78 | 49.19 | 0.85 | 1.73 | -9.19 | 98.37 | 2.04 | 51.23 | 102.45 |

Table 10. Quantification of High-Concentration HACA Standard (100 U/mL) with Varying Remicade Concentration.

| Remicade™ (µg/mL) | Mobility Shift Result | | | | | Final Concentration | | |
|---|---|---|---|---|---|---|---|---|
| | Average (U/mL) | SD | CV (%) | % Change | Recovery (%) | HACA Bound to unlabeled Remicade™ | Total HACA | Recovery (%) |
| 0 | 104.61 | 0.50 | 0.48 | NA | 104.61 | NA | 104.61 | 104.61 |
| 100 | 15.34 | 0.24 | 1.59 | -85.34 | 15.34 | 81.54 | 96.88 | 96.88 |
| 50 | 25.86 | 0.61 | 2.37 | -75.29 | 25.86 | 68.71 | 94.57 | 94.57 |
| 25 | 40.50 | 1.42 | 3.50 | -61.28 | 40.50 | 53.82 | 94.32 | 94.32 |
| 12.5 | 59.90 | 0.16 | 0.27 | -42.74 | 59.90 | 39.80 | 99.70 | 99.70 |
| 6.25 | 76.27 | 0.94 | 1.23 | -27.10 | 76,27 | 25.34 | 101.60 | 101.60 |

(continued)

| Remicade™ (µg/mL) | Mobility Shift Result | | | | | Final Concentration | | |
|---|---|---|---|---|---|---|---|---|
| | Average (U/mL) | SD | CV (%) | % Change | Recovery (%) | HACA Bound to unlabeled Remicade™ | Total HACA | Recovery (%) |
| 3.125 | 88.80 | 1.01 | 1.14 | -15.11 | 88.80 | 14.77 | 103.58 | 103.58 |
| 1.56 | 94.38 | 0.72 | 0.76 | -9.78 | 94.38 | 7.83 | 102.21 | 102.21 |
| 0.78 | 104.80 | 1.26 | 1.20 | 0.18 | 104.80 | 4.35 | 109.15 | 109.15 |

**Example 24: A New Paradigm for Anti-TNF Drug Therapy.**

The existing paradigm for anti-TNF drug therapy, based on the drug level and the HACA level determined in a patient sample, is outlined in the following Table 11:

Table 11
**Existing Paradigm**

| HACA | DRUG | Action |
|---|---|---|
| LOW | LOW | Increase Dose |
| MID | LOW | Increase Dose |
| HIGH | LOW | Switch Therapy |
| LOW | MID | Continue |
| MID | MID | Indeterminate |
| HIGH | MID | Switch Therapy |
| LOW | HIGH | Continue |
| MID | HIGH | Continue |
| HIGH | HIGH | Switch Therapy |

This paradigm is confounded, however, by the high variability in drug levels in HACA-indeterminant patients.

The therapeutic paradigm of the present method utilizes a disease activity/severity index derived from an algorithmic-based analysis of one or more biomarkers to select therapy, optimize therapy, reduce toxicity, monitor the efficacy of therapeutic treatment, or a combination thereof, with an anti-TNF drug. In certain aspects, the actions to be taken based on this new paradigm are outline for various illustrative scenarios in the following Table 12:

Table 12. Paradigm of the Present Method

| HACA | DRUG | Disease Activity Index | Action |
|---|---|---|---|
| LOW | LOW | LOW | Continue |
| LOW | LOW | MID | Increase Dose |
| LOW | LOW | HIGH | Increase Dose |
| LOW | MID | LOW | Continue |
| LOW | MID | MID | Increase Dose |
| LOW | MID | HIGH | Increase Dose |
| LOW | HIGH | LOW | Continue or Decrease Dose to avoid toxicity |
| LOW | HIGH | MID | Continue |
| LOW | HIGH | HIGH | Switch Therapy |
| MID | LOW | LOW | Continue |
| MID | LOW | MID | Increase Dose |
| MID | LOW | HIGH | Increase Dose or Change Therapy |
| MID | MID | LOW | Continue |
| MID | MID | MID | Continue |
| MID | MID | HIGH | Switch Therapy |

(continued)

| HACA | DRUG | Disease Activity Index | Action |
|------|------|------------------------|--------|
| MID | HIGH | LOW | Continue or Decrease Dose to avoid toxicity |
| MID | HIGH | MID | Continue |
| MID | HIGH | HIGH | Switch Therapy |
| HIGH | LOW | LOW | Switch Therapy |
| HIGH | LOW | MID | Switch Therapy |
| HIGH | LOW | HIGH | Switch Therapy |
| HIGH | MID | LOW | Switch Therapy |
| HIGH | MID | MOD | Switch Therapy |
| HIGH | MID | HIGH | Switch Therapy |
| HIGH | HIGH | LOW | Switch Therapy |
| HIGH | HIGH | MID | Switch Therapy. |
| HIGH | HIGH | HIGH | Switch Therapy |

It is noted that therapeutic actions for patients with mid-range HACA levels can be followed with monitoring changes in disease activity. In certain instances, high HACA levels can trigger a change in therapy despite other parameters, due to the immunological nature of the condition.

**Example 25: Detection of Low Levels of Remicade in Tissue Samples.**

Patients with Rheumatoid Arthritis (RA) have been shown to have a response to less than 100 ng/mL of Remicade during the course of treatment. A Remicade HPLC mobility shift assay has been developed as discussed herein that detects the presence of Remicade in patient serum avoiding many of the issues with an ELISA format. In certain aspects, the current lower limit of quantitation (LLOQ) for this inventive assay is about 0.49 $\mu$g/mL, allowing analysis of most patients. Our current research indicates that by adjusting various parameters of the fluorescence detector (shifting the emission wavelength to 525 nm and increasing the PMTGain to 16), the Remicade HPLC mobility shift assay can quantitatively detect as little as 50 ng/mL of Remicade in serum with high reproducibility. In fact, this level of sensitivity makes analysis of Remicade levels in small (<10mg) tissue samples possible. Detection of Remicade within tissues enhances our knowledge of the amount of Remicade that has reached the site of inflammation, yielding more information on pharmacokinetic and mechanistic details of the drug.

**Methods**

Isolation of protein from patient tissue is achieved by whole cell extraction. 1-10 mg slices of tissue are placed in a tube and then frozen in a cryo-environment. The cryogenic sample is then homogenized using the Covaris CryoPrep mechanical tissue disruptor. After pulverization, the sample is transferred to a tube containing ~300 $\mu$L extraction buffer (50 mM Tris, pH 8.0, 150 mM NaCl, 1% NP-40, 0.25% deoxycholate, 1 mM EDTA) containing a mammalian protease inhibitor cocktail (Sigma, St Louis, Mo). Samples are then immediately transferred to the acoustic portion of the CryoPrep instrument for further disruption by sonication. Samples are then incubated for 45 min on ice to allow full dissociation of cellular components. Extracts are centrifuged at 4°C for 15 min at high speed. Supernatants are aliquoted and frozen at -80°C. Protein concentrations are quantified using the Lowry protein assay (Bio-Rad). A 200 $\mu$L aliquot is thawed and then 5.0 ng of fluorescently labeled recombinant TNF-$\alpha$ (TNF-Alexa488) is added. After incubation at room temperature for 1 hour, the solution is at equilibrium and various TNF-Alexa488/Remicade complexes of increasing molecular weight have formed. After filtering, the sample is injected on a Phenomenex BioSep S-3000 HPLC size exclusion column. This real time, liquid phase assay resolves Remicade-TNF complexes from free TNF based on the size of the complexes formed.

While the current lower limit of quantitation is suitable for the majority of patients, there is a need to increase the sensitivity for use in RA patients (see above). In one aspect, the assay relies on detection of 25ng of TNF-Alexa488 in a 100uL injection on the HPLC size exclusion column. The use of fluorescence as the method of detection provides flexibility for optimization of excitation and emission wavelengths as well as the ability to increase the gain of the photomultiplier tube (PMT). The current settings used for validation of the Remicade assay are:

FLD $\lambda_{Ex}$=494, $\lambda_{Em}$=519
PMTGain = 12

These settings were chosen based on published wavelengths for the AlexaFluor 488 group as well as normal PMTGain settings for the Agilent 1200 series FLD. Increasing the PMTGain increases the signal and the noise, but up to a certain factor the increase in signal is higher than the increase in the noise. The step from gain to gain is equal to a factor of 2. The most important parameters to optimize are the excitation and emission wavelengths and while the published maximums are a useful staring point, it is often necessary to optimize them because the excitation depends on the compounds themselves as well as the specific instrument characteristics.

When detecting low amounts of Remicade, a specific peak reflecting a complex of TNF-Alexa488 and Remicade arises at a retention time of 9.2 minutes. In one aspect, it is important for the height of this peak to be at least 3 times over background and that the calculated serum concentration to over multiple replicates to have a coefficient of variance less than 20%. The signal to noise of this specific Remicade-TNFAlexa488 peak to normal human serum background can thus be the starting point for increasing the sensitivity of the assay.

To increase the sensitivity, the PMTGain as well as the excitation and emission wavelengths were optimized based on the results of amplification plots and isoabsorbance plots. Remicade was titrated in the presence of dilutions of TNF-Alexa488 at different PMTGain levels ranging from 12-18, using the current excitation and emission wavelengths of 494 and 519 nm, respectively.

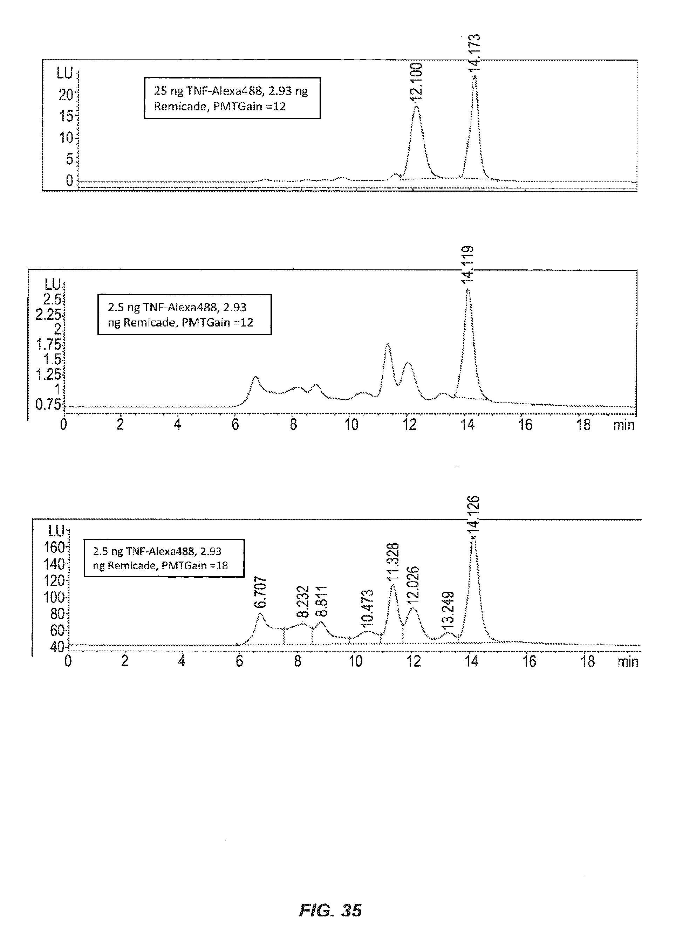

Figure 35 shows a standard amount of TNF-Alexa488 as well as the small peak at Rt=9.2 minutes reflecting a Remicade-TNF complex (top panel). Upon decreasing the amount of TNF-Alexa488 to 2.5 ng, it is clear that the background from 4% Normal Human serum begins to interfere with the resolution of the free TNF peak as well as the peak at 9.2 minutes reflecting a Remicade-TNF complex (middle panel). Increasing the PMTGain to 18 (lower panel) increases the signal and noise equally (data is similar for all PMT levels).

It is clear from the data that the background fluorescence from normal human serum interferes with quantitation of low levels of Remicade using the current settings. To increase the sensitivity of the assay, further modifications of the FLD settings are necessary to decrease the serum background signal. To investigate this, experiments were performed at different excitation and emission wavelengths based on results from isoabsorbance plots. The isoabsorbance plots were taken of normal human serum, TNF-Alexa488, mobile phase (1X PBS/0.1%BSA), and water.

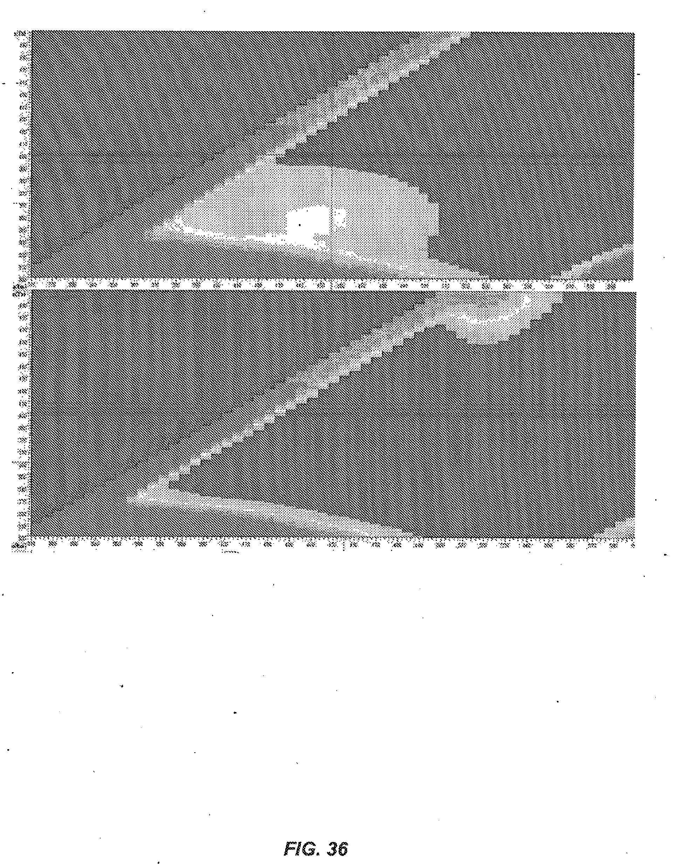

Figure 36 shows excitation wavelengths plotted on the Y-axis and emission wavelengths plotted on the X-axis. Comparing the plots for normal human serum (top panel) and TNF-Alexa488 (bottom panel) shows significant overlap in both excitation and emission maximums (vertex of the v-shaped region in the plots). Shifting the emission wavelength to at least 525nm will likely maintain high sensitivity for TNF-Alexa488 while decreasing the normal serum background. The emission wavelength was set to 525 nm and then experiments repeated looking at TNF-Alexa488 as well as normal human serum background. TNF-Alexa488 was injected in the presence of 4% NHS and the signal-to-noise evaluated.

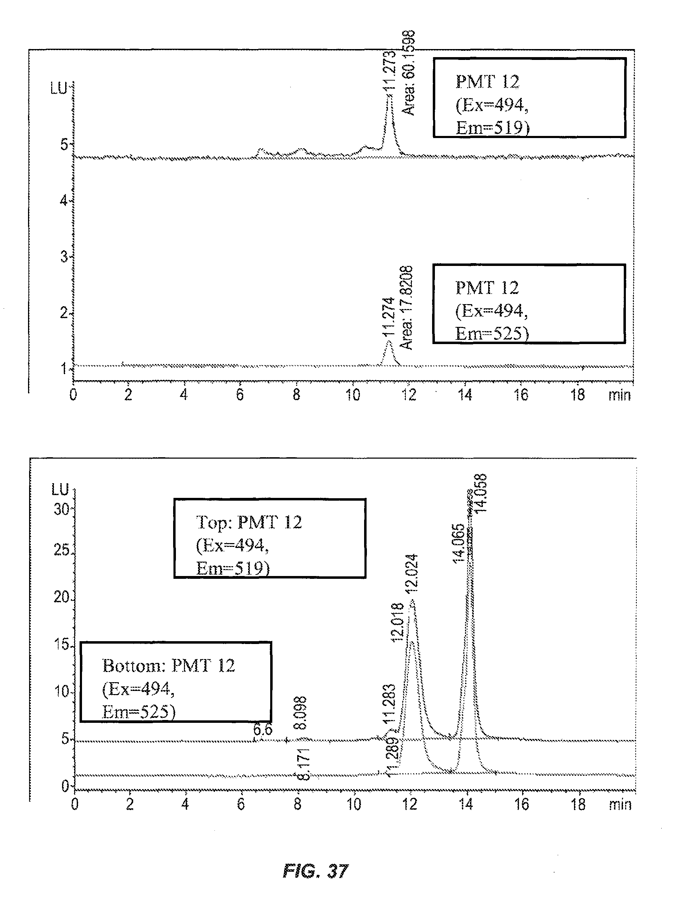

Figure 37 shows the analysis of normal human serum (left panel) and 25ng TNF-Alexa488 (right panel) by HPLC using the indicated settings. The background level of fluorescence from normal human serum is greatly decreased. After demonstrating the level of background fluorescence from serum was decreased, the signal to noise of the assay was evaluated at several different PMTGain levels ranging from 12-18. The results of the analysis, presented in the Table 13 below, establish that a PMTGain of 16 provides significant benefit.

Table 13

| PMT | Emission | Average Area NHS Background (n=2) | Average Area TNF-Alexa488 Peak (n=2) | Signal/Noise |
|---|---|---|---|---|
| 12 | 519 | 45.95 | 544 | 11.84 |
| 12 | 525 | 17.4 | 481.5 | 27.67 |
| 16 | 519 | 1053 | 8747 | 8.31 |
| 16 | 525 | 210.5 | 8019.5 | 38.10 |

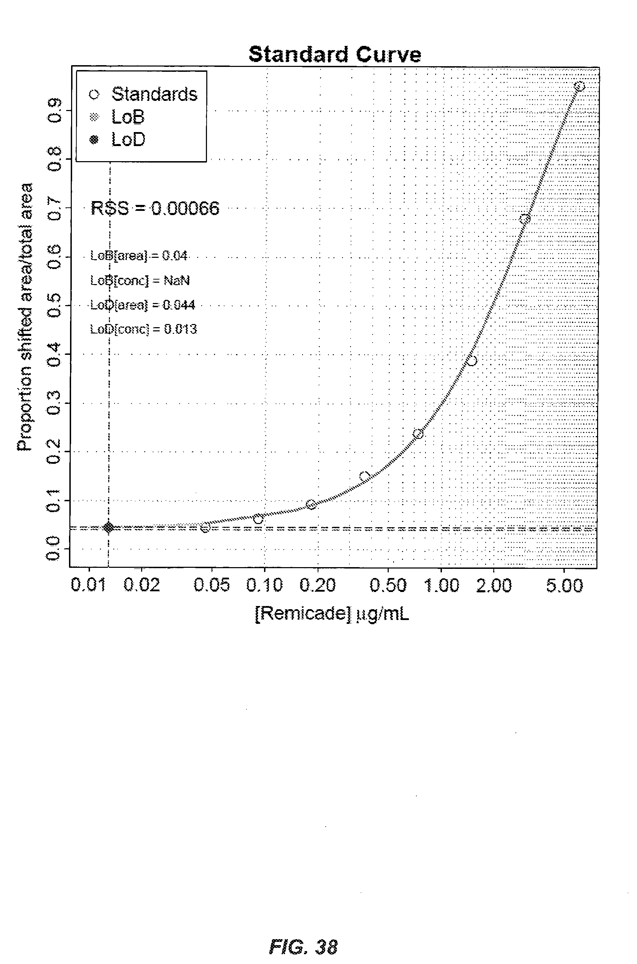

The sensitivity of the assay was then probed by generating standard curves such as the plot shown in Figure 38. 2.5 ng TNF-Alexa488 per injection was used Remicade was titrated in the range of 50 ng/mL-5.86 $\mu$g/mL to establish the limit of detection. The peak at retention time of 9.2 was again monitored as a judge of signal-to-noise and the lowest concentration that repeatedly (n=20) gave rise to a 3:1 peak height was used to calculate the LOQ. The results of this kind of analysis are presented in the following table.

Table 14

| Experimental Settings: PMTGain = 16 $\lambda_{Ex}$ = 494 nm, $\lambda_{Em}$ = 525 nm 2.5 ng Alexa488/100 $\mu$L Injection | |
|---|---|
| LOB (area) | 0.040 |

(continued)

| Experimental Settings: PMTGain = 16 $\lambda_{Ex}$ = 494 nm, $\lambda_{Em}$ = 525 nm 2.5 ng Alexa488/100 µL Injection | |
| --- | --- |
| LOD (area) | 0.044 |
| LOD (n=20) | 13.00 ng/mL |
| LLOQ (n=20) | 51.02 ng/mL |
| | CV% = 21.07<br>Accuracy = 111.40% |

By shifting the Emission wavelength to 525 nm and increasing the PMT gain to 16, the Remicade HPLC mobility shift assay can now quantitatively detect as little as 50 ng/mL of Remicade in serum with high reproducibility. Further optimization may increase the sensitivity to a greater extent, but the new format should allow analysis of RA patients that show response even at very low Remicade serum concentrations. Correlation of low Remicade levels with patient response, clinical outcome, and related biomarkers make decisions for a more personalized approach to treatment.

**Example 26: Clinical study analysis of Mobility Shift Assay vs. ELISA.**

Initial studies were performed as above using samples from active CD patients (N = 117) and UC patients (N = 10) treated with infliximab over several weeks. Mobility shift assay data were compared with ELISA results.

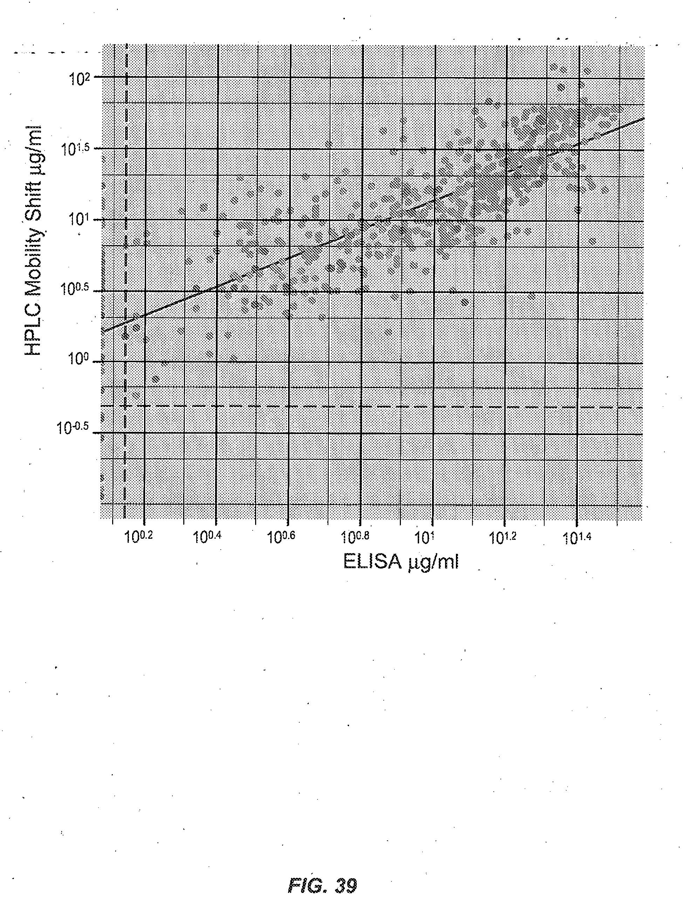

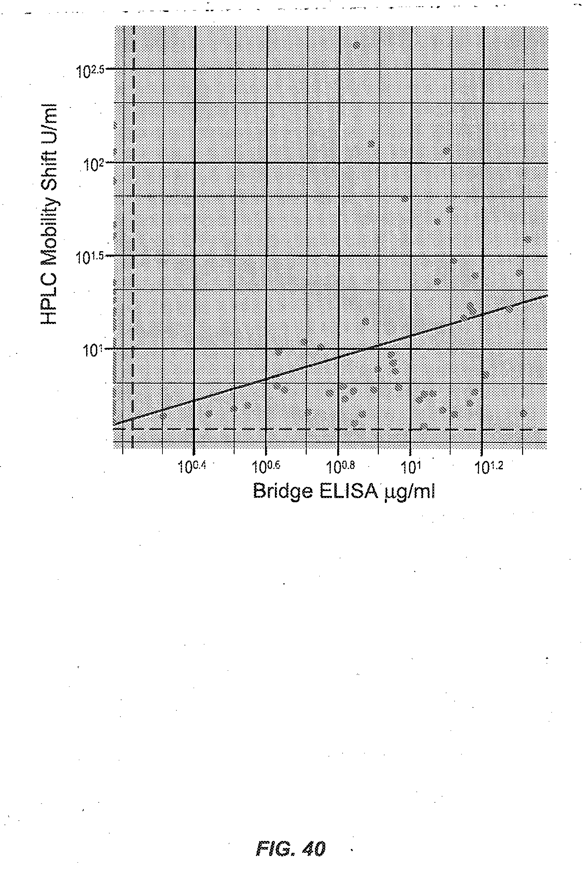

As shown in Figure 39, both methods correlated (correlation coefficient = 0.812, p < 2.2 x $10^{-16}$ for data collected above the lower limits of quantitation) for determination of infliximab in the samples. 6% of samples determined to be infliximab-negative by ELISA were shown to be infliximab-positive by the mobility shift assay. None of the samples determined to be infliximab-negative by the mobility shift assay were determined to be infliximab-positive by ELISA. As determined by mobility shift assay, four infliximab-negative samples were found to be HACA-positive. ELISA and mobility shift assay data were also correlated for determination of HACA, as shown in Figure 40. 37 of the samples determined as HACA-negative by ELISA were found to be HACA-positive by the mobility shift assay.

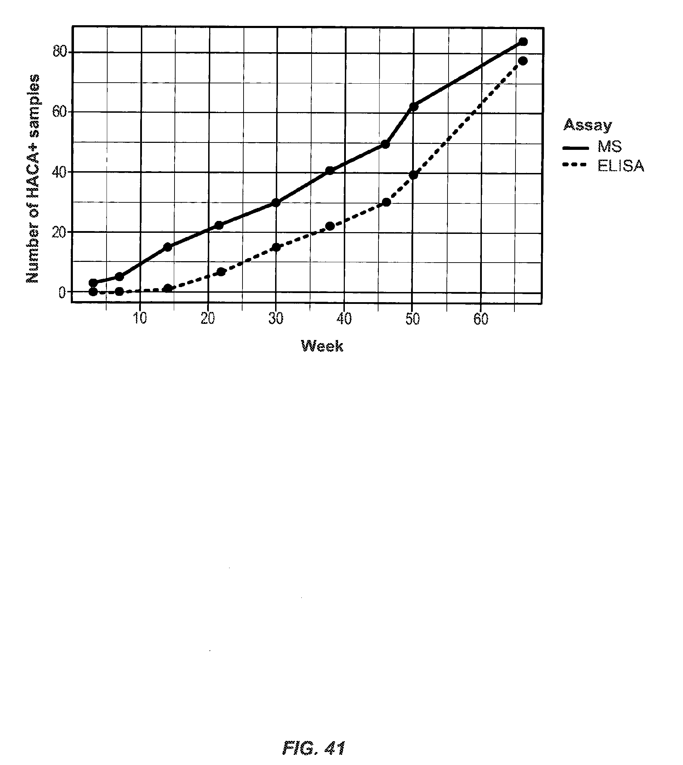

Cumulative counts per week of HACA-positive samples were tabulated over time as shown in Figure 41. While the data for the mobility shift assay (Figure 41, top trace) and ELISA (Figure 41, bottom trace) begin to converge after 60 weeks, the mobility shift assay resulted in higher count of HACA-positive specimens at earlier time points. Fisher's exact test was applied to the data collected at various time points. The p-values as determined by the test were 0.0381, 0.0240, and 0.6791 at 46 weeks, 50 weeks, and 66 weeks, respectively. Taken together, the clinical studies indicate that the mobility shift assay overcomes variability and interference limitations in the ELISA. The technology is also applicable to a broad spectrum of protein therapeutics for conditions such as rheumatoid arthritis and inflammatory bowel disease. Given the critical need for precise detection of drug levels and anti-drug antibodies in developing therapeutic strategies, the mobility shift assay allows for better management of patient treatment.

**Example 27: Evaluation of A Novel Homogeneous Mobility Shift Assay For The Measurement of Human Anti-Chimeric Antibodies (HACA) and Infliximab (IFX) Levels in Patient Serum.**

**Background:** The list of antibody-based biotherapeutics available for the treatment of inflammatory diseases such as inflammatory bowel disease (IBD) and rheumatoid arthritis (RA) is steadily increasing. However, certain patients will generate anti-drug antibodies (ADA) that can cause a range of consequences, including alteration of the drug pharmacokinetics, reduction/loss of drug efficacy, and adverse drug reactions. Monitoring of patients for antibody drug and ADA levels is not only required by the FDA during the drug development process, but is also very important for appropriate patient management during treatment with these drugs. Different methods are available for the assessment of ADA and drug levels, which include solid phase immunoassay, radioimmunoprecipitation (RIPA) and Surface Plasmon Resonance (SPR). However, many disadvantages are observed in these methods, including masked/altered epitopes by antigen immobilization or labeling, inability to define species specificity and isotype detection, failure to detect low affinity antibodies, requirement for dedicated instruments or radiolabeled reagent, and low drug tolerance in the sample. We have developed a non-radio labeled liquid-phase homogeneous mobility shift assay to measure the HACA and drug levels in serum from patients treated with IFX. This method overcomes many of the limitations of the current methods for measuring HACA and drug level.

**Methods:** To perform the mobility shift HACA assay, Alexa Fluor 488 (Alexa488) labeled Infliximab (IFX) containing an Alexa488 loading control is incubated with HACA positive serum and allowed to reach equilibrium. After equilibration, the reaction mixture is then injected onto a HPLC column. The free Alexa488-IFX and immune complexes are resolved by size exclusion chromatography (SEC) HPLC and the intensity of the fluorescence in each resolved peak is measured by a fluorescent detector (FLD). The changes in the ratio of the free Alexa488 IFX peak area to the Alexa488 internal control peak area indicate the amount of the immune complexes formed. Different dilutions of HACA positive serum are used to generate a standard curve, which is fitted with a 5-parameter logistic model to account for asymmetry. The amount of HACA in the samples is calculated from the standard curve. Similar methodology and analysis are used to measure the IFX level in the serum, except that Alexa488 labeled TNF-$\alpha$ is utilized to bind IFX and purified IFX is used as the standard. We have performed a full method validation on both HACA and IFX assays, and compared the clinical sample test results with those obtained from ELISA methods.

**Results:** Validation of the mobility shift HACA assay revealed a lower limit of quantitation of 6.75U/ml in serum samples, which is equivalent to 35.4ng/ml, and this value is lower than the industry requirement (250-500 ng/ml). The linear range of quantitation is 6.75-150 U/ml. The intra-assay and inter-assay precision determination yielded a coefficient of variation of less than 15%, and the accuracy of the assay is within 20%. IFX drug tolerance in the assay is up to 100 $\mu$g/ml in the test serum. Therapeutic levels of azathioprine (AZA) and methotrexate (MTX), presence of rheumatoid factor (774 IU/ml), normal levels of immunoglobulins, TNFs and soluble TNF receptors have no significant interference in the assay. Serum samples from 100 drug naive healthy subjects were tested to set up the cutoff point of 6.75U/ml (Mean+1.65SD). One hundred HACA positive serum samples analyzed by bridge ELISA were also evaluated by the mobility shift assay. Overall, there is a strong correlation between the two methods on HACA levels (Spearman's Rho = 0.337, p = 0.0196). However, the new method was able to identify 23 false positive samples from the bridge ELISA. Similar results were obtained from the validation of the mobility shift IFX assay.

**Conclusions:** Results from this study demonstrated the superiority of the mobility shift assay in measuring HACA and IFX in patient serum samples. This method can also be applied to detect other biopharmaceuticals and ADA in patient serum samples such as those treated with adalimumab.

**Claims**

1.  A method for detecting the presence or level of an autoantibody to an anti-TNFalpha drug in a sample without interference from the anti-TNFalpha drug in the sample, the method comprising:

    (a) contacting the sample with an acid to dissociate preformed complexes of the autoantibody and the anti-TNFalpha drug, wherein the sample has or is suspected of having an autoantibody to the anti-TNFalpha drug;
    (b) contacting the sample with a labeled anti-TNFalpha drug following dissociation of the preformed complexes, wherein optionally step (b) further comprises contacting a labeled internal control with the sample;
    (c) neutralizing the acid in the sample to form labeled complexes of the labeled anti-TNFalpha drug and the autoantibody;
    (d) subjecting the labeled complexes to size exclusion chromatography to separate the labeled complexes; and
    (e) detecting the labeled complexes, thereby detecting the presence or level of the autoantibody without interference from the anti-TNFalpha drug in the sample.

2.  The method of claim 2, wherein the sample is contacted with an acid at a concentration of from 0.1 M to 5M.

3.  The method of any one of claims 1 to 2, wherein the acid is neutralized by adding one or more neutralizing agents to the sample.

4.  The method of any one of claims 1 to 3, wherein the sample is obtained from a subject receiving therapy with the anti-TNFalpha drug.

5.  The method of any one of claims 1 to 4, wherein the complexes are eluted first, followed by free labeled anti-TNFalpha drug.

6.  The method of any one of claims 1 to 5, wherein the anti-TNFalpha drug is labeled with a fluorophore or a fluorescent dye.

7.  A method for optimizing therapy and/or reducing toxicity to an anti- TNFalpha drug in a subject receiving a course of therapy with the anti-TNFalpha drug, the method comprising:

    (a) detecting the presence or level of an autoantibody to the anti-TNFalpha drug in a sample from the subject without interference from the anti-TNFalpha drug in the sample, the method comprising:

(i) contacting the sample with an acid to dissociate preformed complexes of the autoantibody and the anti-TNFalpha drug, wherein the sample has or is suspected of having an autoantibody to the anti-TNFalpha drug;
(ii) contacting the sample with a labeled anti-TNFalpha drug following dissociation of the preformed complexes;
(iii) neutralizing the acid in the sample to form labeled complexes of the labeled anti-TNFalpha drug and the autoantibody; (iv) subjecting the labeled complexes to size exclusion chromatography to separate the labeled complexes; and
(v) detecting the labeled complexes to thereby detect the presence or level of the autoantibody without interference from the anti-TNFalpha drug in the sample; and

(b) determining a subsequent dose of the course of therapy for the subject or
whether a different course of therapy should be administered to the subject based upon the presence or level of the autoantibody,
thereby optimizing therapy and/or reducing toxicity to the anti-TNFalpha drug.

8. The method of claim 7, wherein the subsequent dose of the course of therapy is increased, decreased, or maintained based upon the presence or level of the autoantibody.

9. The method of claims 7 or 8, wherein the different course of therapy comprises a different anti-TNFalpha drug, or wherein the different course of therapy comprises the current course of therapy along with an immunosuppressive agent, or wherein the different course of therapy comprises switching to a course of therapy that is not an anti-TNFalpha drug.

10. The method of any of claims 1 to 9, wherein the anti-TNFalpha drug is selected from the group consisting of infliximab, etanercept, adalimumab, certolizumab pegol, golimumab, CNTO 148, and combinations thereof.

11. The method of any of claims 1 to 10, wherein the autoantibody to the anti- TNFalpha drug is selected from the group consisting of a human anti-chimeric antibody (HACA), a human anti-humanized antibody (HAHA), a human anti-mouse antibody (HAMA), and combinations thereof.

12. The method of any one of claims 1 to 11, wherein the acid comprises an organic acid, an inorganic acid, or mixtures thereof, wherein optionally the organic acid comprises citric acid.

13. The method of any one of claims 1 to 12, wherein the presence or level of the autoantibody is detected in the presence of a high level of the anti-TNFalpha drug, wherein optionally the high level of the anti-TNFalpha drug corresponds to an anti-TNFalpha drug level of from 10 to 100 $\mu$g/ml.

14. The method of any one of claims 1 to 13, wherein the size exclusion chromatography is size exclusion-high performance liquid chromatography (SE-HPLC).

15. The method of any one of claims 1 to 14, wherein the sample is serum.

**Patentansprüche**

1. Verfahren für den Nachweis des Vorhandenseins oder des Wertes eines Autoantikörpers gegen ein Anti-TNF-alpha-Medikament in der Probe, wobei das Verfahren folgendes aufweist:

(a) Kontaktieren der Probe mit einer Säure, um vorgeformte Komplexe des Autoantikörpers und des Anti-TNF-alpha-Medikaments zu trennen, wobei die Probe einen Autoantikörper gegen das Anti-TNF-alpha-Medikament besitzt oder vermutet wird, dass sie diesen besitzt;
(b) Kontaktieren der Probe mit einem markierten Anti-TNF-alpha-Medikament nach der Trennung der vorgeformten Komplexe, wobei Schritt (b) wahlweise weiterhin das Kontaktieren einer markierten internen Kontrolle mit der Probe aufweist;
(c) Neutralisieren der Säure in der Probe, um markierte Komplexe von dem markierten Anti-TNF-alpha-Medikament und dem Autoantikörper zu bilden;
(d) Unterziehen der markierten Komplexe einer Größenausschlusschromatographie (SEC), um die markierten Komplexe zu trennen;

und

(e) Nachweisen der markierten Komplexe und dadurch Nachweisen des Vorhandenseins oder Wertes des Autoantikörpers ohne Interferenz des Anti-TNF-alpha-Medikaments in der Probe.

2.  Verfahren nach Anspruch 1, wobei die Probe bei einer Konzentration von 0,1M bis 5M mit einer Säure kontaktiert wird.

3.  Verfahren nach einem der Ansprüche 1 bis 2, wobei die Säure durch Hinzufügen eines oder mehrerer Neutralisationsmittel neutralisiert wird.

4.  Verfahren nach einem der Ansprüche 1 bis 3, wobei man die Probe von einer Testperson bekommt, die eine Therapie mit dem Anti-TNF-alpha-Medikament erhält.

5.  Verfahren nach einem der Ansprüche 1 bis 4, wobei die Komplexe zunächst eluiert werden, gefolgt von dem frei markierten Anti-TNF-alpha-Medikament.

6.  Verfahren nach einem der Ansprüche 1 bis 5, wobei das Anti-TNF-alpha-Medikament mit einem fluorophoren Farbstoff oder einem Fluoreszenzfarbstoff markiert ist.

7.  Verfahren zum Optimieren der Therapie und/oder Reduzieren der Toxizität gegenüber einem Anti-TNF-alpha-Medikament bei einer Testperson, die eine Therapie mit dem Anti-TNF-alpha-Medikament erhält, wobei das Verfahren folgendes aufweist:

    (a) Nachweisen des Vorhandenseins oder Wertes eines Autoantikörpers gegen das Anti-TNF-alpha-Medikament in einer Probe von der Testperson ohne Interferenz des Anti-TNF-alpha-Medikaments in der Probe, wobei das Verfahren folgendes aufweist:

    (i) Kontaktieren der Probe mit einer Säure, um vorgeformte Komplexe des Autoantikörpers und des Anti-TNF-alpha-Medikaments zu trennen, wobei die Probe einen Autoantikörper gegen das Anti-TNF-alpha-Medikament besitzt oder vermutet wird, dass sie diesen besitzt;

    (ii) Kontaktieren der Probe mit einem markierten Anti-TNF-alpha-Medikament nach der Trennung der vorgeformten Komplexe;

    (iii) Neutralisieren der Säure in der Probe, um markierte Komplexe von dem markierten Anti-TNF-alpha-Medikament und dem Autoantikörper zu bilden;

    (iv) Unterziehen der markierten Komplexe einer Größenausschlusschromatographie (SEC), um die markierten Komplexe zu trennen; und

    (v) Nachweisen der markierten Komplexe und dadurch Nachweisen des Vorhandenseins oder Wertes des Autoantikörpers ohne Interferenz des Anti-TNF-alpha-Medikaments in der Probe; und

    (b) Festlegen einer anschließenden Dosis für die Therapie der Testperson oder ob der Testperson aufgrund des Vorhandenseins oder des Wertes des Autoantikörpers eine andere Therapie verabreicht werden sollte, dadurch Optimieren der Therapie und/oder Reduzieren der Toxizität gegenüber dem Anti-TNF-alpha-Medikament.

8.  Verfahren nach Anspruch 7, wobei die anschließende Dosis der Therapie aufgrund des Vorhandenseins oder des Wertes des Autoantikörpers erhöht, gesenkt oder aufrechterhalten wird.

9.  Verfahren nach Anspruch 7 oder 8, wobei die andere Therapie ein anderes Anti-TNF-alpha-Medikament umfasst, oder wobei die andere Therapie die aktuelle Therapie, zusammen mit einem immunsuppressiven Medikament umfasst, oder wobei die andere Therapie den Wechsel zu einer Therapie umfasst, bei der es sich nicht um ein Anti-TNF-alpha-Medikament handelt.

10.  Verfahren nach einem der Ansprüche 1 bis 9, wobei das Anti-TNF-alpha-Medikament aus der Gruppe ausgewählt ist, die folgendes aufweist: Infliximab, Etanercept, Adalimumab, Certolizumab pegol, Golimumab, CNTO 148 und Kombinationen daraus.

11.  Verfahren nach einem der Ansprüche 1 bis 10, wobei der Autoantikörper gegen das Anti-TNF-alpha-Medikament aus der Gruppe ausgewählt ist, die folgendes aufweist: einen humanen antichimären Antikörper (HACA), einen humanen Anti-Humanen-Antikörper (HAHA), einen humanen Anti-Maus-Antikörper (HAMA) und Kombinationen daraus.

**12.** Verfahren nach einem der Ansprüche 1 bis 11, wobei die Säure eine organische Säure, eine anorganische Säure oder Mischungen daraus aufweist, wobei die organische Säure wahlweise Zitronensäure aufweist.

**13.** Verfahren nach einem der Ansprüche 1 bis 12, wobei das Vorhandensein oder der Wert des Autoantikörpers in der Gegenwart eines hohen Wertes des Anti-TNF-alpha-Medikaments nachgewiesen ist, wobei der hohe Wert des Anti-TNF-alpha-Medikaments wahlweise einem Wert eines Anti-TNF-alpha-Medikaments von 10 bis 100 μg/ml entspricht.

**14.** Verfahren nach einem der Ansprüche 1 bis 13, wobei die Größenausschlusschromatographie eine Größenausschluss-Hochleistungsflüssigkeitschromatographie (SE-HPLC) ist.

**15.** Verfahren nach einem der Ansprüche 1 bis 14, bei dem die Probe ein Serum ist.

## Revendications

**1.** Procédé destiné à détecter la présence ou le niveau d'un auto-anticorps contre un médicament anti-TNFalpha dans un échantillon sans interférence provenant du médicament anti-TNFalpha dans l'échantillon, le procédé comprenant :

(a) une mise en contact de l'échantillon avec un acide afin de dissocier des complexes préformés de l'auto-anticorps et du médicament anti-TNFalpha, dans lequel l'échantillon a ou est suspecté d'avoir un auto-anticorps contre le médicament anti-TNFalpha ;
(b) une mise en contact de l'échantillon avec un médicament anti-TNFalpha marqué après la dissociation des complexes préformés, dans lequel facultativement l'étape (b) comprend en outre une mise en contact d'un contrôle interne marqué avec l'échantillon ;
(c) une neutralisation de l'acide dans l'échantillon afin de former des complexes marqués du médicament anti-TNFalpha marqué et de l'auto-anticorps ;
(d) une soumission des complexes marqués à une chromatographie d'exclusion diffusion afin de séparer les complexes marqués ; et
(e) une détection des complexes marqués, et ainsi une détection de la présence ou du niveau de l'auto-anticorps sans interférence provenant du médicament anti-TNFalpha dans l'échantillon.

**2.** Procédé selon la revendication 1, dans lequel l'échantillon est mis en contact avec un acide à une concentration de 0,1 M à 5M.

**3.** Procédé selon l'une quelconque des revendications 1 à 2, dans lequel l'acide est neutralisé par un ajout d'un ou plusieurs agents de neutralisation à l'échantillon.

**4.** Procédé selon l'une quelconque des revendications 1 à 3, dans lequel l'échantillon est obtenu à partir d'un sujet recevant une thérapie avec le médicament anti-TNFalpha.

**5.** Procédé selon l'une quelconque des revendications 1 à 4, dans lequel les complexes sont élués en premier, suivis de médicament anti-TNFalpha non marqué.

**6.** Procédé selon l'une quelconque des revendications 1 à 5, dans lequel le médicament anti-TNFalpha est marqué avec un fluorophore ou un colorant fluorescent.

**7.** Procédé destiné à optimiser une thérapie et/ou à réduire la toxicité par rapport à un médicament anti-TNFalpha chez un sujet recevant un plan de thérapie avec le médicament anti-TNFalpha, le procédé comprenant :

(a) une détection de la présence ou du niveau d'un auto-anticorps contre le médicament anti-TNFalpha dans un échantillon provenant du sujet sans interférence provenant du médicament anti-TNFalpha dans l'échantillon, le procédé comprenant :

(i) une mise en contact de l'échantillon avec un acide afin de dissocier des complexes préformés de l'auto-

EP 2 676 137 B1

anticorps et du médicament anti-TNFalpha, dans lequel l'échantillon a ou est suspecté d'avoir un auto-anticorps contre le médicament anti-TNFalpha ;

(ii) une mise en contact de l'échantillon avec un médicament anti-TNFalpha marqué après la dissociation des complexes préformés ;

(iii) une neutralisation de l'acide dans l'échantillon afin de former des complexes marqués du médicament anti-TNFalpha marqué et de l'auto-anticorps ;

(iv) une soumission des complexes marqués à une chromatographie d'exclusion diffusion afin de séparer les complexes marqués ; et

(v) une détection des complexes marqués, et ainsi une détection de la présence ou du niveau de l'auto-anticorps sans interférence provenant du médicament anti-TNFalpha dans l'échantillon ; et

(b) une détermination d'une dose ultérieure du plan de thérapie pour le sujet ou
si un plan de thérapie différent doit être administré au sujet en se basant sur la présence ou le niveau de l'auto-anticorps,
et ainsi une optimisation de la thérapie et/ou une réduction de la toxicité par rapport au médicament anti-TNFalpha.

8. Procédé selon la revendication 7, dans lequel la dose ultérieure du plan de thérapie est augmentée, diminuée ou maintenue en se basant sur la présence ou le niveau de l'auto-anticorps.

9. Procédé selon la revendication 7 ou 8, dans lequel le plan de thérapie différent comprend un médicament anti-TNFalpha différent, ou dans lequel le plan de thérapie différent comprend le plan de thérapie actuel ainsi qu'un agent immunosuppresseur, ou dans lequel le plan de thérapie différent comprend le passage à un plan de thérapie qui n'est pas un médicament anti-TNFalpha.

10. Procédé selon l'une quelconque des revendications 1 à 9, dans lequel le médicament anti-TNFalpha est choisi parmi le groupe constitué par infliximab, étanercept, adalimumab, certolizumab pégol, golimumab, CNTO 148, et des combinaisons de ceux-ci.

11. Procédé selon l'une quelconque des revendications 1 à 10, dans lequel l'auto-anticorps contre le médicament anti-TNFalpha est choisi parmi le groupe constitué par un anticorps humain anti-chimère (HACA), un anticorps humain anti-humain (HA-HA), un anticorps humain anti-murin (HAMA), et des combinaisons de ceux-ci.

12. Procédé selon l'une quelconque des revendications 1 à 11, dans lequel l'acide comprend un acide organique, un acide inorganique, ou des mélanges de ceux-ci, dans lequel facultativement l'acide organique comprend de l'acide citrique.

13. Procédé selon l'une quelconque des revendications 1 à 12, dans lequel la présence ou le niveau de l'auto-anticorps est détecté en présence d'un niveau élevé du médicament anti-TNFa, et dans lequel facultativement le haut niveau du médicament anti-TNFalpha correspond à un niveau de médicament anti-TNFa de 10 à 100 $\mu$g/ml.

14. Procédé selon l'une quelconque des revendications 1 à 13, dans lequel la chromatographie d'exclusion diffusion est une chromatographie en phase liquide à haute performance d'exclusion diffusion (SE-HPLC).

15. Procédé selon l'une quelconque des revendications 1 à 14, dans lequel l'échantillon est du sérum.

*FIG. 1*

A

Dose Response Curve of Humira Binding
to TNF-Alexa647

B

Dose Response Curve of Humira Binding to
TNF-Alexa647 in 1% Normal Human Serum

C

Dose Response Curve of Humira Binding to
TNF-Alexa647 in 1% Pooled RA+ Patient Serum

|  | mAU's |
|---|---|
| HILLSLOPE | 2.180 |
| EC50 | 117.4 |

|  | mAU's |
|---|---|
| HILLSLOPE | 1.454 |
| EC50 | 81.58 |

|  | mAU's |
|---|---|
| HILLSLOPE | 0.8481 |
| EC50 | 218.3 |

*FIG. 2*

EP 2 676 137 B1

FIG. 3

(1) Preparation of Alexa[647]-labeled Remicade

(2) Incubation with patient serum (HACA) to form the complex

(3) Quantification of the complex by SE-HPLC

(4) Assay works in the presence of remicade

*FIG. 4*

**(A)** 1X PBS, pH 7.4 (Blank Elution)

**(B)** 125ng Goat Anti-human IgG

**(C)** 6.25ng Remicade-Alexa[647]

**(D)** 1.9ng Goat Anti-human IgG + 6.25ng Remicade-Alexa[647]

**(E)** 3.9ng Goat Anti-human IgG + 6.25ng Remicade-Alexa[647]

**(F)** 7.8ng Goat Anti-human IgG + 6.25ng Remicade-Alexa[647]

**(G)** 15.6ng Goat Anti-human IgG + 6.25ng Remicade-Alexa[647]

**(H)** 31.2ng Goat Anti-human IgG + 6.25ng Remicade-Alexa[647]

**(I)** 62.5ng Goat Anti-human IgG + 6.25ng Remicade-Alexa[647]

**(J)** 125ng Goat Anti-human IgG + 6.25ng Remicade-Alexa[647]

*FIG. 5*

*FIG. 6*

A.

**Dose-response Curve of Remaining Remicade-Alexa647 Affter Incubation with Different Concentration of Anti-human IgG Antibody**

|  | Standard |
|---|---|
| HILLSLOPE | -1.718 |
| EC50 | 3.359 |

B.

**Dose-response of Remicade-Alexa647 Complex in the Presence on Different Concentration of Anti-Human IgG**

|  | Standard |
|---|---|
| HILLSLOPE | 0.9035 |
| EC50 | 18.97 |

*FIG. 7*

EP 2 676 137 B1

FIG. 8

| Acession # | Bridge ELISA | | Mobility Shift Assay (4% Serum) | | |
|---|---|---|---|---|---|
| | Quantitative Result | Qualitative Result | HACA Shift Assay | HACA Area/Remicade-647 Area | Remicade (nM) |
| SK07010477 | 22.26 | Positive | Positive | 0.78 | 3.33 |
| SK07060083 | 1.41 | Negative | Negative | 0.1 | 4.06 |
| SK07070083 | 1.41 | Negative | Negative | 0.1 | 8.81 |
| SK07070305 | 1.41 | Negative | Positive | 0.46 | 7.34 |
| SK07070595 | 1.41 | Negative | Positive | 0.25 | 8.35 |
| SK07071213 | 2.48 | Positive | Positive | 0.16 | 5.30 |
| SK07081127 | 22.07 | Positive | Positive | 0.28 | 3.00 |
| SK07110035 | 1.41 | Negative | Positive | 0.18 | >66.7 |
| SK07141447 | 2.62 | Positive | Positive | 0.42 | 2.43 |
| SK07171059 | 10.11 | Positive | Positive | 18.02 | 2.59 |
| SK07171095 | 10.03 | Positive | Positive | 0.24 | 2.24 |
| SK07210210 | 9.26 | Positive | Positive | 0.8 | <0.67 |
| SK07231216 | 25.58 | Positive | Positive | Complete Shift | 1.34 |
| SK07310149 | 2.74 | Positive | Positive | 0.21 | <0.67 |
| SK08040168 | 22.21 | Positive | Positive | Complete Shift | <0.67 |
| SK08051035 | 9.72 | Positive | Positive | 8.7 | 1.89 |
| SK08070307 | 2.49 | Positive | Positive | 0.23 | 3.14 |
| SK08120222 | 9.2 | Positive | Positive | 0.25 | <0.67 |
| SK08260093 | 23.15 | Positive | Positive | 0.48 | 1.04 |
| SK08260783 | 2.67 | Positive | Positive | 0.25 | 3.30 |
| 62.5ng Remicade - 647 | | | | 0.12 | |
| 100ng TNF - 647 | | | | | <0.67 |

*FIG. 9*

EP 2 676 137 B1

| | Bridge HACA Assay | Biotin and DIG-Based Homogeneous Bridging ELISA | HACA Assay of the Present Invention |
|---|---|---|---|
| Assay Format | HRP-Avidin<br>Bio-Remicade<br>HACA<br>Remicade | HRP-anti-DIG<br>DIG-Remicade<br>HACA<br>Bio-Remicade<br>Avidin | Fl-Remicade + HACA<br>↓<br>Complex |
| Non-specific Background Interference | High | High | Low |
| Sensitivity | Low | Medium | High |
| Possibility of False-Positive and False-Negative | High | High | Low |
| IgG4 HACA Detection | No | No | Yes |
| Ig Isotype identification | No | No | Yes |
| Tolerance of Drug in the Sample | Poor | Poor | Good |

*FIG. 10*

FIG. 11

*FIG. 12*

EP 2 676 137 B1

*FIG. 13*

EP 2 676 137 B1

FIG. 14

EP 2 676 137 B1

FIG. 15

FIG. 16

# A. Non-neutralizing HACA Assay

# B. Neutralizing HACA Assay

*FIG. 17*

*FIG. 18*

FIG. 19

*FIG. 20*

FIG. 21

| HILLSLOPE | -1.813 |
|-----------|--------|
| EC50 | 1.213 |

FIG. 22

*FIG. 23*

FIG. 24

FIG. 25

FIG. 26

**HACA Assay
IFX Interference**

- 0 μg/mL Cold IFX
- 60 μg/mL Cold IFX
- 20 μg/mL Cold IFX
- 6.66 μg/mL Cold IFX

*FIG. 27*

EP 2 676 137 B1

# HACA Assay
## IFX Interference with Acid Dissociation

- ■ 0 μg/mL Cold IFX (No Acid Dissociation)
- ● 0 μg/mL Cold IFX
- ▲ 60 μg/mL Cold IFX
- ▼ 20 μg/mL Cold IFX
- ◆ 6.6 μg/mL Cold IFX

*FIG. 28*

EP 2 676 137 B1

*FIG. 29*

*FIG. 30*

*FIG. 31*

*FIG. 32*

*FIG. 33*

*FIG. 34*

FIG. 35

FIG. 36

FIG. 37

FIG. 38

FIG. 39

*FIG. 40*

*FIG. 41*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2009035216 A
- US 5231024 A, Moeller
- WO 2011056590 A
- US 2011056777 W
- US 6838250 B
- US 6406862 B
- US 20060024682 A
- US 20060019410 A
- US 20040137536 A
- US 20040033537 A
- US 6074835 A
- US 6033864 A
- US 804106 A
- US 20030105060 A
- WO 0189361 A
- US 6309643 B
- WO 03053220 A

- US 20040043931 A
- WO 2008036802 A
- WO 2009012140 A
- WO 2009108637 A
- WO 2010132723 A
- WO 2011008990 A
- WO 2011050069 A
- US 2011066624 W
- US 6835815 B
- US 6858391 B
- US 7592437 B
- US 20030190639 A
- US 20050054021 A
- US 20070072180 A
- SK 07070305
- SK 07070595
- SK 07110035

### Non-patent literature cited in the description

- describe the immunogenicity of anti-tumor necrosis factor antibodies-and provides improved methods of anti-antibody measurement. **AARDEN et al.** CURRENT OPINION IN IMMUNOLOGY. ELSEVIER, 01 August 2008, vol. 20, 431-435
- discuss the improvement of drug tolerance in immunogenicity testing by acid treatment on Biacore. **SICKERT et al.** JOURNAL OF IMMUNOLOGICAL METHODS. ELSEVIER SCIENCE PUBLISHERS, 20 May 2008, vol. 334, 29-36
- describes the detection of neutralizing anti-therapeutic protein antibodies in serum or plasma samples containing high levels of the therapeutic protein. **LOFGREN et al.** JOURNAL OF IMMUNOLOGICAL METHODS. ELSEVIER SCIENCE PUBLISHERS, 20 January 2006, vol. 308, 101-108
- discloses an Affinity Capture Elution (ACE) assay for detection of anti-drug antibody to monoclonal antibody therapeutics in the presence of high levels of drug. **BOURDAGE et al.** JOURNAL OF IMMUNOLOGICAL METHODS. ELSEVIER SCIENCE PUBLISHERS, 25 September 2007, vol. 327, 10-17

- describes the detection of antibodies against therapeutic proteins in the presence of residual therapeutic protein using a solid-phase extraction with acid dissociation (SPEAD) sample treatment prior. **SMITH ; ELISA et al.** REGULATORY TOXICOLOGY AND PHARMACOLOGY. ACADEMIC PRESS, 13 November 2007, vol. 49, 230-237
- report an acid dissociation bridging ELISA for detection of antibodies directed against therapeutic proteins in the presence of antigen. **PATTON et al.** JOURNAL OF IMMUNOLOGICAL METHODS. ELSEVIER SCIENCE PUBLISHERS, 01 September 2005, vol. 304, 189-195
- report the demonstration of specific antibodies against infliximab induced during treatment of a patient with ankylosing spondylitis. **CANAN ABAY et al.** RHEUMATOLOGY INTERNATIONAL ; CLINICAL AND EXPERIMENTAL INVESTIGATIONS. SPRINGER, 01 March 2006, vol. 26, 473-480
- describe the individualized monitoring of drug bioavailability and immunogenicity in rheumatoid arthritis patients treated with the tumor necrosis factor alpha inhibitor infliximab. **BENDTZEN et al.** ARTHRITIS & RHEUMATISM. JOHN WILEY & SONS, INC, 01 December 2006, vol. 54, 3782-3789

- report the formation, distribution, and elimination of infliximab and anti-infliximab immune complexes in cynomologus monkeys. **ROJAS J.R. et al.** JOURNAL OF PHARMACOLOGY AND EXPERIMENTAL THERAPEUTICS. AMERICAN SOCIETY FOR PHARMACOLOGY AND EXPERIMENTAL THERAPEUTICS, 01 May 2005, vol. 313, 578-585
- **JONES et al.** *Nature,* 1989, vol. 338, 225-228
- **PENNICA, D. et al.** *Nature,* 1984, vol. 312, 724
- **R. HAUGLAND.** The Handbook-A Guide to Fluorescent Probes and Labeling Technologies. Molecular probes, Inc, 2005
- **GOKE et al.** *J., Gastroenterol.,* 1997, vol. 32, 480
- **RIJCKEN et al.** *Gut,* 2002, vol. 51, 529
- **JOHNE et al.** *Scand J Gastroenterol.,* 2001, vol. 36, 291-296
- **LINDBERG et al.** *Gut,* 1992, vol. 33, 909-913
- **SENDID et al.** *, Clin. Diag. Lab. Immunol.,* 1996, vol. 3, 219-226
- **FAILLE et al.** *Eur. J. Microbiol. Infect. Dis.,* 1992, vol. 11, 43 8-446
- **FROSH et al.** *Proc Natl. Acad. Sci. USA,* 1985, vol. 82, 1194-1198
- **FUKAZAWA et al.** Immunology of Fungal Disease. Marcel Dekker Inc, 1989, 37-62
- **NISHIKAWA et al.** *Microbiol. Immunol.,* 1990, vol. 34, 825-840
- **POULAIN et al.** *Eur. J. Clin. Microbiol.,* 1993, vol. 23, 46-52
- **SHIBATA et al.** *Arch. Biochem. Biophys.,* 1985, vol. 243, 338-348
- **TRINEL et al.** *Infect. Immun.,* 1992, vol. 60, 3845-3851
- **KIKUCHI et al.** *Planta,* 1993, vol. 190, 525-535
- **NEUMAN et al.** *Clin. Chem.,* 2007, vol. 53, 1652-1657
- **NAITO et al.** *Anti-Aging Medicine,* 2010, vol. 7 (5), 36-44
- **UCHID et al.** *PNAS,* 1998, vol. 95 (9), 4882-4887
- **ABRAMSON et al.** *Atherosclerosis,* 2005, vol. 178 (1), 115-21
- **BARRETT et al.** *Nat. Genet.,* 2008, vol. 40, 955-62
- **WANG et al.** *Amer. J. Hum. Genet.,* 2009, vol. 84, 399-405
- **HUGOT et al.** *Nature,* 2001, vol. 411, 599-603
- **OGURA et al.** *Nature,* 2001, vol. 411, 603-606
- **HAMPE et al.** *Lancet,* 2001, vol. 357, 1925-1928
- **CHO et al.** *Inflamm. Bowel Dis.,* 1997, vol. 3, 186-190
- **AKOLKAR et al.** *Am. J. Gastroenterol.,* 2001, vol. 96, 1127-1132
- **OHMEN et al.** *Hum. Mol. Genet.,* 1996, vol. 5, 1679-1683
- **PARKES et al.** *Lancet,* 1996, vol. 348, 1588
- **CAVANAUGH et al.** *Ann. Hum. Genet.,* 1998, vol. 62, 291-8
- **BRANT et al.** *Gastroenterology,* 1998, vol. 115, 1056-1061
- **CURRAN et al.** *Gastroenterology,* 1998, vol. 115, 1066-1071
- **HAMPE et al.** *Am. J. Hum. Genet.,* 1999, vol. 64, 808-816
- **ANNESE et al.** *Eur. J. Hum. Genet.,* 1999, vol. 7, 567-573
- **OGURA et al.** *J. Biol. Chem.,* 2001, vol. 276, 4812-4818
- **INOHARA et al.** *J. Biol. Chem.,* 2001, vol. 276, 2551-2554
- **DIB et al.** *Nature,* 1996, vol. 380, 152-154
- **SUGIMURA et al.** *Am. J. Hum. Genet.,* 2003, vol. 72, 509-518
- **RUTGEERTS.** *N. Engl. J. Med.,* 2003, vol. 348, 601-608
- **AFIF et al.** Clinical Utility of Measuring Infliximab and Human Anti-Chimeric Antibody Levels in Patients with Inflammatory Bowel Disease. *Digestive Disease Week,* 30 May 2009
- **RADSTAKE et al.** *Ann. Rheum. Dis.,* 2009, vol. 68 (11), 1739-45
- **BENDTZEN et al.** *Scand. J. Gastroenterol.,* 2009, vol. 44 (7), 774-81
- **AIKAWA et al.** Immunogenicity of Anti-TNF-alpha agents in autoimmune diseases. *Clin. Rev. Allergy Immunol.,* 2010, vol. 38 (2-3), 82-9
- **BENDTZEN et al.** Individual medicine in inflammatory bowel disease: monitoring bioavailability, pharmacokinetics and immunogenicity of anti-tumour necrosis factor-alpha antibodies. *Scand. J. Gastroenterol.,* 2009, vol. 44 (7), 774-81
- **AFIF et al.** Clinical utility of measuring infliximab and human anti-chimeric antibody concentrations in patients with inflammatory bowel disease. *Am. J. Gastroenterol.,* 2010, vol. 105 (5), 1133-9